# EUROPEAN PATENT APPLICATION

(11) **EP 4 733 315 A2**
(43) Date of publication of application: **29.04.2026**
(21) Application number: 26152775.8
(22) Date of filing: 11.06.2019
(51) Int. Cl.: C07K 14/705

(54) **TREATMENTS ETC**

(30) Priority: 13.06.2018 EP 18177441; 12.12.2018 GB 201820215
(62) Divisional of application: 19732925.3
(71) Applicant: Kymab Ltd., Cambridge CB22 3AT (GB)
(72) Inventor: RAUNER, Martina, Cambridge, CB22 3AT (GB); HOFBAUER, Lorenz C., Cambridge, CB22 3AT (GB); PLATZBECKER, Uwe, Cambridge, CB22 3AT (GB); BASCHANT, Ulrike, Cambridge, CB22 3AT (GB); THEURL, Igor, Cambridge, CB22 3AT (GB); GERMASCHEWSKI, Volker, Cambridge, CB22 3AT (GB)
(74) Representative: Zwicker, Jörk

(57) **Abstract**

The invention relates to a protein for use in diagnosing and treating primary or secondary sclerosing diseases, a fusion protein, and nucleotide sequence and a vector, and to a pharmaceutical composition for use in diagnosing and treating primary or secondary sclerosing diseases. The invention also relates to a transferrin receptor 2 inhibitor for use in the treatment of bone diseases, iron metabolism disorders or hematopoietic disorders.

## Description

### Field of the Invention

The invention relates to the diagnosis, treatment or prevention of sclerosing diseases. The invention also relates to transferrin receptor 2 inhibitors for use in the treatment of bone diseases, iron metabolism disorders, and hematopoietic disorders.

### Background

Transferrin receptor 2 (Tfr2) is mainly expressed in the liver. Commercially-available anti-Tfr2 antibodies are available. For example, 1B1 (MyBioSource, MBS833691), 3C5 (Abnova, H00007036-M01), CY-TFR (Abnova, MAB6780) and B-6 (Santa Cruz Biotechnology, sc376278), 353816 (R&D Systems, MAB3120) and 9F8 1C11 (Santa Cruz Biotechnology, sc32271).

### Statement of Invention

In a first configuration, the invention relates to a protein for use in diagnosing and/or treating primary or secondary sclerosing diseases, a fusion protein, and nucleotide sequence and a vector, and to a pharmaceutical composition for use in diagnosing and treating primary or secondary sclerosing diseases.

In a second configuration, the invention relates to transferrin receptor 2 inhibitors for use in the treatment of bone diseases, iron metabolism disorders, and hematopoietic disorders.

The invention, thus, provides:-
Protein having an amino acid sequence that has at least 70, 75, 80, 85, 90, 95, 96, 97, 98 or 99% identity with the sequence of SEQ ID NO. 1, or the fragments thereof, for use in diagnosing and treating primary or secondary sclerosing diseases.

Protein comprising sequence SEQ ID NO. 1 or SEQ ID NO. 2 for use in diagnosing and/or treating primary or secondary sclerosing diseases.

A transferrin receptor 2 inhibitor for use in the treatment of bone diseases, iron metabolism disorders, and /or hematopoietic disorders.

### Brief Description of the Drawings

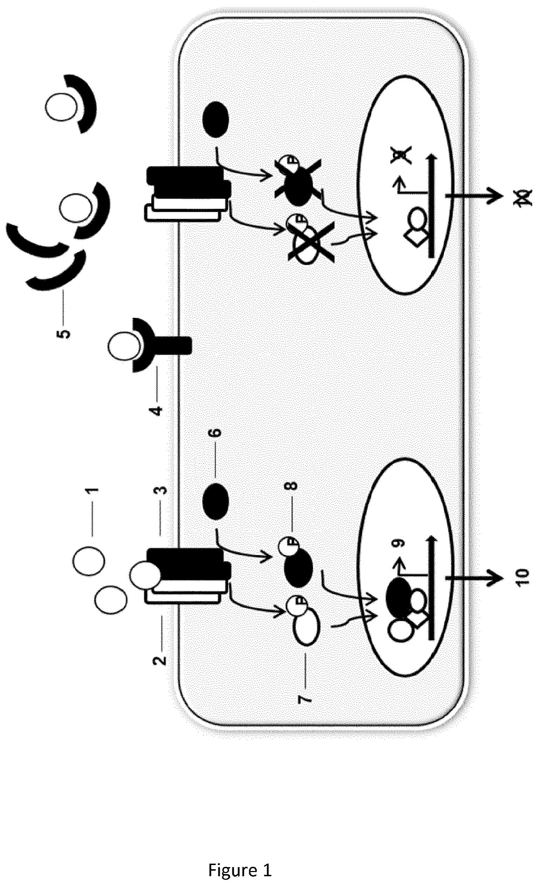

**Figure 1****:** schematic depiction of the influence of BMPs (1) on bone formation (left-hand side). BMPs bind BMP receptors (BMPR-I (2) or BMPR-II (3)), triggering a signaling cascade (phosphorylation (8) of Smad protein (6) and MAP kinase (7)) which activates bone formation (10) by the expression of osteoblast genes (9). Schematic depiction of the binding of BMPs (1) to Tfr2α (4) (center). Schematic depiction of the influence of the protein (5) according to the invention on bone formation (10) (right-hand side). The proteins (5) according to the invention bind BMPs (1), as a result of which binding to BMP receptors (BMPR-I (2) or BMPR-II (3)) does not occur, and bone formation is not activated.

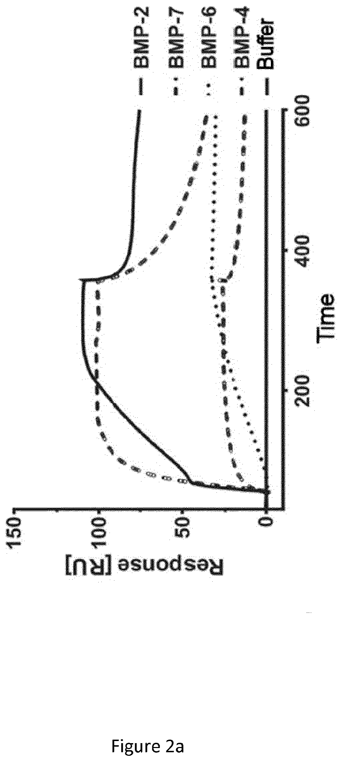

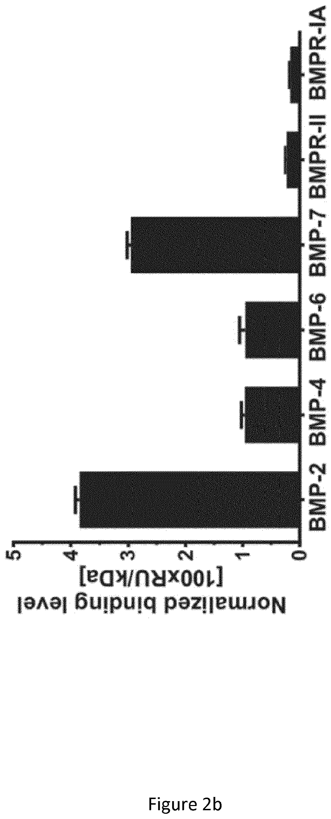

**Figure 2** shows SPR measurements of the binding of BMPs and the proteins according to the invention. Fig. 2A shows the binding of BMP-2, BMP-4, BMP-6 and BMP-7 to Tfr2-ECD. Fig. 2B shows a quantification of the binding level based on the molar mass of BMP-2, BMP-4, BMP-6 and BMP-7 to Tfr2-ECD compared with the binding level of BMPR-II and BMPR-IA.

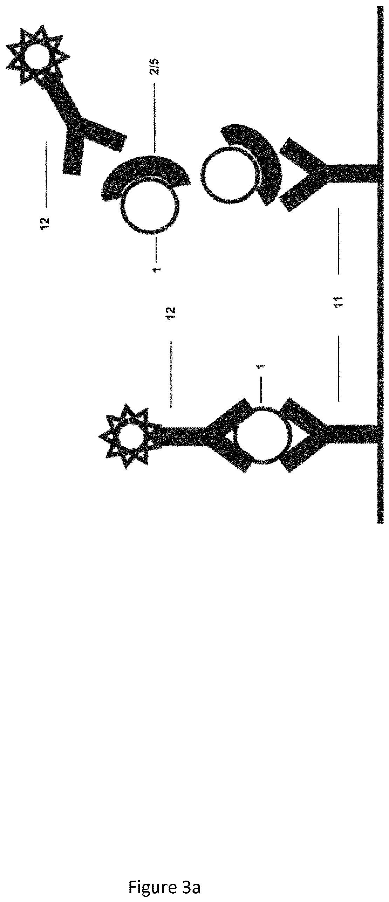

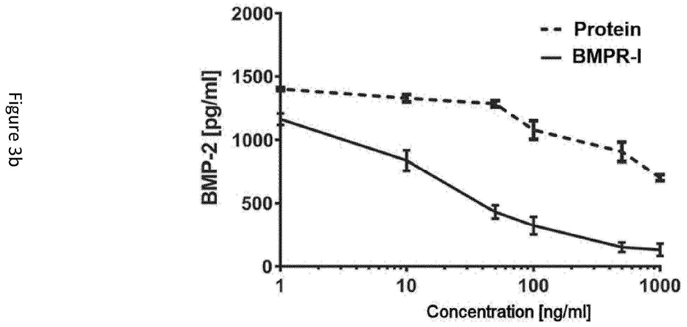

**Figure 3** schematically shows the BMP-2-competitive ELISA (enzyme linked immunosorbent assay): signal due to binding of BMPs (1), in particular BMP-2, to the capture antibody (11) and binding of the detection antibody (12) to BMP-2 (left-hand side). Reduced signal owing to the binding of the protein (5) according to the invention or BMPR-I (2) to BMP-2, as a result of which binding of the capture antibody (11) and detection antibody (12) does not occur (right-hand side). Fig. 3B: BMP-2-competitive ELISA: Influence of the concentration of the protein according to the invention or BMP-I on the signal of the BMP-2 detection antibody while the BMP-2 concentration remains unchanged.

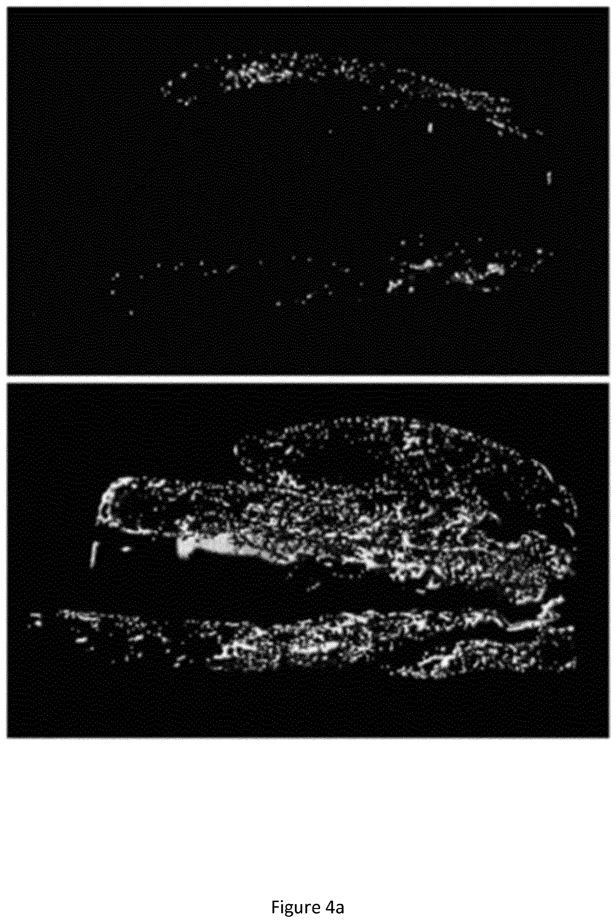

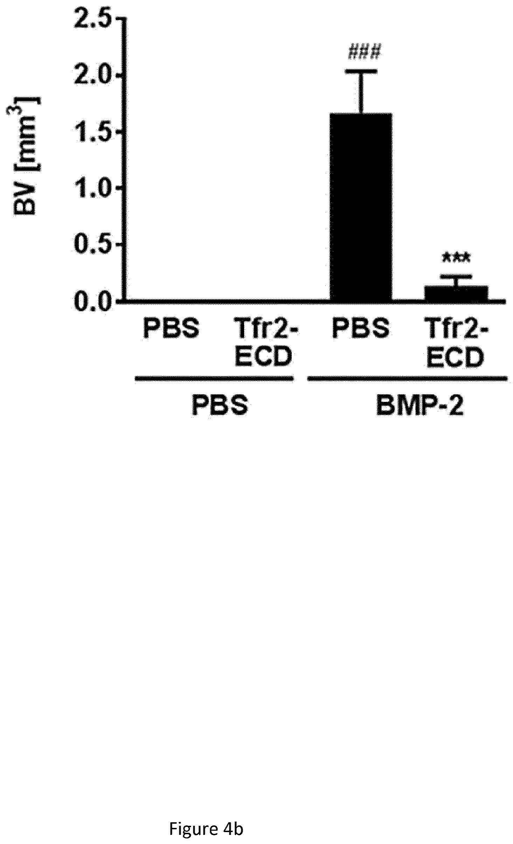

**Figure 4** shows the inhibition of HO in mice (C57BL/6 mice) using the protein according to the invention, in particular Tfr2-ECD, by means of binding of BMP-2. Fig. 4A and 4B show the mineralization by determining the bone volume by means of µCT (microtomography). Fig. 4A shows the CT scans of the bone formation when BMP-2 is applied and when BMP-2 is applied together with Tfr2-ECD. Fig. 4B shows the quantification of the bone volume. PBS is used as a negative control. When BMP-2 is applied, the increase in the bone volume is evident after two weeks. Applying BMP-2 together with Tfr2-ECD exhibits a significant reduction in bone formation compared with BMP-2 as a reference (average value ± standard deviation; n = 3 - 6 per group; ***p < 0.001 with respect to PBS (control).

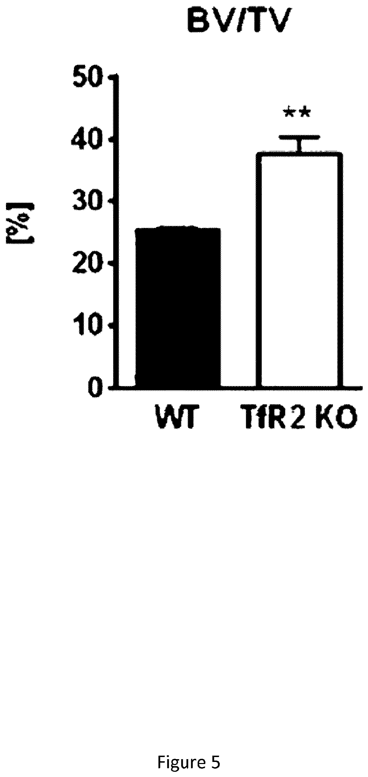

**Figure 5** is a graph of different trabecular bone volumes.

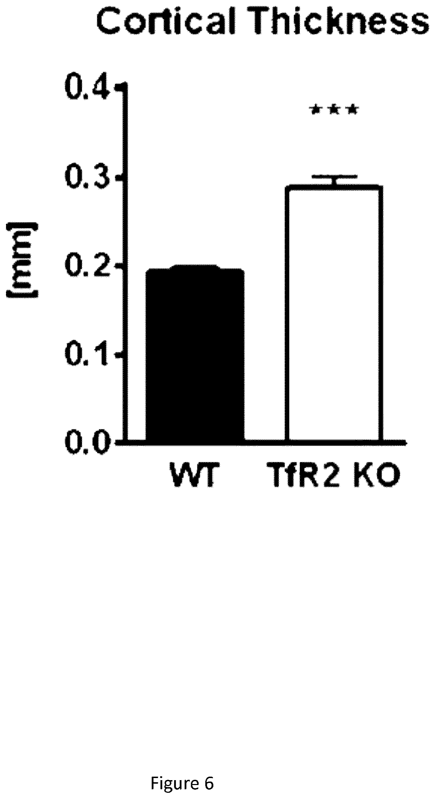

**Figure 6** is a graph of different cortical bone thicknesses.

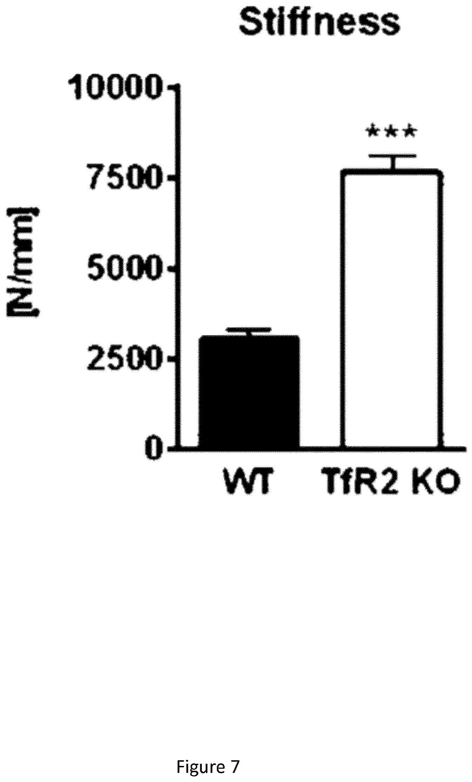

**Figure 7** is a graph of different bone qualities.

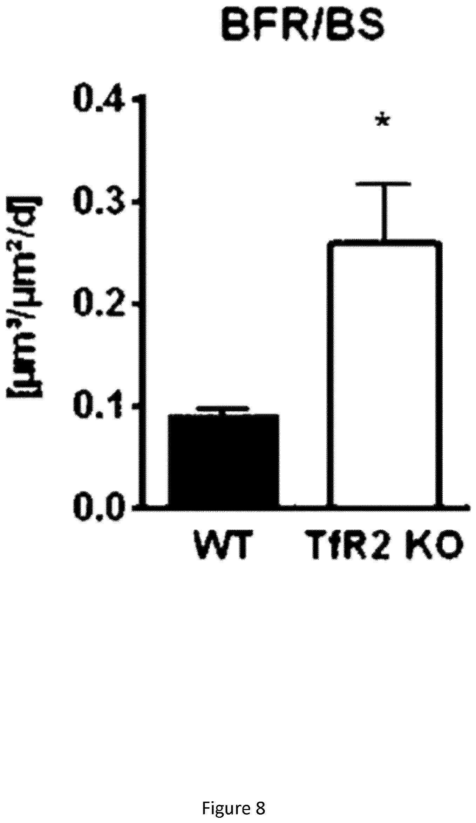

**Figure 8** is a graph of different bone formation.

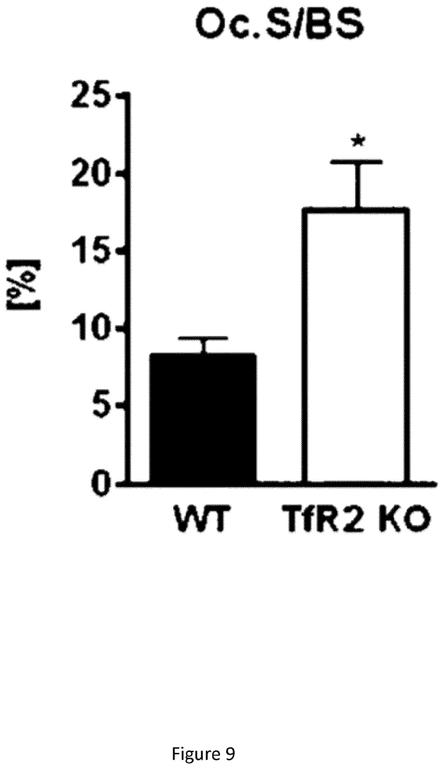

**Figure 9** is a graph of different bone resorption.

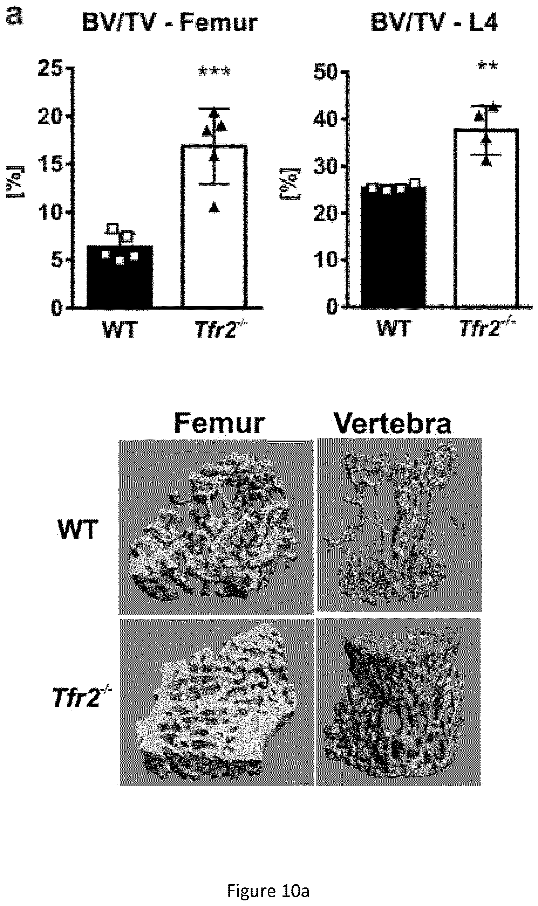

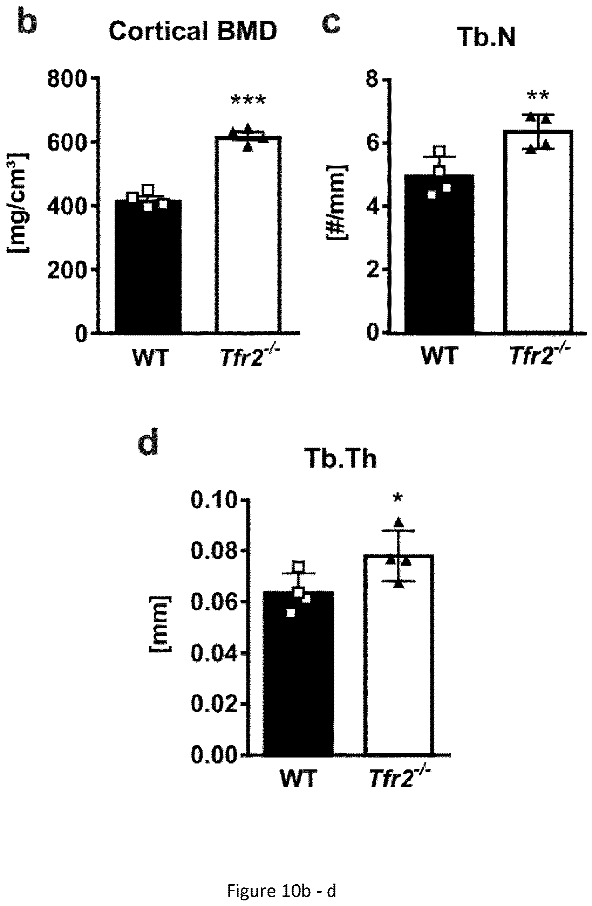

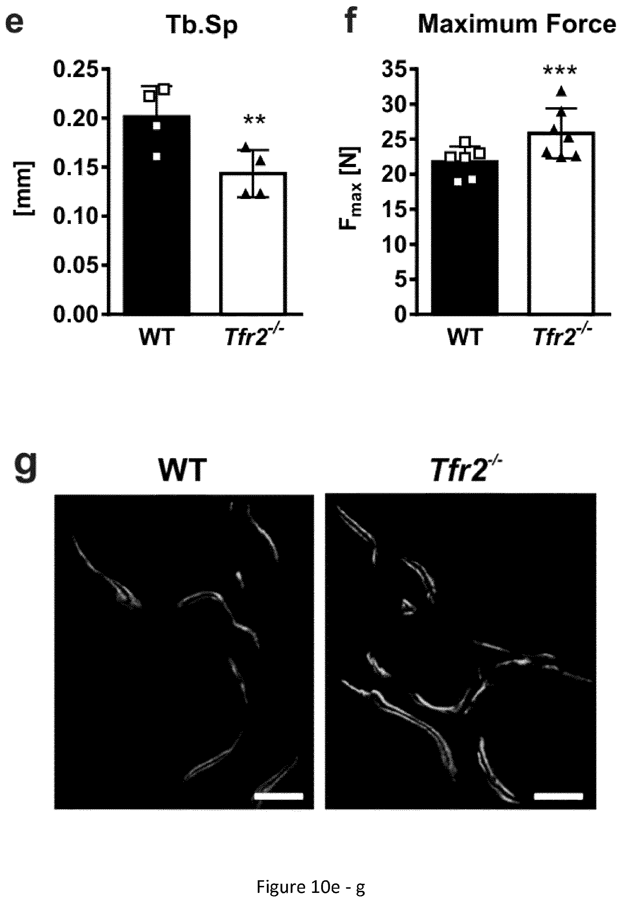

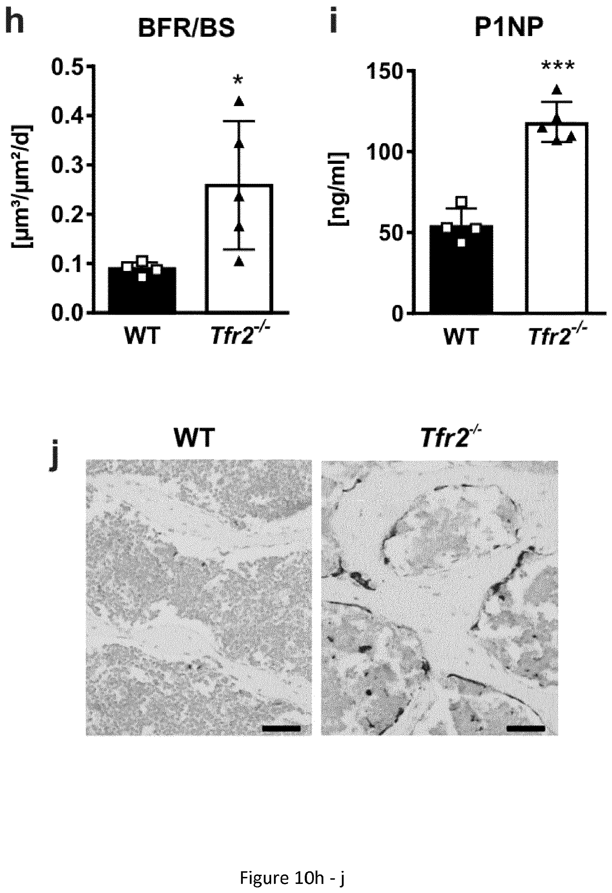

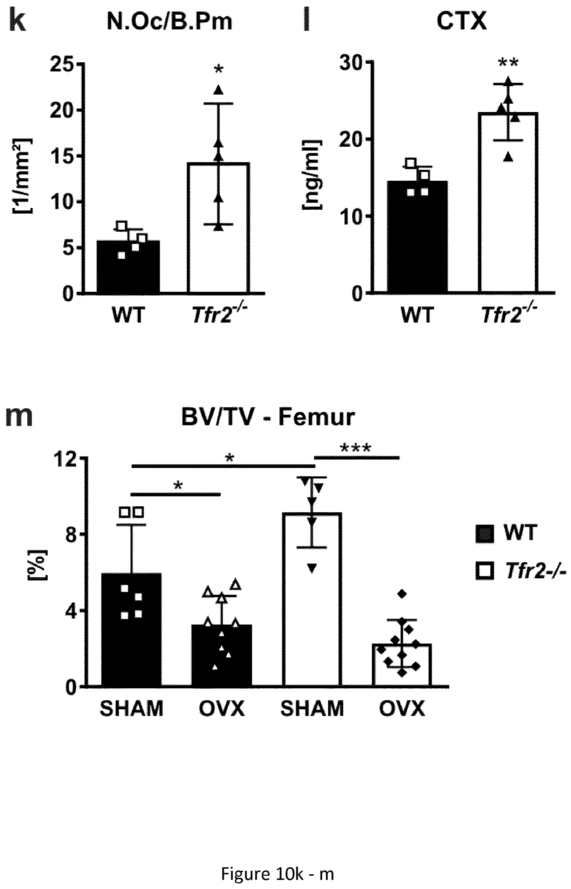

**Figure 10****: *Tfr2* deficiency results in high bone mass.** (a-I) The bones and serum bone turnover markers of ten-week-old male WT or *Tfr2*^{*-*/*-*} mice were analyzed using µCT, histology, and ELISAs. (a) 3D reconstruction and quantitation of the bone volume/total volume (BV/TV) of the distal femur and the fourth vertebral body of WT and *Tfr2*^{*-*/*-*} mice. Femur, n=5 per group; vertebral body, n=4 per group. (b) Cortical bone mineral density (BMD) at the femoral mid-shaft. n=4 per group. (c-e) Quantitation of vertebral trabecular number (Tb.N), trabecular thickness (Tb.Th), and trabecular separation (Tb.Sp). n=4 per group. (f) Maximum force at the femoral shaft assessed by 3-point-bending. (WT, n=6; Tfr2^{-/-}, n=7). (g) Representative histological sections from the third vertebral body of WT and *Tfr2*^{*-*/*-*} mice showing calcein double staining. Bar indicates 100 µm. These experiments were repeated four times with similar results. (h) Quantification of the bone formation rate/bone surface (BFR/BS). (WT, n=4; Tfr2^{-/-}, n=5). (i) Quantification of serum P1NP as a marker of bone formation. (WT, n=4; Tfr2^{-/-}, n=5). (j) Representative histological tartrate-resistant acid phosphatase sections from the fourth vertebral body of WT and *Tfr2*^{*-*/*-*} mice showing osteoclasts stained in pink. Bar indicates 100 µm. These examinations were performed four times with similar results. (k) Quantification of the number of osteoclasts/bone perimeter (N.Oc/B.Pm). (WT, n=4; Tfr2^{-/-}, n=5). (I) Quantification of serum CTX as a marker of bone resorption. (WT, n=4; Tfr2^{-/-}, n=5). (a-f, h-i, k-I) A two-tailed t-test was used for statistical analysis. (m) µCT analysis of femoral bone of 12-week-old sham-operated (SHAM) or ovariectomized (OVX) WT and *Tfr2*^{*-*/*-*} mice. (WT Sham, n=6-10; WT OVX, n=9; Tfr2^{-/-} Sham, n=5; Tfr2^{-/-} OVX, n=10). Two-way ANOVA with Bonferroni post-hoc test was used for statistical analysis. Data in all subpanels are presented as mean±SD with significances defined as *p<0.05, **p<0.01, ***p<0.001.

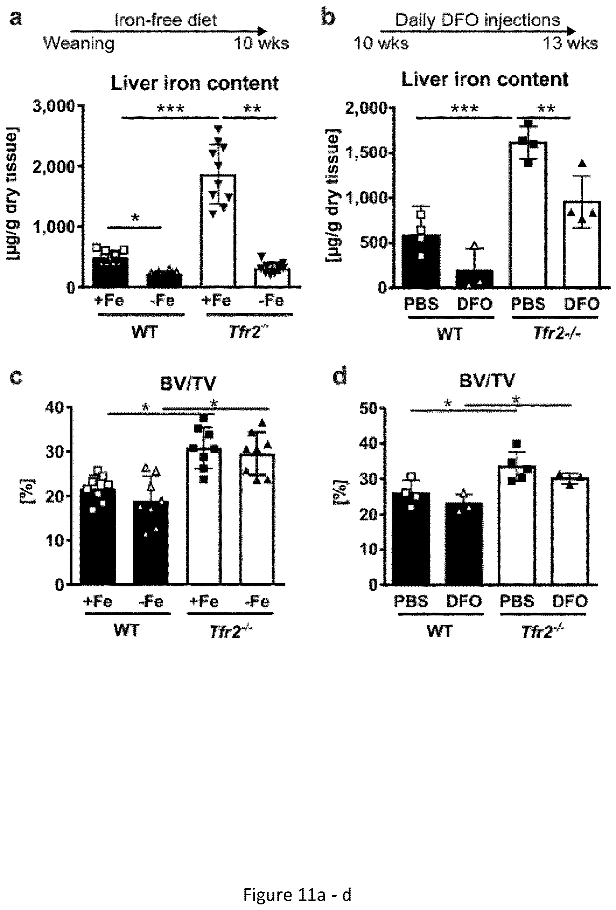

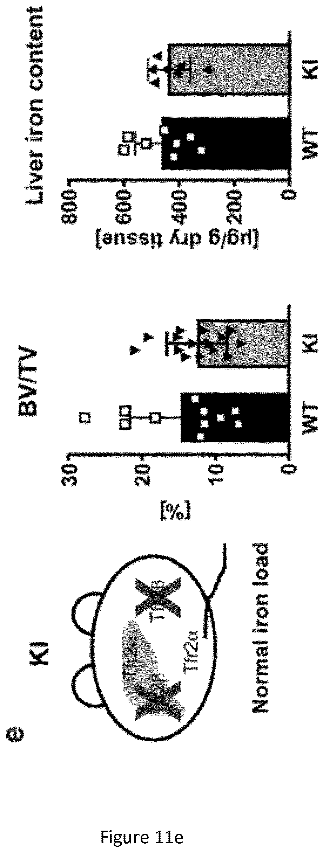

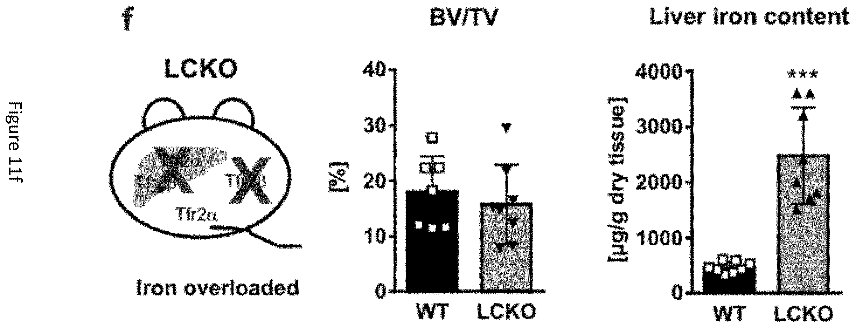

**Figure 11****: High bone mass in *Tfr2*^{*-*/*-*} mice is independent of iron overload and the hepatic function of Tfr2.** (a, c) Male WT and *Tfr2*^{*-*/*-*} mice received a purified diet without iron (-Fe) starting from weaning until the age of 10 weeks. Control mice received a standard diet with 0.2 g iron/kg food (+Fe). (a) Liver iron content was determined on dried tissue using photometry. (mean ±SD; n=10 per group). (c) Bone volume/total volume (BV/TV) was assessed using µCT. (mean±SD; WT +Fe n=9; WT -Fe, Tfr2^{-/-} +Fe and Tfr2^{-/-} -Fe, n=8 per group). (b, d) Ten-week old female WT and Tfr2^{*-*/*-*} mice received daily i.p injections of 250 mg/kg deferoxamine (DFO) or PBS for three weeks. At 13 weeks of age, mice were sacrificed to measure (b) iron liver content and (d) BV/TV. (n=3-5 per group). (a-d) Two-way ANOVA with Bonferroni post-hoc test was used for statistical analysis. (e) Schematic representation of *Tfr2* knock-in (KI) mice, which lack the *Tfr2β* isoform. BV/TV and iron liver content of 10-week-old male *Tfr2* KI mice and control mice. (BV/TV, WT n=11, KI n=15; Liver iron, WT n=8, KI n=7). (f) Schematic representation of the Tfr2 setup of liver-specific *Tfr2α* knock-out (LCKO) mice on a *Tfr2* KI background. BV/TV and liver iron content of 10-week-old male LCKO and WT mice. (n=8 per group). (e,f) A two tailed t-test was used for statistical analysis. All data are presented as mean ±SD. *p<0.05; **p<0.01; ***p<0.001.

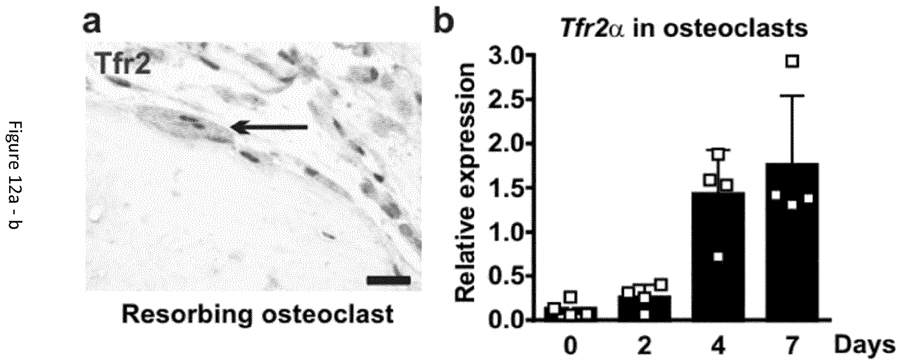

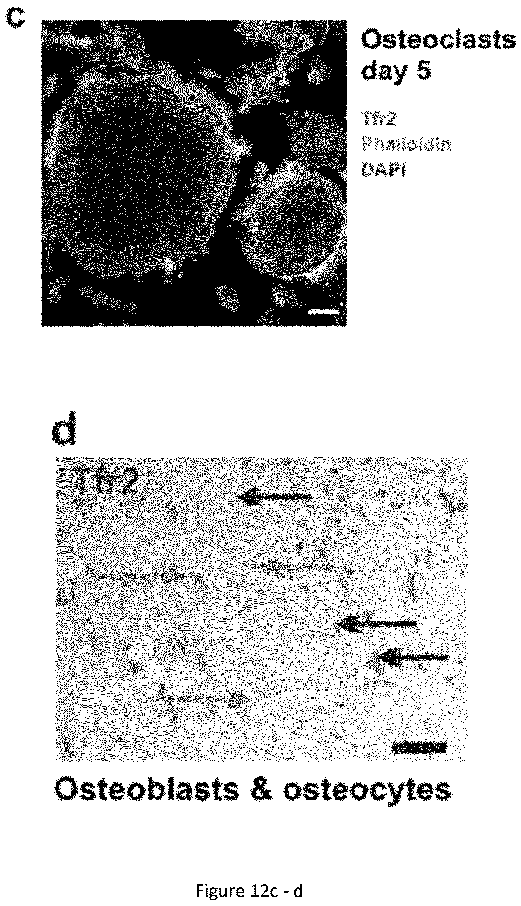

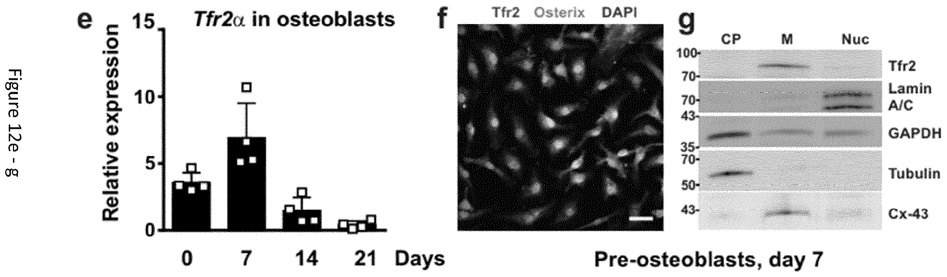

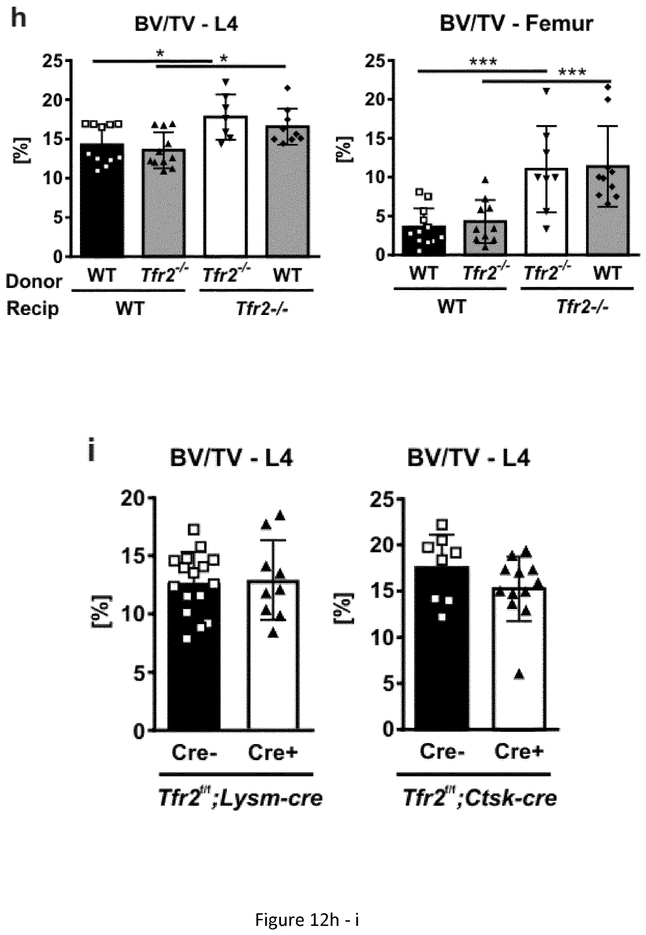

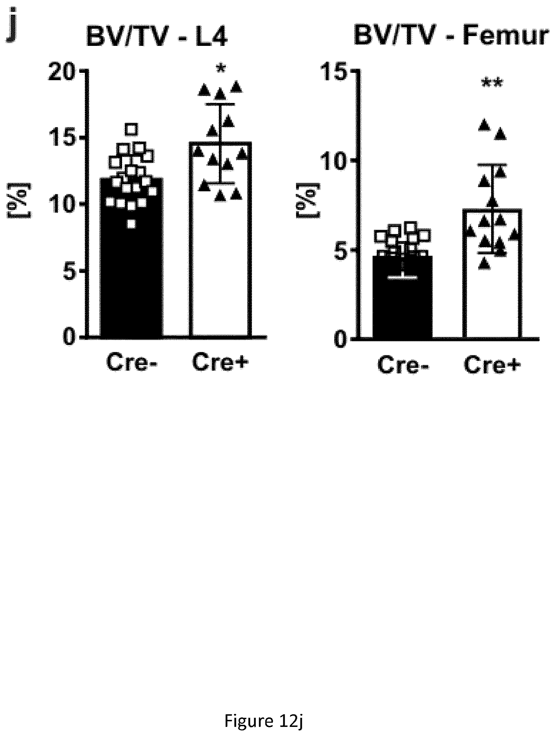

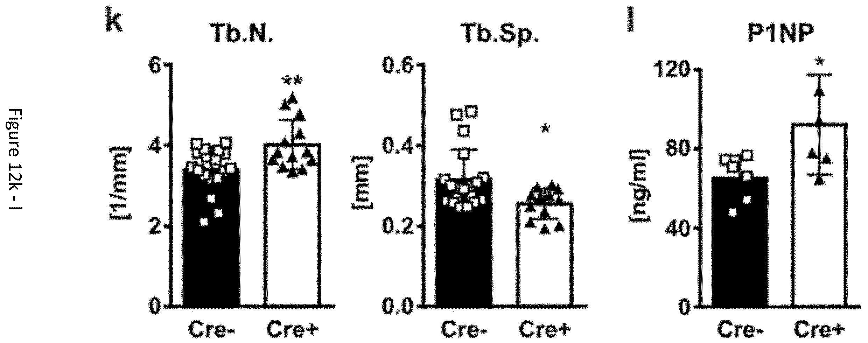

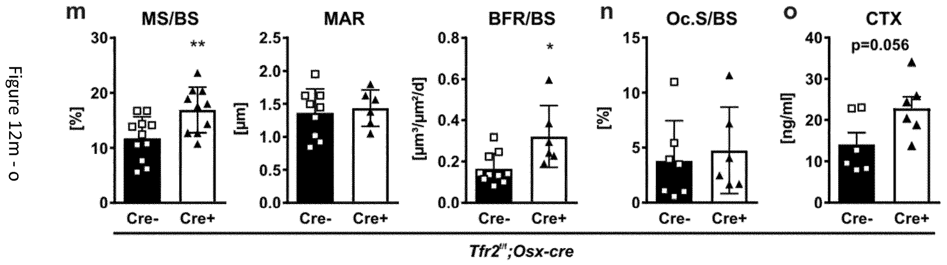

**Figure 12****: Deficiency of *Tfr2* in osteogenic cells increases bone mass.** (a, d) Immunohistochemical analysis of Tfr2 on vertebral bone of WT mice. One representative image is shown out of three. Arrows indicate Tfr2 expression in multinucleated osteoclasts and osteoblasts/osteocytes (osteoblasts: black arrows; osteocytes: orange arrows). Scale bar: 20 µm. (b, e) *Tfr2α* mRNA expression during osteoclast (b) and osteoblast (e) differentiation of WT cells (n=4). (c, f) Immunofluorescence staining for Tfr2 in mature osteoclasts (c) and immature osteoblasts (f). Scale bar: 20 µm. These staining were repeated twice with similar results. (g) Subcellular fractioning of day 7 osteoblast protein lysates from WT mice. One representative blot is shown out of three. CP: cytoplasm; M: membrane; Nuc: nuclear fraction. Cx-43: connexin-43. (h) Bone marrow was transplanted from 12-week-old male WT or *Tfr2*^{*-*/*-*} mice into lethally irradiated 9-week-old male WT and *Tfr2*^{*-*/*-*} recipient (recip) mice. After 16 weeks, the bone volume/total volume (BV/TV) was measured using µCT. (L4, WT-WT n=11, Tfr2^{-/-}-WT n=11, Tfr2^{-/-}-Tfr2^{-/-} n=7, WT-Tfr2^{-/-} n=9; Femur, WT-WT n=12, Tfr2^{-/-}-WT n=10, Tfr2^{-/-}-Tfr2^{-/-} n=8, WT-Tfr2^{-/-} n=10). Two-way ANOVA with Bonferroni post-hoc test was used for statistical analysis. (i) BV/TV of the fourth lumbar vertebrae of 10-week-old male cre-positive and crenegative *Tfr2*^{*f*/*f*};*Lysm-Cre* and *Tfr2*^{*f*/}*^{f};Ctsk-Cre* mice. (Tfr2^{f/f}; Lysm-cre, Cre- n=16, Cre+ n= 9; Tfr2^{f/f}; CtskCre, Cre- n=8, Cre+ n=12). (j-o) Bone analysis of 10-week-old male cre-positive and cre-negative littermate control *Tfr2*^{*f*/*f*};*Osx-Cre* mice. (j) BV/TV (L4, Cre- n=18, Cre+ n=12; Femur,
   Cre- n=18, Cre+ n=13). (k) Trabecular number (Tb.N) and trabecular separation (Tb.Sp) (Tb.N., Cre- n=18, Cre+ n=13; Tb.Sp., Cre- n=19, Cre+ n=12). (l) Serum levels of P1NP. (n=6 per group). (m) Mineralizing surface per bone surface (MS/BS), mineral apposition rate (MAR), and bone formation rate per bone surface (BFR/BS) determined at the lumbar spine. (MS/BS, Cre- n=11, Cre+ n=10; MAR, Cre- n=9, Cre+ n=6; BFR, Cre- n=10, Cre+ n=6). (n) Osteoclast surface per bone surface (Oc.S/BS) analyzed at the lumbar spine. (Cre- n=7, Cre+ n=6). (o) Serum CTX levels (n=6 per group). (i-o) A two-tailed t-test was used for statistical analysis. All data are presented as mean±SD. *p<0.05; **p<0.01; ***p<0.001.

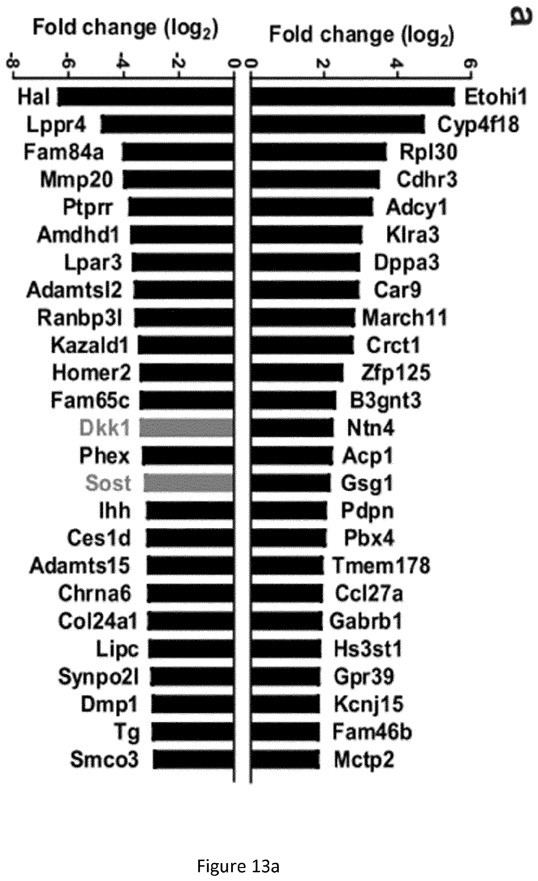

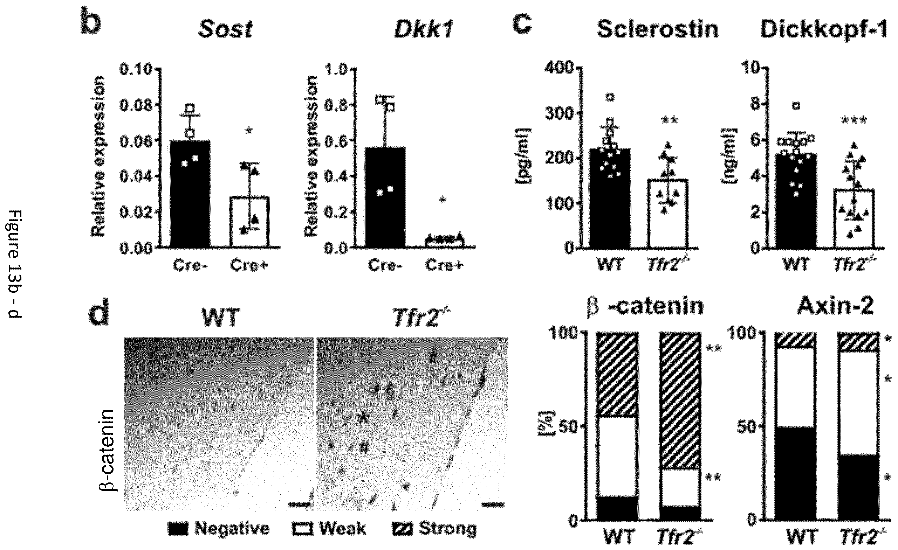

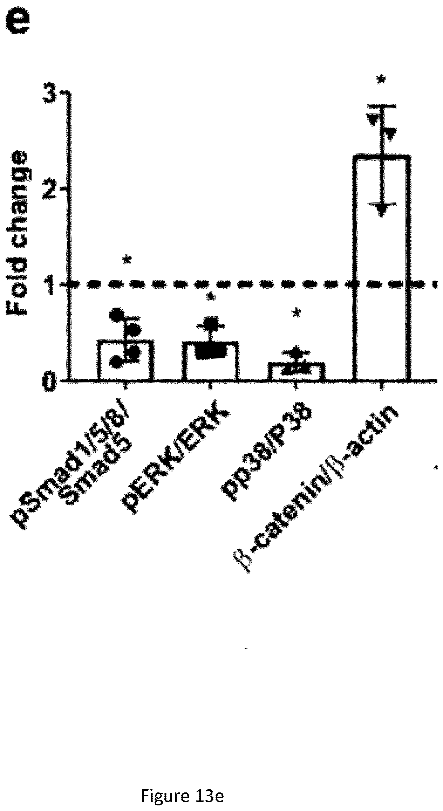

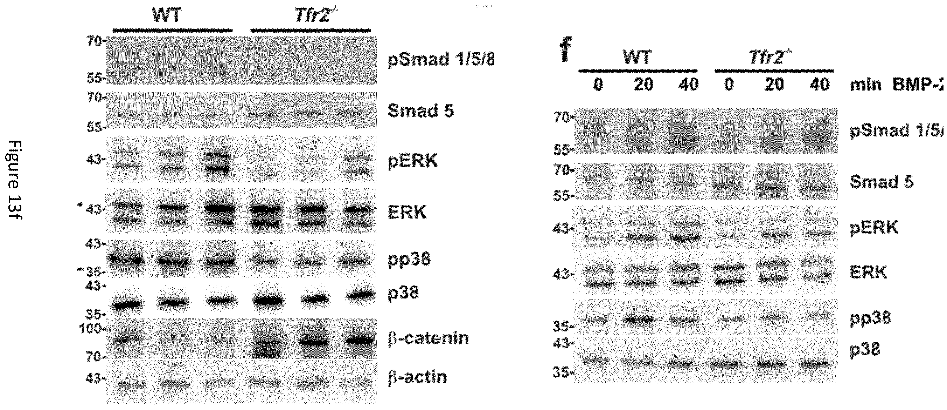

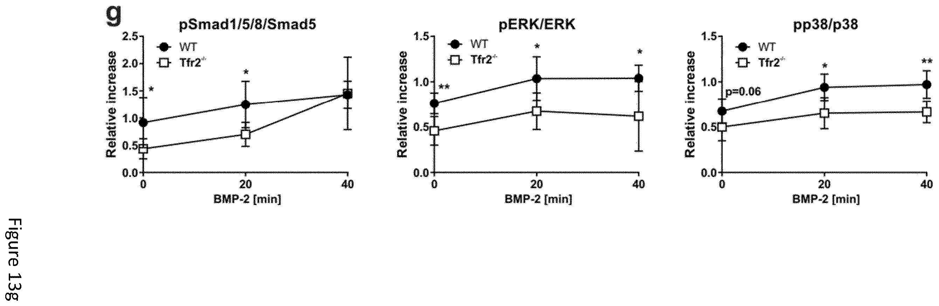

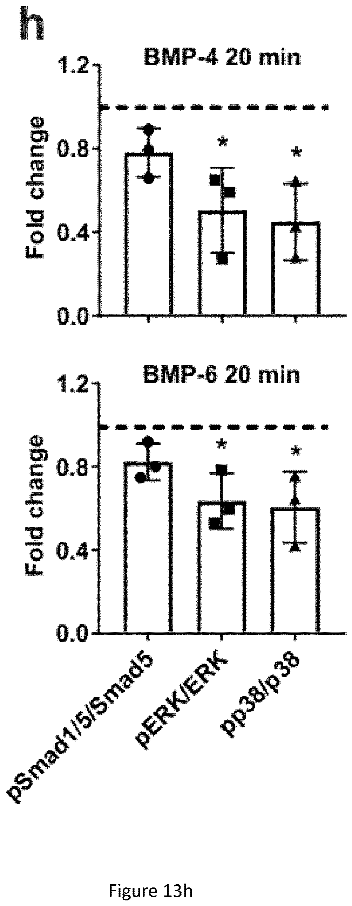

**Figure 13****: Down-regulation of BMP signaling and Wnt inhibitors in Tfr2-deficiency.** (a) Top 25 most increased and decreased genes as identified using next generation sequencing of day 7 osteoblasts from WT and *Tfr2*^{*-*/*-*} mice (n=4 per genotype). (b) Gene expression of *Dkk1* and *Sost* in day 7 differentiated osteoblasts from *Tfr2*^{*f*/*f*};*Osx-Cre* mice and littermate controls. Normalized to β-actin. (n=4 per group). (c) Serum concentrations of Dickkopf-1 and sclerostin in WT and *Tfr2*^{*-*/*-*} mice. (sclerostin, WT n=13, Tfr2^{-/-} n=10; Dickkopf, WT n=15, Tfr2^{-/-} n=14). (d) Immunohistochemical analysis of β-catenin and axin-2 on femoral bone sections from WT and *Tfr2*^{*-*/*-*} mice. Scale bar: 20 µm. Cells were quantified according to their staining intensity (negative (exemplified with an asterisk), weak (hash), strong (section mark)). (n=9 per group). (e) Analysis of the status of activated Smad and MAPK signaling in *ex vivo* differentiated osteoblasts (day 7) from *Tfr2*^{*-*/*-*} mice normalized to WT osteoblasts. Phosphorylated proteins were normalized to their unphosphorylated counterparts. Expression of β-catenin was normalized to β-actin. Quantification is the result of densitometry of 5 independent Western blot experiments. Dotted line represents the WT level. (Smad1/5/8 n=4; pERK, pp38 and β-catenin n=3). (f-g) *Ex vivo* differentiated osteoblasts from WT and *Tfr2*^{*-*/*-*} mice were treated with 50 ng/ml BMP-2 for 0-20-40 min. After protein extraction, phosphorylation of signaling proteins was analyzed using Western blot (f). (g) Graphs represent the quantification of 4 independent experiments. (n=4 per group). (h) Induction of Smad1/5, ERK, and p38 in WT and *Tfr2*^{*-*/*-*} osteoblasts after 20 min of stimulation with 50 ng/ml BMP-4 or BMP-6. Dotted line represents the WT level. (n=3 per group). (b-e, g-h) A two-tailed *t-*test was used for statistical analysis. All data are presented as mean±SD (expect for (d), which shows percentages). *p<0.05; **p<0.01; ***p<0.001.

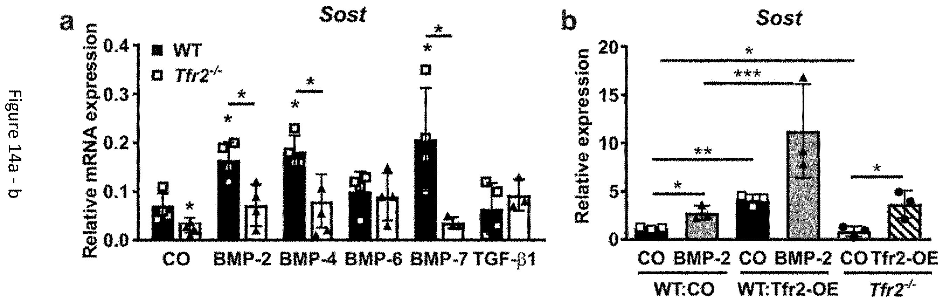

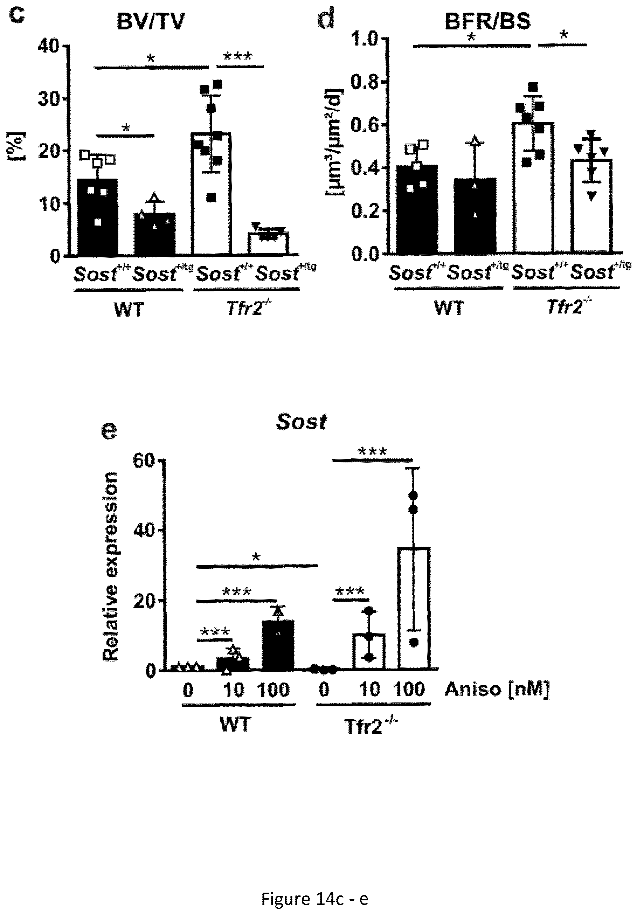

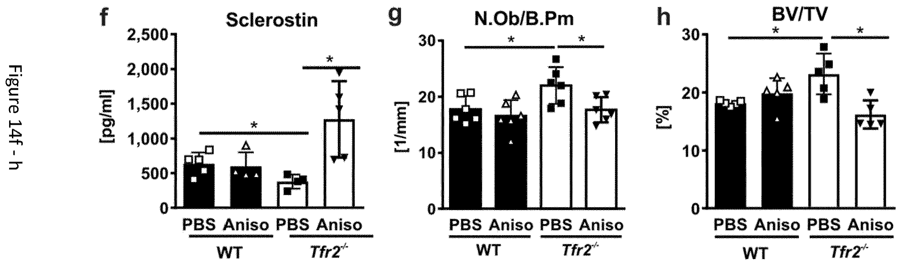

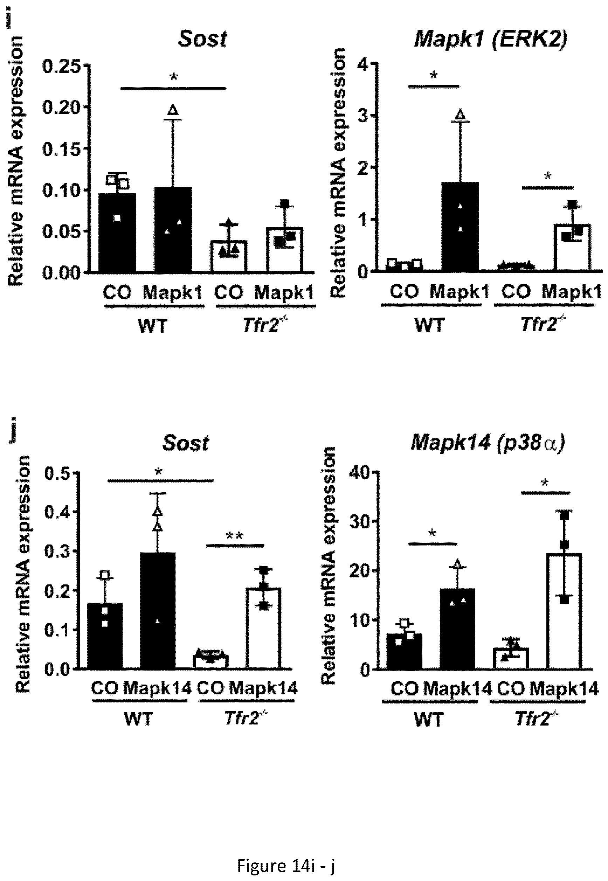

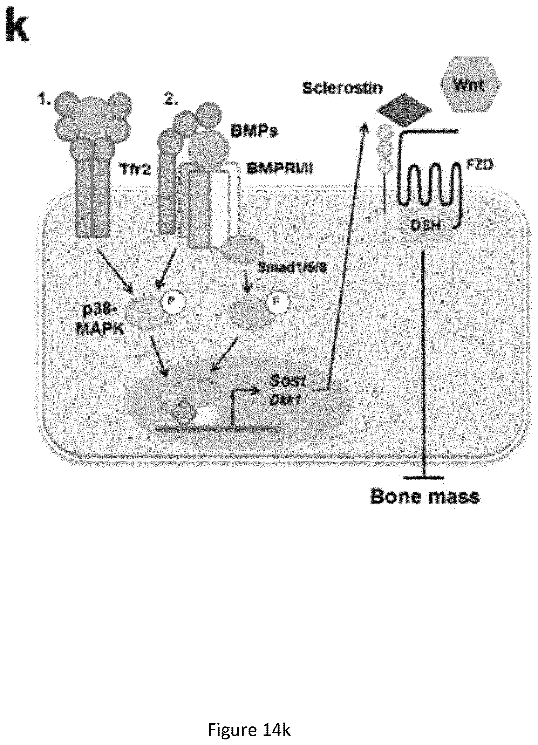

**Figure 14****: High bone mass in *Tfr2*-deficiency is rescued by overexpressing SOST or reactivating MAPK signaling.** (a) WT or *Tfr2*^{*-*/*-*} osteoblasts were differentiated for 7 days and stimulated with 50 ng/ml BMPs or TGF-β1 for 48 h. *Sost* mRNA expression was determined using qPCR. (n=4). A two-tailed *t*-test was used for statistical analysis. (b) Day 7 WT or *Tfr2*^{*-*/*-*} osteoblasts were transfected with an empty pcDNA3.1 vector (CO) or a pcDNA3.1 vector containing the Tfr2 gene (Tfr2-OE). Cells were either treated with 50 ng/ml BMP-2 or PBS. After 48 h, mRNA expression of Sost was determined using qPCR. (n=3). (c) Bone volume/total volume (BV/TV), WT/Sost^{+/+} n=6, WT Sost^{+/tg} n=4, Tfr2^{-/-} Sost^{+/+} n=8, Tfr2^{-/-} Sost^{+/tg} n=5 and (d) bone formation rate/bone surface (BFR/BS) of 10-week-old female *Tfr2*^{*-*/*-*} or WT mice containing one (*SOST*^{+/tg}) or no allele (SOST^{+/+}) of the SOST transgene. (WT/Sost^{+/+} n=5, WT/Sost^{+/tg} n=3, Tfr2^{-/-}/Sost^{+/+} n=7, Tfr2^{-/-}/Sost^{+/tg} n=6). (b-d) Two-way ANOVA with Bonferroni post-hoc test was used for statistical analysis. (e) *Sost* mRNA expression in *ex vivo* differentiated osteoblasts from WT or *Tfr2*^{*-*/*-*} mice after 24 h of anisomycin treatment (100 nM). (n=3 per group). One-way ANOVA with used for statistical analysis. (f-h) 10-week-old male WT and *Tfr2*^{*-*/*-*} mice were treated with 5 mg/kg anisomycin for 3 weeks. Shown are the (f) serum levels of sclerostin (WT/PBS n=5, WT/Aniso n=4, Tfr2^{-/-}/PBS n=4, Tfr2^{-/-}/Aniso n=5). (g) Number of osteoblasts/bone perimeter (N.Ob/B.Pm) (n=6 per group), and (h) BV/TV of the fourth lumbar vertebrae. (n=5 per group). (i-j) Overexpression of ERK2 (pCMV6-Mapk1) and p38α (pCMV6Mapk14) in day 7 WT and *Tfr2*^{*-*/-} osteoblasts. Sost expression was analyzed after 48 h and normalized to β-actin. (n=3). (f-j) Two-way ANOVA with Bonferroni post-hoc test was used for statistical analysis. All data are presented as mean±SD. *p<0.05; **p<0.01; ***p<0.001. (k) Scheme of Tfr2 actions in osteoblasts: Tfr2 binds BMPs and either directly activates BMP/MAPK signaling (1.) or activates them via binding to BMPR (2.) to induce the transcription of *Sost* and *Dkk1.* Secreted sclerostin acts as a Wnt antagonist and inhibits bone formation and decreases bone mass.

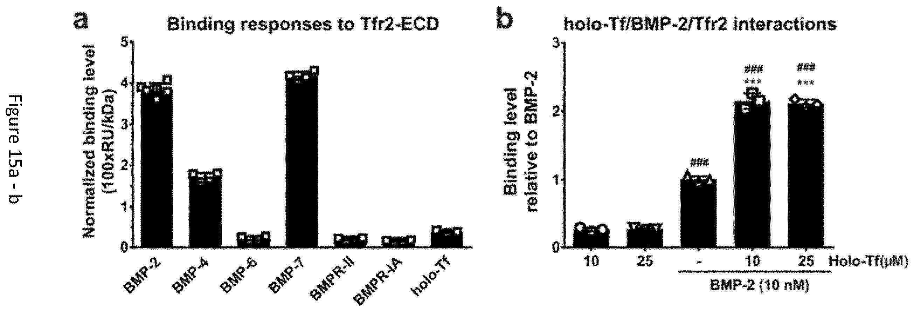

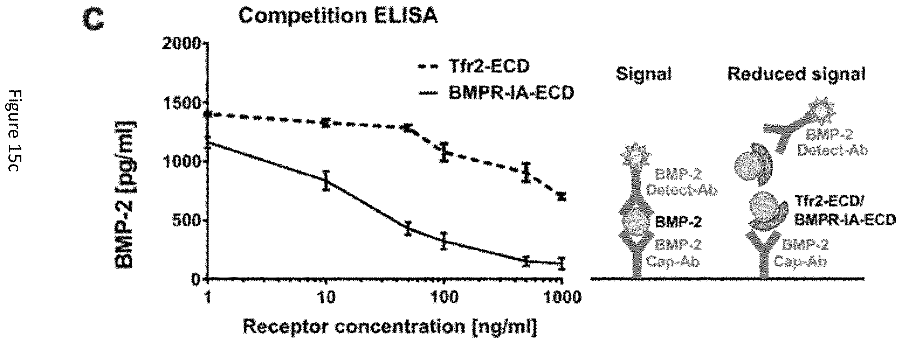

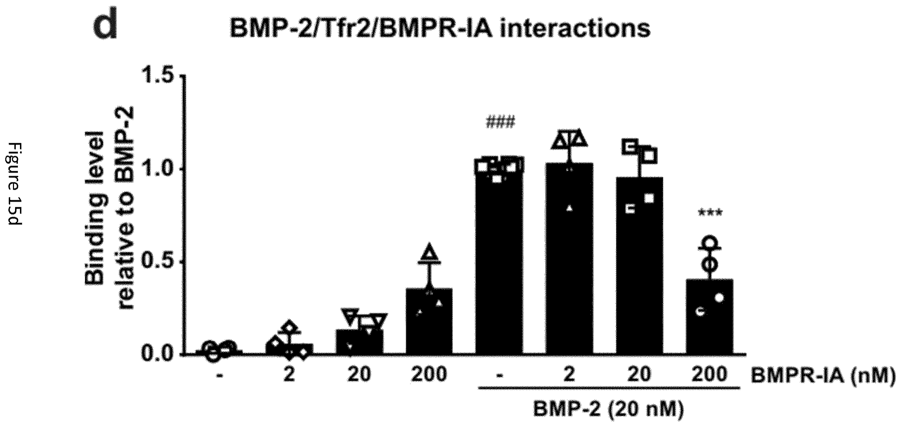

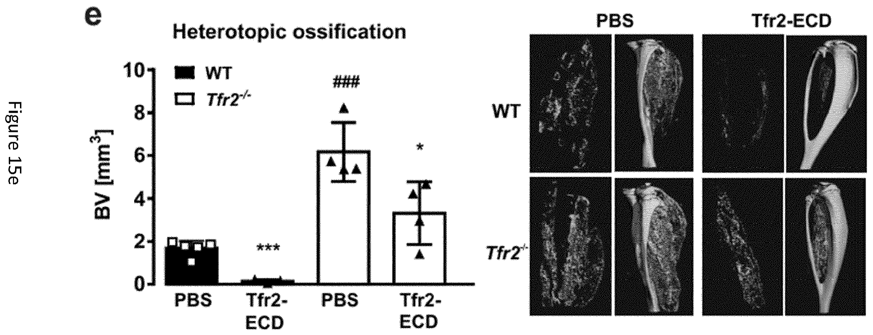

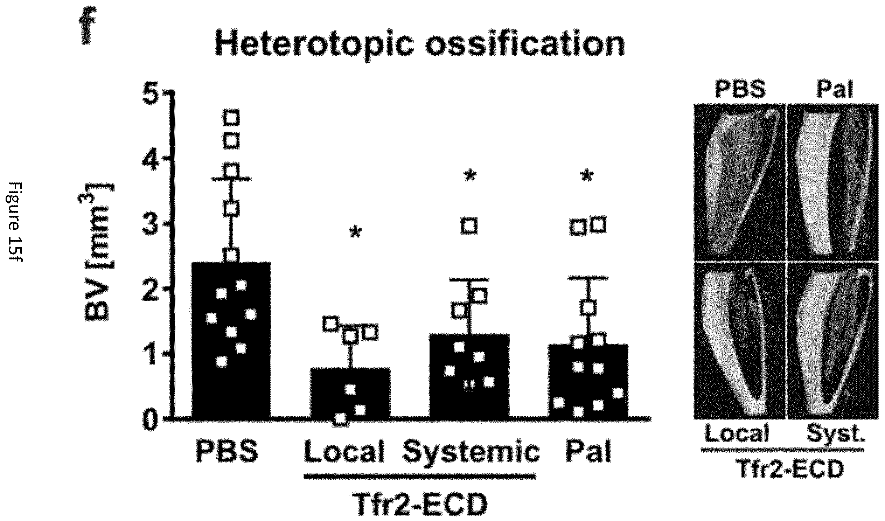

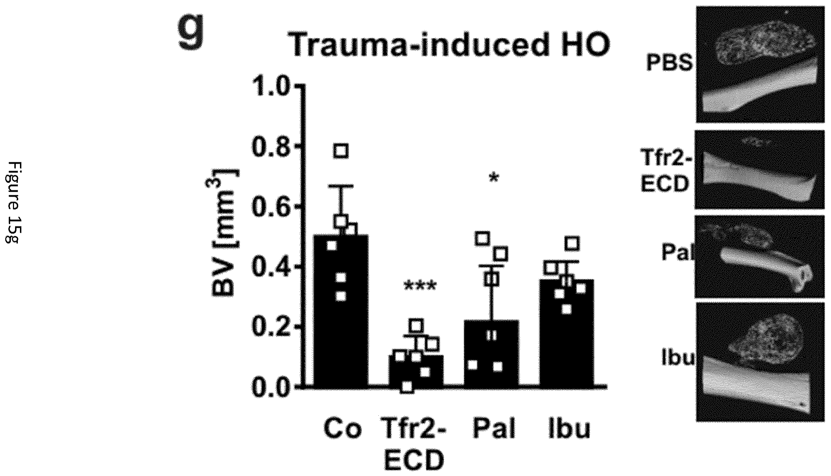

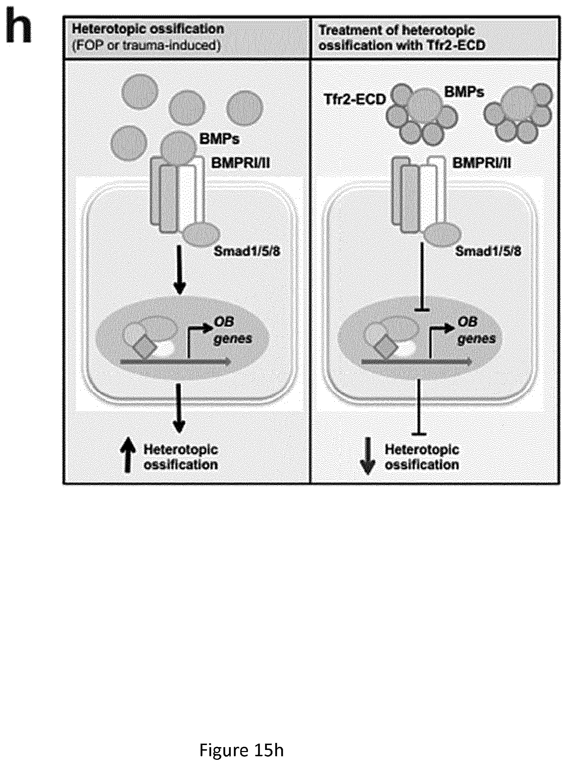

**Figure 15****: Tfr2 binds BMP ligands and blocks heterotopic ossification (HO).** (a-b) Surface plasmon resonance (SPR) experiments using Tfr2-ECD immobilized on the sensor chip and 1 mg/ml holo-transferrin (holo-Tf), different BMP ligands (50 nM), or 200 nM BMP receptors as analytes. All experiments were performed three independent times. (a) Quantification of the binding level normalized to the molecular weight. (BMP-2 n=6, BMP-4, 6, 7, BMPR-II and BMPR-IA n=4 per group, holo-Tf n=3). (b) Binding response of different concentrations of holo-Tf and/or BMP-2. (n=3; ^{###}p<0.001 vs. holo-Tf alone; ***p<0.001 vs. BMP-2 alone). (c) Competitive BMP-2 sandwich ELISA was performed to test Tfr2-ECD (or BMPR-IA as a positive control) binding to BMP-2. A defined concentration of BMP-2 (1,500 pg/ml) was used with increasing concentrations of Tfr2-ECD and BMPR-IA. The principle of the assays is shown in the right schematic. (n=4). (d) SPR analyses. BMP-2 was injected either alone or with increasing concentrations of BMPR-IA. Binding response is relative to BMP-2 alone. (n=4; ^{###}p<0.001 vs. BMPR-IA alone; ***p<0.001 vs. BMP-2 alone). (b, d) One-way ANOVA was used for statistical analysis. (e) HO in 12-week-old female WT and Tfr2^{-/-} mice after two weeks quantified by µCT (without the bone volume of the tibia). Representative images are shown at the right. (WT PBS n=5, WT Tfr2-ECD n=3, Tfr2^{-/-} PBS n=4, Tfr2^{-/-} Tfr2-ECD n=4; *p<0.05; ***p<0.001 to respective PBS control; ^{###}p<0.001 vs. WT). Two-way ANOVA with Bonferroni post-hoc test was used for statistical analysis. (f) HO in 12-week-old female WT mice after two weeks quantified by µCT (without the bone volume of the tibia). (PBS n=12, local Tfr2-ECD n=6, systemic Tfr2-ECD n=8, Pal n=11; *p<0.05 vs. PBS control). (g) Trauma-induced HO model in 12-week-old female WT mice. HO after three weeks assessed using µCT. (n=6 per group; *p<0.05, ***p<0.001 vs. PBS control). (f, g) One-way ANOVA was used for statistical analysis. All data are presented as mean±SD. (h) Scheme of the mode of action of the Tfr2-ECD in the treatment of HO. Left: HO is induced by overactive BMP signaling, leading to the induction of osteoblastic genes and bone formation. Right: Tfr2-ECD neutralizes BMPs, preventing them from activating BMP signaling. Thus, osteoblastic bone formation is inhibited.

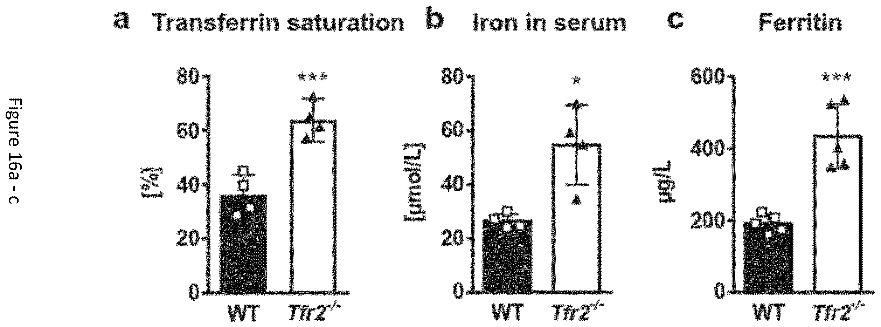

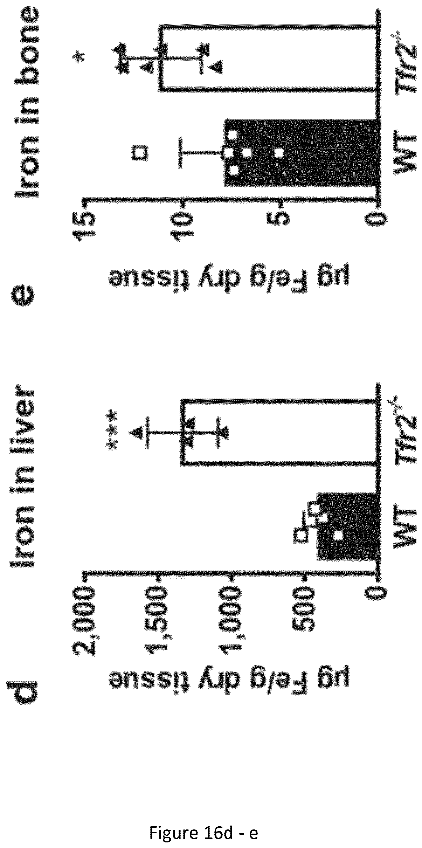

**Figure 16****: *Tfr2* deficiency results in iron overload.** (a) Transferrin saturation, n=4 per group (b) iron, and (c) ferritin were measured in the serum of male, 10-week-old *Tfr2*^{*-*/*-*} and WT mice. Iron, n=4 per group; Ferritin n=5 per group. (d) The iron content in the liver was measured in dried tissue using photometry. (n=4 per group). (e) The iron content in the cortical bone was determined using atomic absorption spectroscopy. (n=6 per group). mean±SD; *p<0.05, ***p<0.001. A two-tailed t-test was used for statistical analysis.

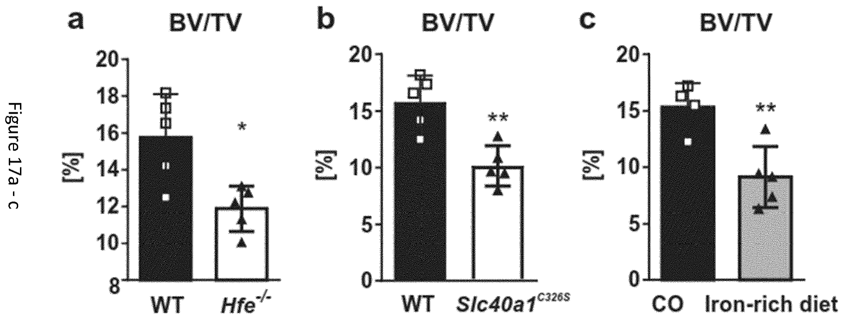

**Figure 17****: Bone mass is decreased in models of iron overload.** Bone volume/total volume was measured using µCT at the fourth vertebral body of male, 10-week-old transgenic and WT mice. (a) *Hfe*^{*-*/*-*} mice. (n=5 per group). (b) *Slc40a1*^{C326S} mice, which have a dysfunctional hepcidin-binding domain. (n=5 per group) and (c) WT mice fed with an iron-rich diet for 8 weeks. (CO n=4, Iron-rich diet n=5). (a-c) mean±SD; *p<0.05, **p<0.01. A two-tailed *t*test was used for statistical analysis.

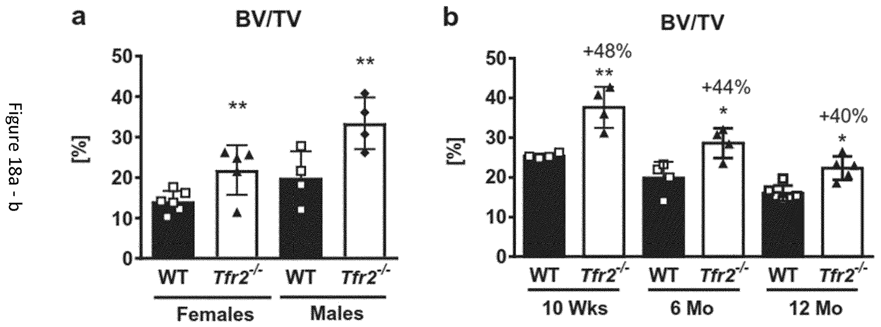

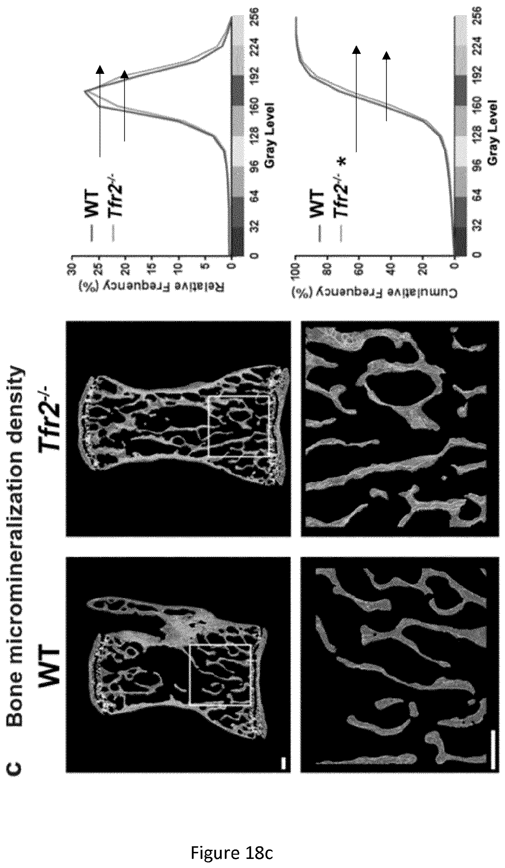

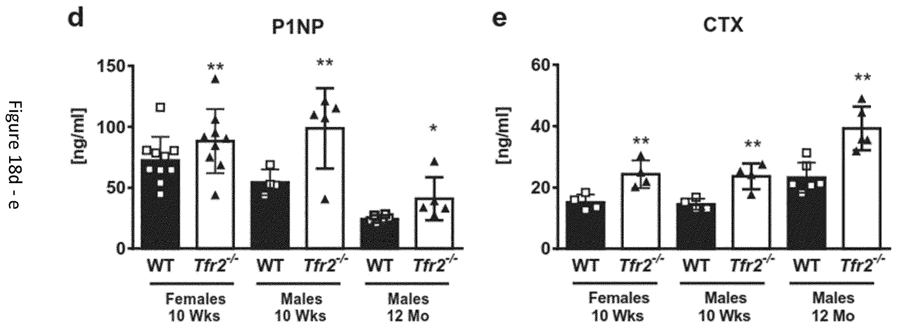

**Figure 18****: Deficiency of *Tfr2* affects bone volume across different ages and in males and females.** (a) Bone volume/total volume (BV/TV) was assessed in 10-week-old male and female WT and *Tfr2*^{*-*/*-*} mice using µCT. (Females, WT n= 6, *Tfr2*^{*-*/*-*} = 5; Males, WT+ *Tfr2*^{*-*/*-*} n=4 per group). (b) BV/TV was analyzed in male WT and *Tfr2*^{*-*/*-*} mice at 10 weeks, 6 months, and 12 months of age. (10 Wks, n=4 per group; 6 Mo, n=4 per group; 12 Mo, WT n=6, *Tfr2*^{*-*/*-*} n=5). (c) Pseudocolored qBSE-SEM images of fourth lumbar vertebrae from 12-week-old male WT and *Tfr2*^{*-*/*-*} mice with trabecular bone region of interest shown as white squares. The gray scale pixel distribution was stretched between 0 and 256 levels relative to halogenated dimethacrylate standards as indicated in the methods. Gray scale images were divided into 8 equal intervals, each represented by a different color to aid visual presentation of digital images. In these pseudocolored images low mineralization density is yellow and high density is gray. Graphs show relative and cumulative frequency of micromineralization densities in which the gray scale pixel distribution is shown in relation to each of the 8 equally sized color bins. Cumulative frequency distributions of bone micromineralization densities were compared using the Kolmogorov- Smirnov test. (mean; WT *n=4, Tfr2*^{*-*/*-*} n=5; *p<0.05). Bars 200 im. (d-e) Serum P1NP and CTX level of male and female WT and *Tfr2*^{*-*/*-*} mice of different ages. (P1NP Females, WT n=10, *Tfr2*^{*-*/*-*} n=9; Males 10 Wks, WT n=4, *Tfr2*^{*-*/*-*} n=5; Males 12 Mo, WT n=6, *Tfr2*^{*-*/*-*} n=5;). (ab, d-e) mean±SD; *p<0.05, **p<0.01. A two-tailed *t*-test was used for statistical analysis.

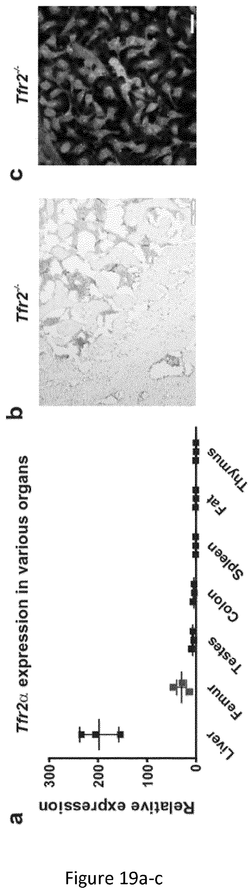

**Figure 19****: Tfr2 expression in bone tissue.** (a) *Tfr2a* mRNA expression in various organs isolated from WT mice. (mean±SD; n=3) (b) Immunohistochemical analysis of Tfr2 in bones of 12-week-old *Tfr2-*/*-* mice. One representative image is shown out of three. Scale bar: 100 µm. (c) Immunofluorescence of Tfr2 in osteoblasts derived from 12-week-old *Tfr2*^{*-*/*-*} mice. One representative image is shown out of four. Scale bar: 20 µm.

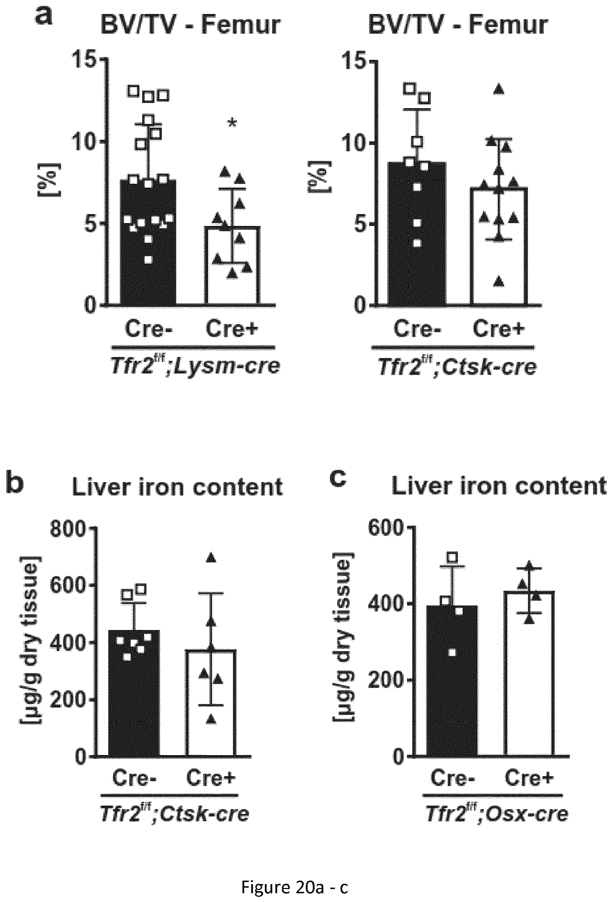

Figure 20: Bone volume and iron handling in bone cell-specific Tfr2-deficient mice. (a) Femoral bone volume/total volume (BV/TV) of male 10-week-old *Tfr2*^{f/f};*Lysm-cre* and *Tfr2*^{f/f};*Ctsk*cre mice and their littermate controls. (*Tfr2*^{f/f};*Lysm-cre,* Cre- n=17, Cre+ n=9; *Tfr2*^{f/f};*Ctsk-cre,* Cre-n=8, Cre+ n=12;). (b-c) Iron content was measured in the liver of 10-weekold male *Tfr2f*/*f;Ctskcre.* (Cre-, n=7, Cre+ n=6) and *Tfr2*^{f/f};*Osx-cre* mice and their corresponding littermate controls. (n=4 per group). (a-c) mean±SD; *p<0.05A two-tailed t-test was used for statistical analysis.

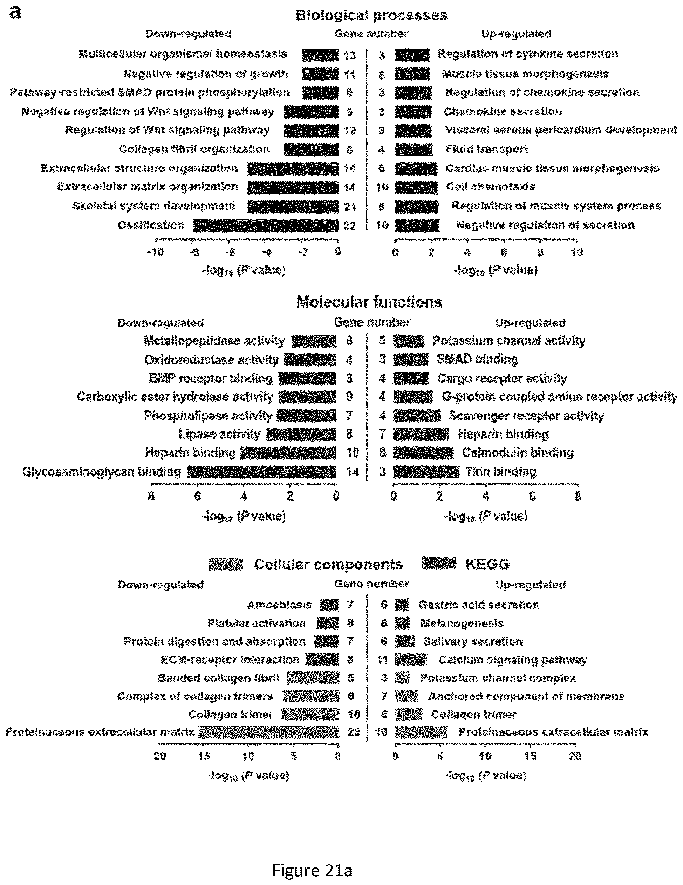

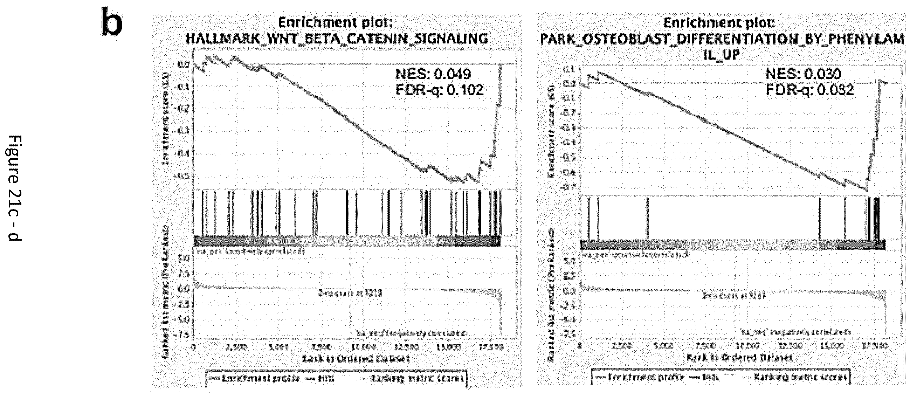

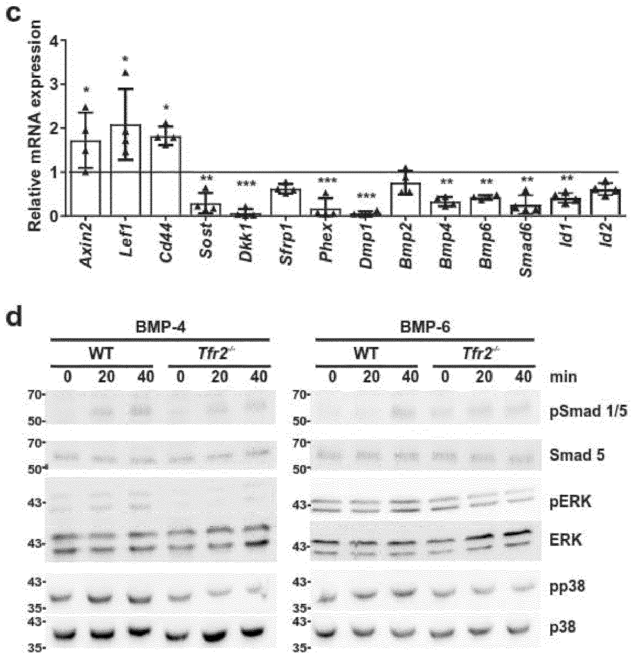

**Figure 21****: Down-regulation of BMP signaling and Wnt inhibitors in Tfr2 deficiency.** (a) Next generation sequencing was performed in osteoblasts that have been differentiated for 7 days from the bone marrow of WT and *Tfr2*^{*-*/*-*} mice (n=4 per group). Gene ontology analysis for biological processes, molecular functions and cellular components that are underrepresented (downregulated, cutoff: -1.5 (log2)) or overrepresented (up-regulated, cutoff: +1.0 (log2)) in *Tfr2*^{*-*/*-*} osteoblasts. The Wald-test implemented in DESeq2 was used for statistical analysis. (b) Gene set enrichment analysis was carried out using the Broad Institute GSEA software. Enrichment plots are shown for Wnt signaling and osteoblast differentiation (n=4 per group). (c) Validation of regulated genes using qPCR on a different set of osteoblasts isolated from *Tfr2*^{*-*/*-*} mice and normalized to WT mice. Genes were normalized to β-actin and GAPDH (mean±SD; n=4 per group; *p<0.05; **p<0.01; ***p<0.001). A two-tailed t-test was used for statistical analysis. (d) *Ex vivo* differentiated osteoblasts from WT and *Tfr2*^{*-*/*-*} mice were treated with 50 ng/ml BMP-4 or BMP-6 for 0-20-40 min. After protein extraction, phosphorylation of signaling proteins was analyzed using Western blot. One experiment of four is shown.

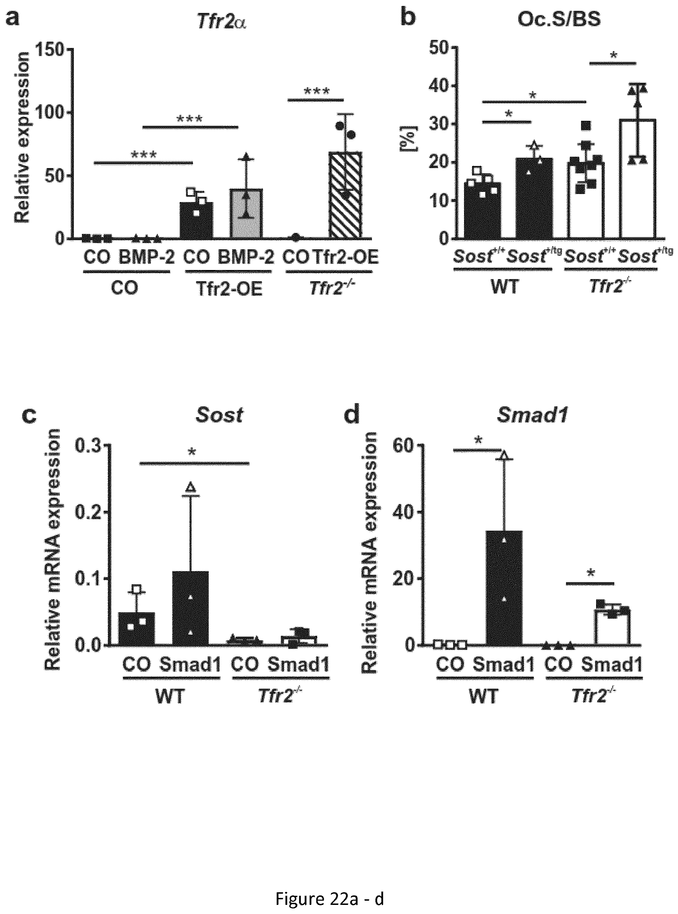

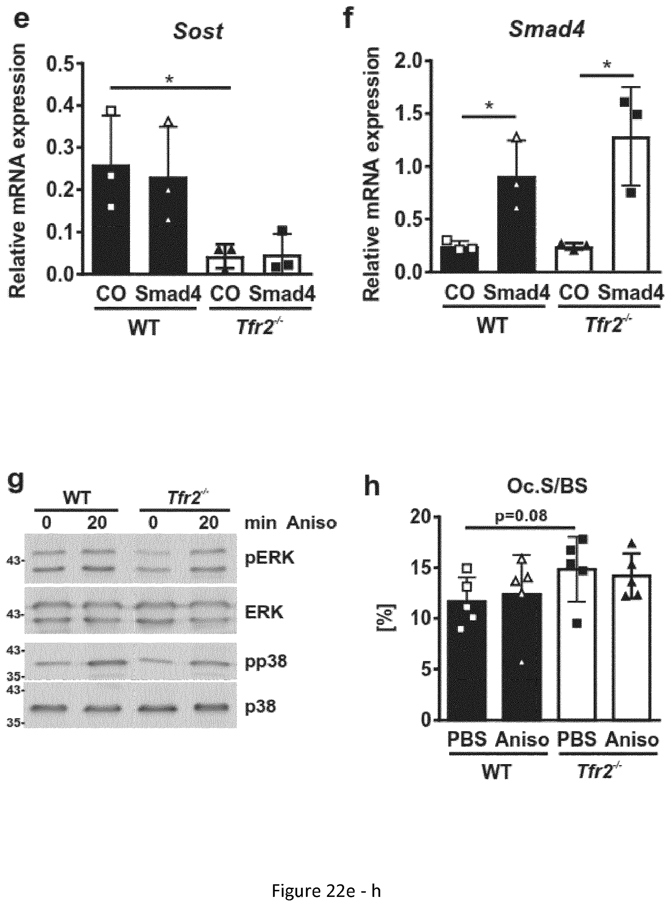

**Figure 22****: Regulation of Sost expression by *Tfr2* and anisomycin treatment.** (a) Day 7 differentiated WT or *Tfr2*^{*-*/*-*} osteoblasts were transfected with an empty pcDNA3.1 vector (CO) or a pcDNA3.1 vector containing the Tfr2α gene (Tfr2-OE). After 48 h, mRNA expression of *Tfr2a* was determined using qPCR. (n=3). (b) Osteoclast surface/bone surface (Oc.S/BS) of 10-weekold female WT and *Tfr2*^{*-*/*-*} mice containing one (SOST^{+/tg}) or no transgenic SOST allele (*SOST*^{+/+}). (WT Sost^{+/+} n=5, WT Sost^{+/tg} n=3, Tfr2^{-/-} Sost^{+/+} n=9, Tfr2^{*-*/*-*}Sost^{+/tg} n=5). (c-f) Overexpression of *Smad1* (pCMV6-Smad1) and *Smad4* (pCMV6-Smad4) in day 7 differentiated WT and *Tfr2*^{*-*/*-*} osteoblasts. *Sost, Mapk1, and Mapk14* expression was analyzed after 48 h and normalized to β-actin. (n=3). (g) Osteoblasts were isolated from WT or *Tfr2* mice and stimulated with anisomycin (100 nM) for 20 min. Western blot analysis was used to assess the activation of ERK (pERK) and p38 (pp38) signaling. One representative blot is shown of three independent experiments. (h) Osteoclast surface/bone surface (Oc.S/BS) of male, 10-week-old WT and *Tfr2*^{*-*/*-*} mice mice treated with 5 mg/kg anisomycin for 3 weeks. (n=5 per group). (a-f, h) mean±SD; *p<0.05; **p<0.01; ***p<0.001. Two-way ANOVA with Bonferroni post-hoc test was used for statistical analysis.

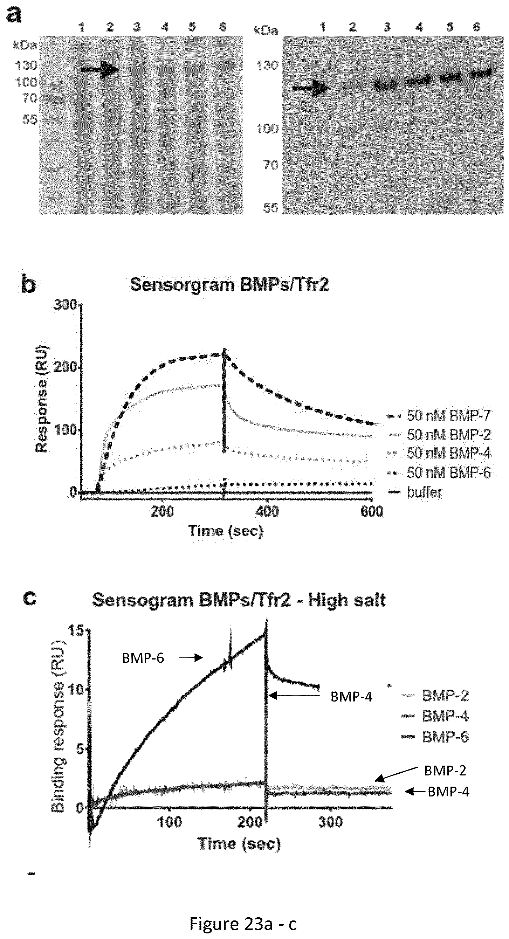

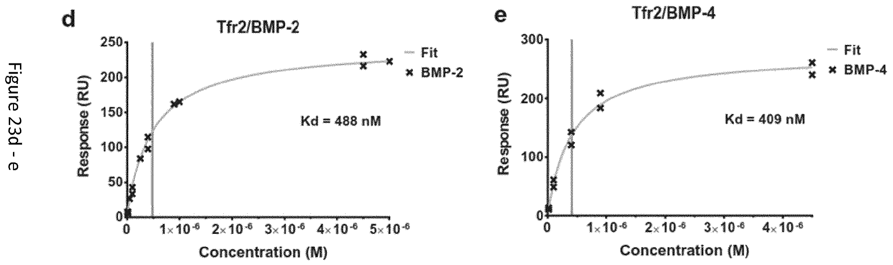

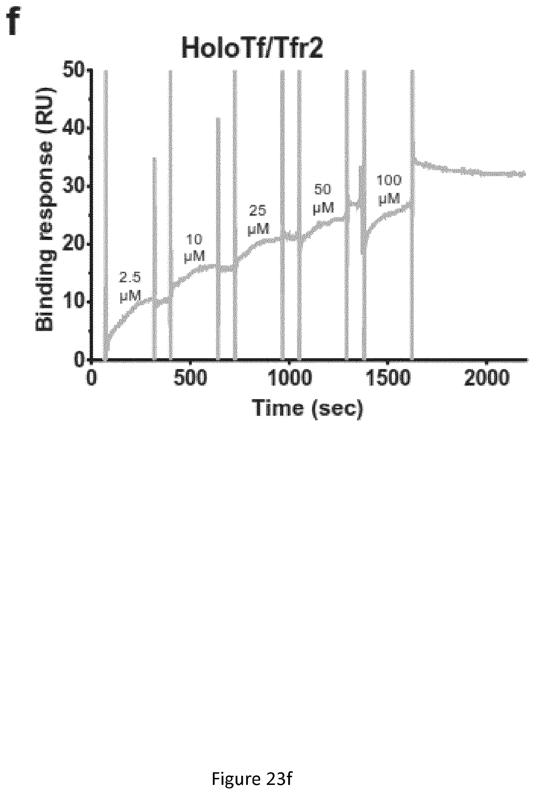

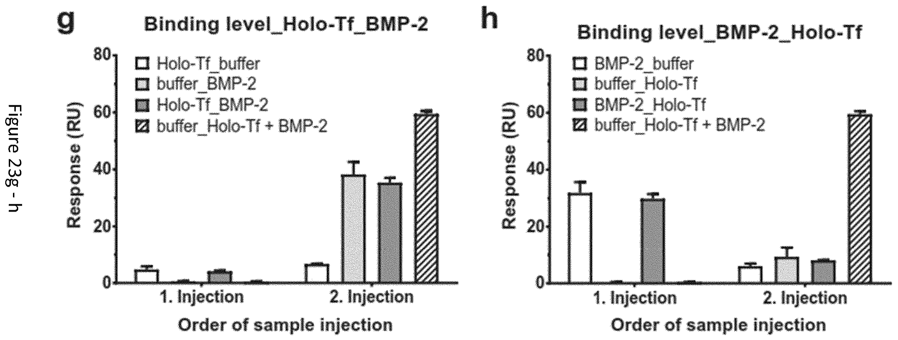

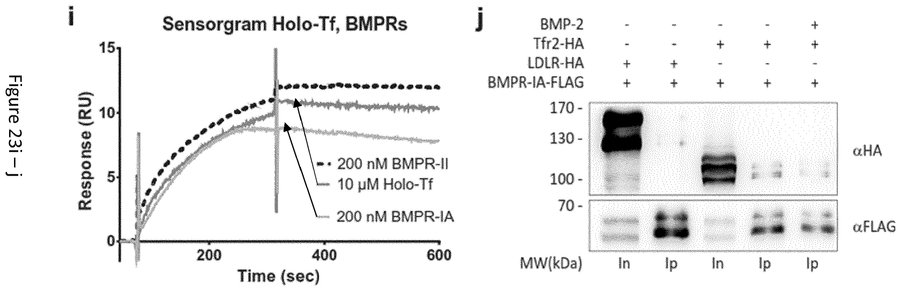

**Figure 23****: Tfr2-ECD binds to BMPs.** (a) Coomassie staining of SDS-PAGE and Western blot of Tfr2 on Tfr2-ECD eluates. Lane 1 represents washing of the column, while 2-6 represent the first elution fractions of Tfr2-ECD from the column. (b) Representative sensograms of BMPs at a concentration of 50 nM to immobilized Tfr2-ECD. (a+b) These experiments were repeated three times with similar results. (c) Binding of Tfr2-ECD to immobilized BMP-2, 4, and 6 at high salt concentrations in the running buffer (500 mM NaCl). Experiment was performed twice. (d-e) Steady state affinities determined via SPR of Tfr2-BMP-2 and Tfr2-BMP-4 binding using BMP-2 or BMP-4 immobilized on sensor chip surfaces and various concentrations of Tfr2 streaming over the chip. This experiment was performed twice. (f) Sensogram of holo-transferrin (holo-Tf) binding to Tfr2-ECD. Increasing concentrations of holo-Tf were streamed over the chip sequentially without intermediate regeneration. This experiment was performed twice. (g-h) Sequential binding experiments. Either BMP-2 was first injected or holo-Tf followed by either holo-Tf or BMP-2. N=3; mean±SD. (i) Representative sensograms of BMPR-IA, BMPR-II, and holo-Tf to immobilized Tfr2-ECD. (g-i) These experiments were repeated three times with similar results. (j) Co-immunoprecipitation (Co-IP) of BMPR-IA and Tfr2 overexpressed in HuH7 hepatoma cells. Co-IP of BMPR-IA with LDLR was used as a negative control. Co-IP of Tfr2 and BMPR-IA was performed with and without BMP-2. One experiment of three is shown.

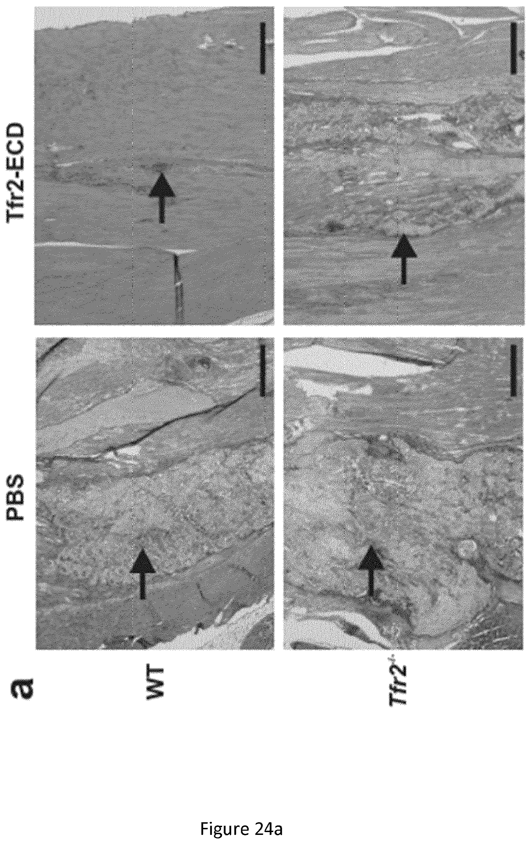

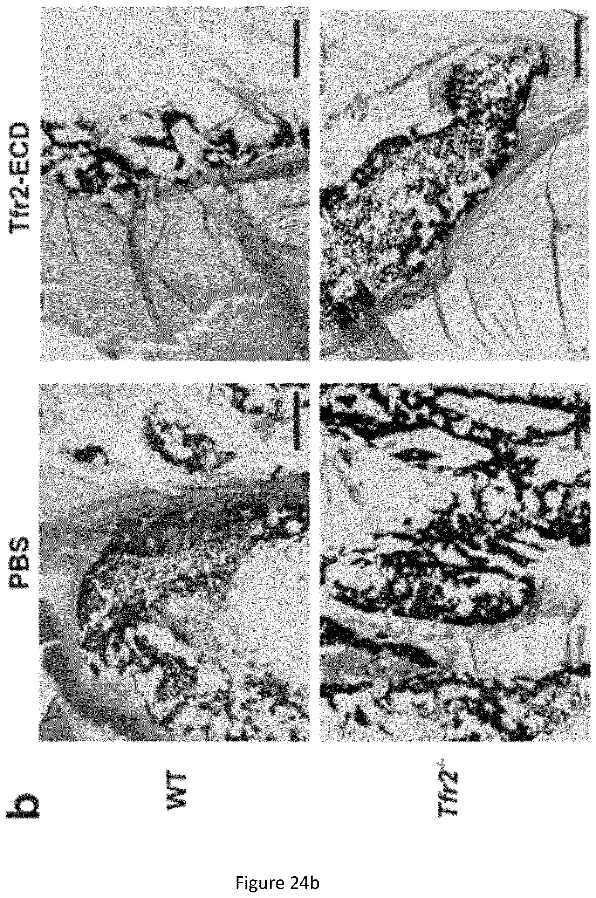

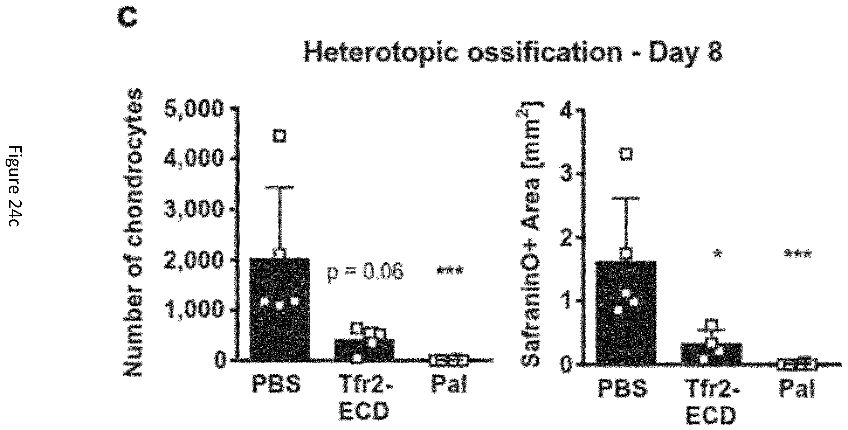

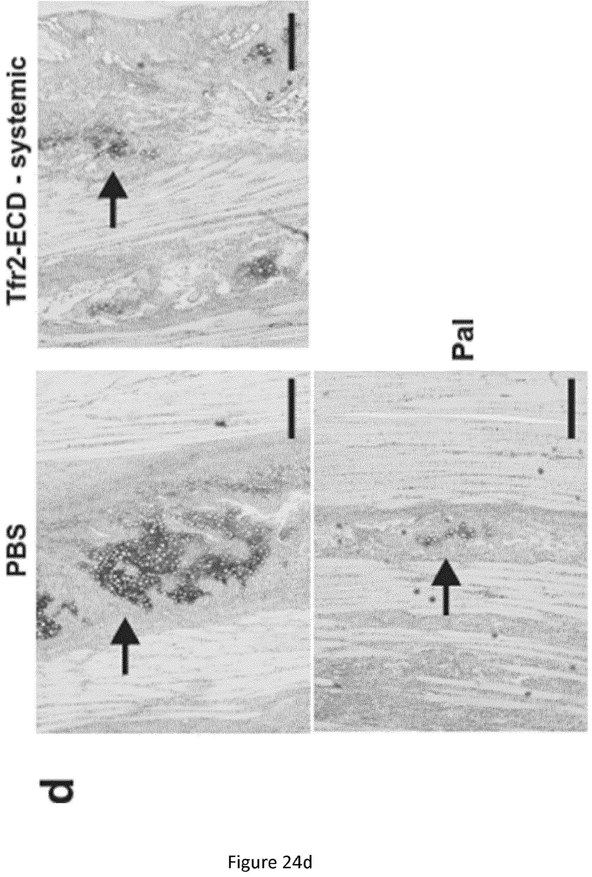

**Figure 24****: Tfr2-ECD blocks heterotopic ossification.** (a-b) Heterotopic ossification was induced by injecting 2.5 µg BMP-2 into the tibialis anterior muscle of WT and *Tfr2*^{*-*/*-*} mice. In some experiments, Tfr2-ECD was additionally injected at equal concentrations as BMP-2. After two weeks, the ossification in the muscle was assessed using (a) HE staining (ossification indicated by an arrow) or (b) von Kossa/van Giemson staining (black areas represent ossification). One representative image per groups is shown (n=3-6 per group). Scale bar: 200µm. (c-d) Heterotopic ossification was induced by injecting 2.5 µg BMP-2 into the tibialis anterior muscle of WT mice. Mice were either treated daily with PBS (i.p. injections every other day), palovarotene (100 µg/mouse orally for the first five days; then 50 µg/mouse for the remainder of the experiment) or with Tfr2-ECD (10 mg/kg BW i.p. for the first 10 days; then 5 mg/kg BW for the remaining time). After 8 days, cartilage production in the muscle was assessed using Safranin O staining. (c) Quantification of the number of chondrocytes (indicated by arrows) and the Safranin O-positive area. (mean±SD, PBS n= 5, Tfr2-ECD n=4, Pal n=6; *p<0.05, ***p<0.001). (d) Representative images. Scale bar: 100 µm. (a-b, d) These experiments were repeated twice with similar results.

### Detailed Description

Unless otherwise defined herein, scientific and technical terms shall have the meanings that are commonly understood by those of ordinary skill in the art. Further, unless otherwise required by context, singular terms shall include pluralities and plural terms shall include the singular. The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin *exempli gratia* and is used herein to indicate a non-limiting example. Thus, the abbreviation "e.g." is synonymous with the term "for example."

In the specification and claims, the term "about" is used to modify, for example, the quantity of an ingredient in a composition, concentration, volume, process temperature, process time, yield, flow rate, pressure, and like values, and ranges thereof, employed in describing the embodiments of the disclosure. The term "about" refers to variation in the numerical quantity that can occur, for example, through typical measuring and handling procedures used for making compounds, compositions, concentrates or use formulations; through inadvertent error in these procedures; through differences in the manufacture, source, or purity of starting materials or ingredients used to carry out the methods, and like proximate considerations. The term "about" also encompasses amounts that differ due to aging of a formulation with a particular initial concentration or mixture, and amounts that differ due to mixing or processing a formulation with a particular initial concentration or mixture. Where modified by the term "about" the claims appended hereto include equivalents to these quantities.

As used herein, "administer" or "administration" refers to the act of injecting or otherwise physically delivering a substance as it exists outside the body (e.g., an anti-Tfr2 antibody provided herein) into a patient, such as by mucosal, intradermal, intravenous, intramuscular delivery and/or any other method of physical delivery described herein or known in the art. When a disease, or a symptom thereof, is being treated, administration of the substance typically occurs after the onset of the disease or symptoms thereof. When a disease, or symptoms thereof, are being prevented, administration of the substance typically occurs before the onset of the disease or symptoms thereof.

The term "antibody", "immunoglobulin" or "Ig" may be used interchangeably herein and means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. As used herein, the term "antibody" encompasses intact polyclonal antibodies, intact monoclonal antibodies, antibody fragments (such as Fab, Fab', F(ab')₂, and Fv fragments), single chain Fv (scFv) mutants, multispecific antibodies such as bispecific antibodies (including dual binding santibodies), chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. The term "antibody" can also refer to a Y-shaped glycoprotein with a molecular weight of approximately 150 kDa that is made up of four polypeptide chains: two light (L) chains and two heavy (H) chains. There are five types of mammalian Ig heavy chain isotypes denoted by the Greek letters alpha (α), delta (δ), epsilon (ε), gamma (γ), and mu (µ). The type of heavy chain defines the class of antibody, i.e., IgA, IgD, IgE, IgG, and IgM, respectively. The γ and α classes are further divided into subclasses on the basis of differences in the constant domain sequence and function, e.g., IgG1, hlgG2, mlgG2A, migG2B, IgG3, IgG4, IgA1 and IgA2. In mammals, there are two types of immunoglobulin light chains, λ and κ. The "variable region" or "variable domain" of an antibody refers to the aminoterminal domains of the heavy or light chain of the antibody. The variable domains of the heavy chain and light chain may be referred to as "V_{H}" and "V_{L}", respectively. These domains are generally the most variable parts of the antibody (relative to other antibodies of the same class) and contain the antigen binding sites. An example of antibodies are heavy chain-only (ie, H2) antibodies that comprise a dimer of a heavy chain (5'-VH-(optional Hinge)-CH2-CH3-3') and are devoid of a light chain.

The antibodies described herein may be oligoclonal, polyclonal, monoclonal (including fulllength monoclonal antibodies), camelised, chimeric, CDR-grafted, multi-specific, bi-specific (including dual-binding antibodies), catalytic, chimeric, humanized, fully human, anti-idiotypic, including antibodies that can be labelled in soluble or bound form as well as fragments, variants or derivatives thereof, either alone or in combination with other amino acid sequences provided by known techniques. An antibody may be from any species. Antibodies described herein can be naked or conjugated to other molecules such as toxins, radioisotopes, etc.

The term "antigen binding site," "antigen binding domain," "antigen binding region," "antigen binding fragment," and similar terms refer to that portion of an antibody which comprises the amino acid residues that interact with an antigen and confer on the binding agent its specificity and affinity for the antigen (e.g. the complementarity determining regions (CDRs)). The antigen binding region can be derived from any animal species, such as rodents (e.g. rabbit, rat or hamster) and humans. Preferably, the antigen binding region will be of human origin.

Antigen binding fragments described herein can include single-chain Fvs (scFv), single- chain antibodies, single domain antibodies, domain antibodies, Fv fragments, Fab fragments, F(ab') fragments, F(ab')₂ fragments, antibody fragments that exhibit the desired biological activity, disulfide- stabilised variable region (dsFv), dimeric variable region (diabody), anti-idiotypic (antild) antibodies (including, e.g. anti-Id antibodies to antibodies), intrabodies, linear antibodies, single-chain antibody molecules and multispecific antibodies formed from antibody fragments and epitope-binding fragments of any of the above. In particular, antibodies and antibody fragments described herein can include immunoglobulin molecules and immunologically active fragments of immunoglobulin molecules, i.e., molecules that contain an antigen-binding site. Digestion of antibodies with the enzyme, papain, results in two identical antigen-binding fragments, known also as "Fab" fragments, and a "Fc" fragment, having no antigen-binding activity but having the ability to crystallize. "Fab" when used herein refers to a fragment of an antibody that includes one constant and one variable domain of each of the heavy and light chains. The term "Fc region" herein is used to define a C-terminal region of an immunoglobulin heavy chain, including native-sequence Fc regions and variant Fc regions. The "Fc fragment" refers to the carboxy-terminal portions of both H chains held together by disulfides. The effector functions of antibodies are determined by sequences in the Fc region, the region which is also recognized by Fc receptors (FcR) found on certain types of cells. Digestion of antibodies with the enzyme, pepsin, results in a F(ab')₂ fragment in which the two arms of the antibody molecule remain linked and comprise two-antigen binding sites. The F(ab')₂ fragment has the ability to crosslink antigen.

"Fv" when used herein refers to the minimum fragment of an antibody that retains both antigenrecognition and antigen-binding sites. This region consists of a dimer of one heavy and one light chain variable domain in tight, non-covalent or covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The term "monoclonal antibody" as used herein refers to an antibody obtained from a population of substantially homogeneous antibodies, i.e. the individual antibodies comprising the population are identical except for possible naturally occurring mutations and/or post-translation modifications (e.g. isomerizations, amidations) that may be present in minor amounts. Monoclonal antibodies are highly specific and are directed against a single antigentic determinant or epitope. In contrast, polyclonal antibody preparations typically include different antibodies directed against different antigenic determinants (or epitopes). The term "monoclonal antibody" as used herein encompasses both intact and full-length monoclonal antibodies as well as antibody fragments (such as Fab, Fab', F(ab')₂, Fv), single chain (scFv) mutants, fusion proteins comprising an antibody portion, and any other modified immunoglobulin molecule comprising an antigen recognition site. Furthermore, "monoclonal antibody" refers to such antibodies made in any number of ways including, but not limited to, hybridoma, phage selection, recombinant expression, and transgenic animals.

The monoclonal antibodies herein can include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies that exhibit the desired biological activity.

The term "humanised antibody" refers to a subset of chimeric antibodies in which a "hypervariable region" from a non-human immunoglobulin (the donor antibody) replaces residues from a hypervariable region in a human immunoglobulin (recipient antibody). In general, a humanized antibody will include substantially all of at least one, and typically two, variable domains, in which all or substantially all of the hypervariable loops correspond to those of a non-human immunoglobulin sequence, and all or substantially all of the framework regions are those of a human immunoglobulin sequence, although the framework regions may include one or more substitutions that improve antibody performance, such as binding affinity, isomerization, immunogenicity, etc.

The term "bispecific antibody" means an antibody which comprises specificity for two target molecules, and includes, but is not limited to, formats such as DVD-Ig (see DiGiammarino et al., "Design and generation of DVD-Ig™ molecules for dual-specific targeting", Meth. Mo. Biol., 2012, 889, 145-156), mAb² (see WO2008/003103, the description of the mAb² format is incorporated herein by reference), FIT-Ig (see WO2015/103072, the description of the FIT-Ig scaffold is incorporated herein by reference), mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH3, Diabody-CH3, Triple body, Miniantibody, minibody, TriBi minibody, scFv-CH3 KIH, scFv-CH-CL-scFv, F(ab')2-scFv, scFv-KIH, Fab-scFv-Fc, tetravalent HCab, ImmTAC, knobs-in-holes, knobs-in-holes with common light chain, knobs-in-holes with common light chain and charge pairs, charge pairs, charge pairs with common light chain, DT-IgG, DutaMab, IgG(H)-scFv, scFv-(H)IgG, IgG(L)-scFv, scFv-(L)IgG, IgG(L,H)-Fv, IgG(H)-V, V(H)-IgG, IgG(L)V, V(L)-IgG, KIH IgG-scFab, 2scFv-IgG, IgG-2scFv, scFv4-Ig and zybody. For a review of bispecific formats, see Spiess, C., et al., Mol. Immunol. (2015). In another embodiment, the bispecific molecule comprises an antibody which is fused to another non-Ig format, for example a T-cell receptor binding domain; an immunoglobulin superfamily domain; an agnathan variable lymphocyte receptor; a fibronectin domain (e.g. an Adnectin^{™}); an antibody constant domain (e.g. a CH₃ domain, e.g., a CH₂ and/or CH₃ of an Fcab^{™}) wherein the constant domain is not a functional CH₁ domain; an scFv; an (scFv)₂; an sc-diabody; an scFab; a centyrin and an epitope binding domain derived from a scaffold selected from CTLA-4 (Evibody^{™}); a lipocalin domain; Protein A such as Z-domain of Protein A (e.g. an Affibody^{™} or SpA); an A-domain (e.g. an Avimer^{™} or Maxibody^{™}); a heat shock protein (such as and epitope binding domain derived from GroEl and GroES); a transferrin domain (e.g. a trans-body); ankyrin repeat protein (e.g. a DARPin^{™}); peptide aptamer; C-type lectin domain (e.g. Tetranectin^{™}); human γ- crystallin or human ubiquitin (an affilin); a PDZ domain; scorpion toxin; and a kunitz type domain of a human protease inhibitor.

In one embodiment, the bispecific antibody is a mAb². A mAb² comprises a V_{H} and V_{L} domain from an intact antibody, fused to a modified constant region, which has been engineered to form an antigen-binding site, known as an "Fcab". The technology behind the Fcab/mAb² format is described in more detail in WO2008/003103, and the description of the mAb² format is incorporated herein by reference.

In another embodiment, the bispecific antibody is a "dual binding antibody". As used herein, the term "dual binding antibody" is a bispecific antibody wherein both antigen-binding domains are formed by a V_{H}/V_{L} pair, and includes FIT-Ig (see WO2015/103072, incorporated herein by reference), mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH3, Diabody-CH3, Triple body, Miniantibody, minibody, scFv-CH₃ KIH, scFv-CH-CL-scFv, F(ab')₂-scFv, scFv-KIH, FabscFv-Fc, tetravalent HCab, ImmTAC, knobs-in-holes, knobs-in-holes with common light chain, knobs-in-holes with common light chain and charge pairs, charge pairs, charge pairs with common light chain, DT-IgG, DutaMab, IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv and scFv4-Ig.

The term "hypervariable region", "CDR region" or "CDR" refers to the regions of an antibody variable domain which are hypervariable in sequence and/or form structurally defined loops. Generally, antigen binding sites of an antibody include six hypervariable regions: three in the V_{H} (CDRH1, CDRH2, CDRH3), and three in the V_{L} (CDRL1, CDRL2, CDRL3). These regions of the heavy and light chains of an antibody confer antigen-binding specificity to the antibody. CDRs may be defined according to the Kabat system (see Kabat, E. A.et al., 1991, "Sequences of Proteins of Immunological Interest", 5th edit., NIH Publication no. 91-3242, U.S. Department of Health and Human Services). Other systems may be used to define CDRs, which as the system devised by Chothia *et al* (see Chothia, C. & Lesk, A. M., 1987, "Canonical structures for the hypervariable regions of immunoglobulins", J. Mol. Biol., 196, 901-917) and the IMGT system (see Lefranc, M. P., 1997, "Unique database numbering system for immunogenetic analysis", Immunol. Today, 18, 50). An antibody typically contains 3 heavy chain CDRs and 3 light chain CDRs. The term CDR or CDRs is used here to indicate one or several of these regions. A person skilled in the art is able to readily compare the different systems of nomenclature and determine whether a particular sequence may be defined as a CDR.

An inhibitor herein may, for example, be a human antibody. A "human antibody" is an antibody that possesses an amino-acid sequence corresponding to that of an antibody produced by a human and/or has been made using any of the techniques for making human antibodies and specifically excludes a humanized antibody comprising non- human antigen-binding residues. The term "specifically binds to" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In one embodiment, the extent of binding of an antibody to an unrelated target is less than about 10% of the binding of the antibody to the target as measured, e.g. by a radioimmunoassay (RIA).

An antibody or a fragment thereof that specifically binds to a Tfr2 antigen may be cross-reactive with related antigens. Preferably, an antibody or a fragment thereof that specifically binds to a Tfr2 antigen does not cross-react with other antigens (but may optionally cross-react with Tfr2 of a different species, e.g. rhesus, or murine). An antibody or a fragment thereof that specifically binds to a Tfr2 antigen can be identified, for example, by immunoassays, BIAcore^{™}, or other techniques known to those of skill in the art. An antibody or a fragment thereof binds specifically to a Tfr2 antigen when it binds to a Tfr2 antigen with higher affinity than to any cross-reactive antigen as determined using experimental techniques, such as radioimmunoassays (RIA) and enzyme-linked immunosorbent assays (ELISAs). Typically, a specific or selective reaction will be at least twice background signal or noise and more typically more than 10 times (such as more than 15 times, more than 20 times, more than 50 times or more than 100 times) background. See, e.g. Paul, ed., 1989, Fundamental Immunology Second Edition, Raven Press, New York at pages 332-336 for a discussion regarding antibody specificity.

As used herein, "authorization number" or "marketing authorization number" refers to a number issued by a regulatory agency upon that agency determining that a particular medical product and/or composition may be marketed and/or offered for sale in the area under the agency's jurisdiction. As used herein "regulatory agency" refers to one of the agencies responsible for evaluating, e.g. the safety and efficacy of a medical product and/or composition and controlling the sales/marketing of such products and/or compositions in a given area. The Food and Drug Administration (FDA) in the US and the European Medicines Agency (EPA) in Europe are but two examples of such regulatory agencies. Other non-limiting examples can include SDA, MPA, MHPRA, IMA, ANMAT, Hong Kong Department of Health-Drug Office, CDSCO, Medsafe, and KFDA.

As used herein, a "buffer" refers to a chemical agent that is able to absorb a certain quantity of acid or base without undergoing a strong variation in pH.

The composition of the invention may comprise a carrier. As used in this context, the term "carrier" refers to a diluent, adjuvant (e.g., Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

As used herein, the term "composition" is intended to encompass a product containing the specified ingredients (e.g. an antibody of the invention) in, optionally, the specified amounts, as well as any product which results, directly or indirectly, from combination of the specified ingredients in, optionally, the specified amounts.

In an example the inhibitor or protein of the invention comprise an antibody constant region with effector function. The term "effector function" (or "effector-enabled") as used herein refers to one or more of antibody dependant cell mediated cytotoxic activity (ADCC), complementdependant cytotoxic activity (CDC) mediated responses, Fc-mediated phagocytosis or antibody dependant cellular phagocytosis (ADCP) and antibody recycling via the FcRn receptor.

An "effective amount" refers to an amount effective, at dosages and for periods of time necessary, to achieve the desired effect, including a therapeutic or prophylactic result. A "therapeutically effective amount" refers to the minimum concentration required to effect a measurable improvement or prevention of a particular disorder. A therapeutically effective amount herein may vary according to factors such as the disease state, age, sex, and weight of the patient, and the ability of the antibody to elicit a desired response in the individual. A therapeutically effective amount is also one in which toxic or detrimental effects of the antibody are outweighed by the therapeutically beneficial effects. A "prophylactically effective amount" refers to an amount effective, at the dosages and for periods of time necessary, to achieve the desired prophylactic result. In some embodiments, the effective amount of an antibody of the invention is from about 0.1 mg/kg (mg of antibody per kg weight of the subject) to about 100 mg/kg. In certain embodiments, an effective amount of an antibody provided therein is about 0.1 mg/kg, about 0.5 mg/kg, about 1 mg/kg, 3 mg/kg, 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg about 90 mg/kg or about 100 mg/kg (or a range therein). In some embodiments, "effective amount" as used herein also refers to the amount of an antibody of the invention to achieve a specified result (e.g. inhibition of a Tfr2 biological activity of a cell).

The term "epitope" as used herein refers to a localized region on the surface of an antigen, such as Tfr2 polypeptide or Tfr2 polypeptide fragment, that is capable of being bound to one or more antigen binding regions of an antibody, and that has antigenic or immunogenic activity in an animal, preferably a mammal, and most preferably in a human, that is capable of eliciting an immune response. An epitope having immunogenic activity is a portion of a polypeptide that elicits an antibody response in an animal. An epitope having antigenic activity is a portion of a polypeptide to which an antibody specifically binds as determined by any method well known in the art, for example, by the immunoassays described herein. Antigenic epitopes need not necessarily be immunogenic. Epitopes usually consist of chemically active surface groupings of molecules such as amino acids or sugar side chains and have specific three-dimensional structural characteristics as well as specific charge characteristics. A region of a polypeptide contributing to an epitope may be contiguous amino acids of the polypeptide or the epitope may come together from two or more non-contiguous regions of the polypeptide. The epitope may or may not be a three-dimensional surface feature of the antigen. In certain embodiments, a Tfr2 epitope is a three-dimensional surface feature of a Tfr2 polypeptide. In other embodiments, a Tfr2 epitope is linear feature of a Tfr2 polypeptide.

In an example, the composition herein comprises an excipient. The term "excipient" as used herein refers to an inert substance which is commonly used as a diluent, vehicle, preservatives, binders, or stabilizing agent for drugs and includes, but not limited to, proteins (e.g. serum albumin, etc.), amino acids (e.g. aspartic acid, glutamic acid, lysine, arginine, glycine, histidine, etc.), fatty acids and phospholipids (e.g. alkyl sulfonates, caprylate, etc.), surfactants (e.g. SDS, polysorbate, nonionic surfactant, etc.), saccharides (e.g. sucrose, maltose, trehalose, etc.) and polyols (e.g. mannitol, sorbitol, etc.). See, also, Remington's Pharmaceutical Sciences (1990) Mack Publishing Co., Easton, Pa., which is hereby incorporated by reference in its entirety.

An antibody or fragment herein may comprise a heavy chain as described in this paragraph. The term "heavy chain" when used with reference to an antibody refers to five distinct types, called alpha (α), delta (δ), epsilon (ε), gamma (γ) and mu (µ), based on the amino acid sequence of the heavy chain constant domain. These distinct types of heavy chains are well known and give rise to five classes of antibodies, IgA, IgD, IgE, IgG and IgM, respectively, including four subclasses of IgG, namely IgG1, IgG2, IgG3 and IgG4. Preferably the heavy chain is a human heavy chain. In the human population, multiple heavy chain constant region alleles, of each immunoglobulin or immunoglobulin subclass, exist. The nucleotide and amino acid sequences of these allelic variants are accessible on publicly available databases such as IMGT, ENSEMBL Swiss-Prot and Uniprot. Allelic variants may also be identified in various genome sequencing projects. In one embodiment, the antibodies and antibody fragments disclosed herein comprise a heavy chain encoded by a IgG1 constant region allele, which includes, but is not limited to, human IGHG1*01, IGHG1*02, IGHG1*03, IGHG1*04 and IGHG1*05. In one embodiment, the antibodies and antibody fragments disclosed herein comprise a protein encoded by a IgG2 constant region allele, which includes, but is not limited to, human IGHG2*01, IGHG2*02, IGHG2*03, IGHG2*04, IGHG2*05 and IGHG2*06. In one embodiment, the antibodies or antibody fragments disclosed herein comprise a protein encoded by a IgG3 constant region allele, which includes but is not limited to human IGHG3*01, IGHG3*02, IGHG3*03, IGHG3*04, IGHG3*05, IGHG3*06, IGHG3*07, IGHG3*08, IGHG3*09, IGHG3*10, IGHG3*11, IGHG3*12, IGHG3*13, IGHG3*14, IGHG3*15, IGHG3*16, IGHG3*17, IGHG3*18 and IGHG3*19. In one embodiment, the antibodies or antibody fragments disclosed herein comprise a protein encoded by a IgG4 constant region allele, which includes but is not limited to human IGHG4*01, IGHG4*02, IGHG4*03 and IGHG4*04. In another example, the heavy chain is a disabled IgG isotype, e.g. a disabled IgG4. In certain embodiments, the antibodies of the invention comprise a human gamma 4 constant region. In another embodiment, the heavy chain constant region does not bind Fc-γ receptors, and e.g. comprises a Leu235Glu mutation. In another embodiment, the heavy chain constant region comprises a Ser228Pro mutation to increase stability. In another embodiment, the heavy chain constant region is IgG4-PE (see, eg, the sequence table herein). In another embodiment, the antibodies and antibody fragments disclosed herein comprise a heavy chain constant region encoded by a murine IgG1 constant region allele, which includes but is not limited to mouse IGHG1*01 or IGHG1*02. In one embodiment, the antibodies and antibody fragments disclosed herein comprise a heavy chain constant region encoded by a murine IgG2 constant region allele, which includes, but is not limited to, mouse IGHG2A*01, IGHG2A*02, IGHG2B*01, IGHG2B*02, IGHG2C*01, IGHG2C*02 or IGHG2C*03. In one embodiment, the antibodies or antibody fragments disclosed herein comprise a protein encoded by a murine IgG3 constant region allele, which includes but is not limited to mouse IGHG3*01.

The protein, inhibitor or composition of the invention may be administered to the subject in combination with another therapy. The term "in combination" in the context of the administration of other therapies refers to the use of more than one therapy. The use of the term "in combination" does not restrict the order in which therapies are administered to a subject with a disease. A first therapy can be administered before (e.g. 1 minute, 45 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks), concurrently, or after (e.g. 1 minute, 45 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks) the administration of a second therapy to a subject which had, has, or is susceptible to a Tfr2-mediated disease. Any additional therapy can be administered in any order with the other additional therapies. In certain embodiments, the antibodies of the invention can be administered in combination with one or more therapies (e.g. therapies that are not the antibodies of the invention that are currently administered to prevent, treat, manage, and/or ameliorate a Tfr2-mediated disease. Non-limiting examples of therapies that can be administered in combination with an antibody of the invention include analgesic agents, anaesthetic agents, antibiotics, or immunomodulatory agents or any other agent listed in the U.S. Pharmacopoeia and/or Physician's Desk Reference.

As used herein, "injection device" refers to a device that is designed for carrying out injections, an injection including the steps of temporarily fluidically coupling the injection device to a person's tissue, typically the subcutaneous tissue. An injection further includes administering an amount of liquid drug into the tissue and decoupling or removing the injection device from the tissue. In some embodiments, an injection device can be an intravenous device or IV device, which is a type of injection device used when the target tissue is the blood within the circulatory system, e.g. the blood in a vein. A common, but non-limiting example of an injection device is a needle and syringe.

As used herein, "instructions" refers to a display of written, printed or graphic matter on the immediate container of an article, for example the written material displayed on a vial containing a pharmaceutically active agent, or details on the composition and use of a product of interest included in a kit containing a composition of interest. Instructions set forth the method of the treatment as contemplated to be administered or performed.

The terms "Kabat numbering," and like terms are recognized in the art and refer to a system of numbering amino acid residues which are more variable (i.e. hypervariable) than other amino acid residues in the heavy chain variable regions of an antibody, or an antigen binding portion thereof (Kabat et al., (1971) Ann. NY Acad. Sci., 190:382-391 and, Kabat et al. (1991) Sequences of Proteins of Immunological Interest, Fifth Edition, U.S. Department of Health and Human Services, NIH Publication No. 91-3242). For the heavy chain variable region, the hypervariable region typically ranges from amino acid positions 31 to 35 for CDR1, amino acid positions 50 to 65 for CDR2, and amino acid positions 95 to 102 for CDR3.

The antibody, protein or inhibitor of the invention may comprise a light chain as described in this paragraph. The term "light chain" when used in reference to an antibody refers to the immunoglobulin light chains, of which there are two types in mammals, lambda (λ) and kappa (κ). Preferably, the light chain is a human light chain. Preferably the light chain constant region is a human constant region. In the human population, multiple light chain constant region alleles exist. The nucleotide and amino acid sequences of these allelic variants are accessible on publicly available databases such as IMGT, ENSEMBL, Swiss-Prot and Uniprot. In one embodiment, the antibodies or antibody fragments disclosed herein comprise a protein encoded by a human κ constant region allele, which includes, but is not limited to, IGKC*01 (see, eg, the sequence table herein), IGKC*02 (see, eg, the sequence table herein), IGKC*03 (see, eg, the sequence table herein), IGKC*04 (see, eg, the sequence table herein) and IGKC*05 (see, eg, the sequence table herein). In one embodiment, the antibodies or antibody fragments disclosed herein comprise a protein encoded by a human λ constant region allele, which includes but is not limited to IGLC1*01 (see, eg, the sequence table herein), IGLC1*02 (see, eg, the sequence table herein), IGLC2*01 (see, eg, the sequence table herein), IGLC2*02 (see, eg, the sequence table herein), IGLC2*03 (see, eg, the sequence table herein), IGLC3*01 (see, eg, the sequence table herein), IGLC3*02 (see, eg, the sequence table herein), IGLC3*03 (see, eg, the sequence table herein), IGLC3*04 (see, eg, the sequence table herein), IGLC6*01 (see, eg, the sequence table herein), IGLC7*01 (see, eg, the sequence table herein), IGLC7*02 (see, eg, the sequence table herein), IGLC7*03 (see, eg, the sequence table herein). In another embodiment, the antibodies and antibody fragments disclosed herein comprise a light chain constant region encoded by a mouse κ constant region allele, which includes, but is not limited to, IGKC*01, IGKC*03 or IGKC*03. In another embodiment, the antibodies and antibody fragments disclosed herein comprise a light chain constant region encoded by a mouse λ constant region allele, which includes, but is not limited to, IGLC1*01, IGLC2*01 or IGLC3*01.

The subject may be any animal, including, but not limited to, mammals. As used herein, the term "mammal" refers to any vertebrate animal that suckle their young and either give birth to living young (eutharian or placental mammals) or are egg-laying (metatharian or nonplacental mammals). Examples of mammalian species include, but are not limited to, humans and other primates, including non-human primates such as chimpanzees and other apes and monkey species; farm animals such as cattle, sheep, pigs, goats and horses; domestic mammals such as dogs and cats; laboratory animals including rodents such as mice, rats (including cotton rats) and guinea pigs; birds, including domestic, wild and game birds such as chickens, turkeys and other gallinaceous birds, ducks, geese, and the like.

As used herein "substantially all" refers to refers to at least about 60%, at least about 70%, at least about 75%, at least about 80%, at least about 85%, at least about 90%, at least about 95%, at least about 98%, at least about 99%, or about 100%.

The term "variable region" or "variable domain" refers to a portion of the light and heavy chains, typically about the amino-terminal 120 to 130 amino acids in the heavy chain and about 100 to 110 amino acids in the light chain, which differ extensively in sequence among antibodies and are used in the binding and specificity of each particular antibody for its particular antigen. The variability in sequence is concentrated in those regions called complimentarily determining regions (CDRs) while the more highly conserved regions in the variable domain are called framework regions (FR). The CDRs of the heavy chains are primarily responsible for the interaction of the antibody with antigen. Numbering of amino acid positions used herein is according to the EU Index, as in Kabat et al. (1991) Sequences of proteins of immunological interest. (U.S. Department of Health and Human Services, Washington, D.C.) 5th ed. ("Kabat et *al*."). In preferred embodiments, the variable region is a human variable region.

Definitions of common terms in cell biology and molecular biology can be found in "The Merck Manual of Diagnosis and Therapy", 19th Edition, published by Merck Research Laboratories, 2006 (ISBN 0-911910-19-0); Robert S. Porter et al. (eds.), The Encyclopedia of Molecular Biology, published by Blackwell Science Ltd., 1994 (ISBN 0-632-02182-9); Benjamin Lewin, Genes X, published by Jones & Bartlett Publishing, 2009 (ISBN-10: 0763766321); Kendrew et al. (Eds.), Molecular Biology and Biotechnology: a Comprehensive Desk Reference, published by VCH Publishers, Inc., 1995 (ISBN 1-56081-569-8) and Current Protocols in Protein Sciences 2009, Wiley Intersciences, Coligan et al., eds.

Unless otherwise stated, the present invention was performed using standard procedures, as described, for example in Sambrook et al., Molecular Cloning: A Laboratory Manual (4 ed.), Cold Spring Harbor Laboratory Press, Cold Spring Harbor, N.Y., USA (2012); Davis et al., Basic Methods in Molecular Biology, Elsevier Science Publishing, Inc., New York, USA (1995); or Methods in Enzymology: Guide to Molecular Cloning Techniques Vol.152, S. L. Berger and A. R. Kimmel Eds., Academic Press Inc., San Diego, USA (1987); Current Protocols in Protein Science (CPPS) (John E. Coligan, et al., ed., John Wiley and Sons, Inc.), Current Protocols in Cell Biology (CPCB) (Juan S. Bonifacino et al. ed., John Wiley and Sons, Inc.), and Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998) which are all incorporated by reference herein in their entireties.

Other terms are defined herein within the description of the various aspects of the invention.

### First Configuration: Use of extracellular domains of transferrin receptor 2 for diagnosing and treating primary or secondary sclerosing diseases

In a first configuration, the invention relates to a protein for use in diagnosing and/or treating primary or secondary sclerosing diseases, a fusion protein, and nucleotide sequence and a vector, and to a pharmaceutical composition for use in diagnosing and treating primary or secondary sclerosing diseases.

There are numerous sclerosing diseases that are associated with uncontrolled bone formation, including *Fibrodysplasia ossificans progressiva* (FOP). This rare disease is characterized by heterotopic ossification (HO) which leads to ossification outside of the skeleton, in particular of muscles, tendons and soft parts, and thus greatly impairs the mobility of patients. FOP sufferers have an average life expectancy of 56 years and the cause of death is often the inability of the thorax to support normal respiration. FOP patients have a mutation in the ACVR1 gene, which codes for the ACVR1/ALK2 receptor. This receptor is part of the bone morphogenetic protein (BMP) signaling pathway and is of decisive importance in the regulation of cartilage and bone development. This mutation leads to increased activity of the ACVR1/ALK2 receptor and thus to excessive BMP signaling, resulting in increased and uncontrolled bone formation (Shore and Kaplan 2008).

Other sclerosing diseases exist, in addition to FOP, and result from different mechanisms. These include van Buchem disease, sclerosteosis, melorheostosis, pachydermoperiostosis, fibrous dysplasia, osteochondrodysplasia, mucopolysaccharidosis, ankylosing spondylitis, post-traumatic HO, e.g. after joint replacement operations, explosions, amputations, paraplegia, or after calciphylaxis or in the case of malignant diseases or degenerative diseases, e.g. prostate carcinomas, renal cell carcinomas, tumoral calcinosis, breast carcinomas, arthrosis or benign bone lesions. These sclerosing diseases are characterized by uncontrolled ossification within and outside of the skeleton. The invention may be used to treat any of these diseases. The invention may be used to diagnose any of these diseases. The invention may be used to prevent or reduce the risk of any of these diseases.

Conventional methods for treating sclerosing diseases comprise non-specific treatments, steroids, non-steroidal anti-inflammatory drugs (NSAIDs), resection or radiotherapy (Kölbl et al. 2003). WO 2016/039796 A2 describes a method for treating the sclerosing disease *Fibrodysplasia ossificans progressiva* (FOP), comprising the administration of activin receptor type 2A (ACVR2A) antagonist and/or activin receptor type 2B (ACVR2B) antagonist or activin receptor type 1 (ACVR1) antagonist.

However, the applications of these treatments are limited and usually only symptom-relieving, but cannot prevent the disease from progressing. For example, following resection, the likelihood of HO returning is up to 80%. Steroids in particular cause inhibition of bone formation but have a large number of side effects, such as obesity, diabetes, dry skin or muscle wasting.

WO 2015/107075 A1 discloses the human amino acid sequence of the extracellular domains of transferrin receptor 2 (SEQ ID NO. 1 herein). Baschant et al. describe the iron-dependent, cellintrinsic negative regulation of osteoclast formation using transferrin receptor 2 (Baschant et al. 2017).

The object of the present invention is therefore that of providing a drug for treating sclerosing diseases.

The object of the invention is furthermore that of providing a drug that has fewer side effects than known treatment methods.

The object is achieved according to the invention by using a protein having an amino acid sequence that has at least 70, 75, 80, 85, 90, 95, 96, 97, 98 or 99% identity with the sequence of SEQ ID NO. 1, or the fragments thereof, for use in diagnosing and treating primary or secondary sclerosing diseases.

"Identity" is to be understood as the number of matching amino acids based on the total number of amino acids.

A "fragment" in relation to the protein of the invention is to be understood as a portion of the amino acid sequence of the protein, preferably a fragment consisting of the PA domains (SEQ ID NO. 5, or a sequence that has at least 70, 75, 80, 85, 90, 95, 96, 97, 98 or 99% identity thereto), a fragment consisting of the peptidase M28 domains (SEQ ID NO. 6, or a sequence that has at least 70, 75, 80, 85, 90, 95, 96, 97, 98 or 99% identity thereto), or a fragment consisting of the Tfr-like dimerization domains (SEQ ID NO. 7, or a sequence that has at least 70, 75, 80, 85, 90, 95, 96, 97, 98 or 99% identity thereto).

"Primary or secondary sclerosing diseases" are to be understood as diseases associated with ossification of tissue, the sclerosis occurring as a primary or secondary consequence of the disease.

Primary or secondary sclerosing diseases comprise *Fibrodysplasia ossificans progressiva* (FOP), van Buchem disease, sclerosteosis, melorheostosis, pachydermoperiostosis, fibrous dysplasia, osteochondrodysplasia, mucopolysaccharidosis, ankylosing spondylitis, post-traumatic HO, preferably in the case of scleroses after joint replacement operations, explosions, amputations, paraplegia, calciphylaxis or in the case of malign diseases or degenerative diseases, particularly preferably in the case of prostate carcinomas, renal cell carcinomas, tumoral calcinosis, breast carcinomas, arthrosis and benign bone lesions.

In a preferred embodiment, the use is in diagnosing and/or treating heterotopic ossification (HO), van Buchem disease, sclerosteosis or *Fibrodysplasia ossificans progressiva* (FOP).

"HO," also known as *Myositis ossificans,* is to be understood as a disease in which ossification of the soft part tissue outside of the skeletal system occurs as a result of tissue injury.

*"Fibrodysplasia ossificans progressiva* (FOP)," also known as *Fibrodysplasia ossificans multiplex progressiva, Myositis ossificans progressiva* or Munchmeyer's disease, is to be understood as a genetic disease in which progressive ossification of the connective and supporting tissue of the human body occurs.

"Van Buchem disease," also known as van Buchem syndrome, *sclerosteosis, Hyperostosis corticalis generalisata familiaris,* van Buchem-type *endosteal hyperostosis* is to be understood as a hereditary skeletal dysplasia comprising hyperplasia of the long bones and the skullcap, which disease is autosomal recessive.

"Fibrous dysplasia" is to be understood as a disease that is caused by a mutation in the GNAS gene and leads to bone excrescences.

"Melorheostosis" is to be understood as a disease that is caused by a mutation in the LEMD3 gene and leads to bone excrescences. This gene codes for a protein that is involved in the transforming growth factor-β (TGF-β) signaling pathway.

"Mucopolysaccharidosis" is to be understood as a group of lysosomal storage diseases that is autosomal recessive and leads to bone changes.

"Ankylosing spondylitis," or Bechterew's disease, Marie-Strumpell disease, ankylosing spondyloarthritis is a chronic inflammatory disease that is preferably manifested in the spinal column and in the sacroiliac joint. In this case, ankylosis and stiffening often occurs in the spinal column.

The protein having an amino acid sequence SEQ ID NO. 1 can be isolated from the human transferrin receptor (Tfr) 2α, or the human transferrin receptor (Tfr) 2β, preferably the extracellular domains of human Tfr2a.

The protein according to the invention preferably binds members of the transforming growth factor-β (TGF-β)/bone morphogenetic proteins BMP families, preferably BMPs, particularly preferably binds to one or more of BMP-2, BMP-4, BMP-6 and BMP-7, preferably binds to all of these BMPs.

"Transforming growth factor-β (TGF-β)/bone morphogenetic protein, BMP families," is to be understood as a group of similar signaling proteins that bind members of the TGF-β receptor families. The TGF-β/BMP family comprises TGFβ1, TGFβ2, TGFβ3, BMPs, growth differentiation factors (GDFs), activin and inhibin, myostatin, anti-Müllerian hormone (AMH) and nodal.

"Bone morphogenetic proteins (BMPs)" are to be understood as a group of paracrine signaling proteins that bind BMP receptors. In an embodiment, BMPs are selected from BMP1, BMP2, BMP3, BMP4, BMP5, BMP6, BMP7, BMP8a, BMP8b, BMP-9, BMP10 or BMP15, preferably BMP-2, BMP-4, BMP-6 or BMP-7.

Advantageously, when treating primary or secondary sclerosing diseases using the protein according to the invention, the BMP signaling pathway, and thus the bone formation and the Wnt signaling pathway is specifically inhibited.

In an embodiment, the diagnosis of primary or secondary sclerosing diseases using the protein according to the invention is carried out by detecting members of the TGF-β/BMP family, preferably BMPs, particularly preferably one or more of BMP-2, BMP-4, BMP-6 and BMP-7, preferably all of these BMPs.

In an embodiment, the diagnosis of primary or secondary sclerosing diseases using the protein according to the invention is carried out in the blood, in the blood plasma, in the blood serum or in the tissue. In a preferred embodiment, the tissue is bone or cartilage. "Blood plasma" is to be understood as the fluid component of the blood. "Blood serum" is to be understood as the blood plasma without the clotting factor.

In an embodiment, the diagnosis of primary or secondary sclerosing diseases using the protein according to the invention is carried out by means of an immunoassay. An "immunoassay" is to be understood as a detection procedure in which an analyte is detected in a fluid phase by means of antigen-antibody binding.

In an embodiment, the immunoassay is selected from an Enzyme-linked Immunosorbent Assay (ELISA) or Enzyme-linked Immuno Spot Assay (ELIspot Assay). An "ELISA" is to be understood as an antibody-based detection procedure that is based on an enzymatic color reaction. An "ELIspot Assay" is to be understood as a detection procedure for detecting antibodies that are secreted by immune cells following stimulation using antigens and are immobilized on a membrane.

In an embodiment, the diagnosis of primary or secondary sclerosing diseases is carried out in order to assess the prognosis of the disease, to assess the response to treatment and/or for risk stratification. "Risk stratification" is to be understood as assessing the risk of a disease progressing or leading to complications or death.

In an embodiment, the protein according to the invention for use in diagnosing and treating primary or secondary sclerosing diseases comprises sequence SEQ ID NO. 1 or SEQ ID NO. 2.

The protein having an amino acid sequence SEQ ID NO. 2 can be isolated from the murine transferrin receptor (Tfr) 2α, or the murine transferrin receptor (Tfr) 2β, preferably the extracellular domains of murine Tfr2α.

In an embodiment, the protein according to the invention comprises or consists of 232 amino acids to 801 amino acids, preferably 487 amino acids to 801 amino acids, particularly preferably 600 amino acids to 750 amino acids.

In an embodiment, the protein according to the invention for use in diagnosing and/or treating primary or secondary sclerosing diseases is the human transferrin receptor (Tfr) 2α (SEQ ID NO. 3), the murine transferrin receptor (Tfr) 2α (SEQ ID NO. 4), the human transferrin receptor (Tfr) 2β (SEQ ID NO. 1) or the extracellular domains of human Tfr2α (SEQ ID NO. 1), the murine transferrin receptor (Tfr) 2β (SEQ ID NO. 2) or the extracellular domains of murine Tfr2α (SEQ ID NO. 2).

The invention further relates to a fusion protein comprising at least one protein according to the invention for use in diagnosing and/or treating primary or secondary sclerosing diseases.

In an embodiment, the fusion protein comprises at least one protein tag. In an embodiment, the at least one protein tag is selected from a polyhistidine (His) tag, glutathione S-transferase (GST) tag, maltose binding protein (MBP) tag, Myc tag, streptavidin (Strep) tag or a dye, preferably a fluorescent dye, particularly preferably a green fluorescent protein (GFP) or a yellow fluorescent protein (YFP).

In an embodiment, the protein according to the invention or the fusion protein comprising at least one protein according to the invention comprises at least one modification.

In an embodiment, the at least one modification is selected from proteins containing D-amino acids, pseudopeptide bonds, amino alcohols, non-proteinogenic amino acids, amino acids having modified side groups and/or circular proteins.

Proteins comprising modifications are advantageously more stable.

In an embodiment, the protein according to the invention or the fusion protein comprising at least one protein according to the invention is used in the treatment of a disease associated with increased BMP receptor activation.

The invention further relates to a nucleotide sequence comprising a sequence coding for a protein according to the invention or a fusion protein comprising at least one protein according to the invention for use in diagnosing and/or treating primary or secondary sclerosing diseases.

In an embodiment, the nucleotide sequence comprises SEQ ID NO. 8 or SEQ ID NO. 9.

A further aspect of the invention relates to a vector for use in diagnosing and/or treating primary or secondary sclerosing diseases, comprising a nucleotide sequence comprising a sequence coding for a protein according to the invention or a fusion protein comprising at least one protein according to the invention.

A "vector" is to be understood as a nucleic acid carrier for transferring a nucleic acid into a cell by means of transfection or transduction. In an embodiment, vectors are selected from plasmids, viral vectors or other nucleic acid carriers that contain a nucleotide sequence comprising a sequence coding for a protein according to the invention or a fusion protein comprising at least one protein according to the invention by means of genetic recombination (recombinant).

The invention further relates to a pharmaceutical composition for use in treating primary or secondary sclerosing diseases, comprising at least one protein according to the invention or a fusion protein comprising at least one protein according to the invention.

In an embodiment, the pharmaceutical composition is a solution, tablet or capsule. In an embodiment, the protein according to the invention is used as a coating for implant materials, preferably metals or plastics materials, and/or implants, preferably protheses, screws or nails.

In an embodiment, the pharmaceutical composition is administered locally in an intraarticularly or intramuscularly, or systemically or subcutaneously or intravenously, or orally. In an embodiment, the pharmaceutical composition is in a suitable form for intraarticular, intramuscular, subcutaneous, intravenous or oral administration.

In an embodiment, the pharmaceutical composition contains the protein according to the invention or the fusion protein comprising at least one protein according to the invention in a dose of from 10 µg/kg to 100 mg/kg body weight per administration.

In a further embodiment, the pharmaceutical composition furthermore contains a pharmaceutically acceptable diluent or base. In an embodiment, the pharmaceutically acceptable diluent or base is an aqueous solution, preferably a buffered aqueous solution, an aqueous saline solution or an aqueous glycine solution. In an embodiment, the buffered aqueous solution is selected from a histidine-buffered aqueous solution having a pH of from pH 5.0 to pH 7.0, or a sodium succinate-, sodium citrate-, sodium phosphate-, or potassium phosphate-buffered aqueous solution. In an embodiment, the buffered aqueous solution has a concentration of from 1 mmol/I (mM) to 500 mM, preferably 1 mM to 50 mM. In a further embodiment, the pharmaceutically acceptable diluent or base comprises sodium chloride, preferably in a concentration of between 0 mM and 300 mM, particularly preferably in a concentration of 150 mM.

In an embodiment, the pharmaceutical composition furthermore comprises at least one pharmaceutically acceptable excipient. An "excipient" is understood to be a compound that adjusts physiological conditions with regard to the pH and/or the ionic strength, and/or increases the stability of the pharmaceutical composition. In an embodiment, the at least one pharmaceutically acceptable excipient is selected from sodium acetate, sodium chloride, potassium chloride, calcium chloride or sodium lactate.

In an embodiment, the pharmaceutical composition is sterile. The pharmaceutical composition is sterilized by means of known methods.

A further aspect of the invention relates to the use of the pharmaceutical composition in diagnosing and treating primary or secondary sclerosing diseases.

In an embodiment, the use of the pharmaceutical composition is for administration to a subject. A "subject" is to be understood as an individual or a patient. In an embodiment, the subject is selected from humans or animals, eg a male human, a female human, an adult human or a child human. In an embodiment, are selected from rodents, preferably mice, rats, hamsters or guinea pigs; dogs, rabbits, farm animals, preferably goats, sheep, pigs; and nonhuman primates, preferably chimpanzees, orangutans or gorillas. In an example, the human has received a prosthetic implant, eg, has received a hip transplant.

In an embodiment, the pharmaceutical composition is used in diagnosing members of the TGFβ/BMP family and treating diseases associated with increased BMP receptor activation.

The invention further relates to a method for diagnosing and/or treating primary or secondary sclerosing diseases, comprising administering the protein according to the invention and/or the pharmaceutical composition.

In an embodiment, the diagnosis and/or treatment of primary or secondary sclerosing diseases is carried out on humans, eg, in an adult human.

For the diagnosis and/or treatment, a sterile pharmaceutical composition, containing a pharmacologically active dose of one or more proteins according to the invention, is administered to a patient in order to diagnose and/or treat primary or secondary sclerosing diseases.

In an embodiment, the administration takes place locally, preferably as a intraarticular or intramuscular injection; or systemically, preferably as a subcutaneous, intramuscular or intravenous injection or infusion, or by means of oral or transdermal administration.

A further aspect of the invention relates to the use of the protein according to the invention or the fusion protein comprising at least one protein according to the invention in diagnosing members of the TGF-β/BMP family or in diagnosing diseases associated with increased BMP receptor activation.

"Increased BMP receptor activation" is to be understood as activation of at least one BMP receptor, which activation is brought about by a mutation of a BMP receptor (Shore and Kaplan 2008), preferably constitutively activating mutations. "Constitutively activating mutations" are to be understood as mutations in which at least one BMP receptor is activated in the absence of BMPs.

A further aspect of the invention relates to the use of a nucleotide sequence, comprising a sequence coding for a protein according to the invention or a fusion protein comprising at least one protein according to the invention, and/or of a vector, comprising a nucleotide sequence comprising a sequence coding for a protein according to the invention or a fusion protein comprising at least one protein according to the invention, in diagnosing members of the TGFβ/BMP family or in diagnosing diseases associated with increased BMP receptor activation.

In addition to the use in diagnosing and/or treating primary or secondary sclerosing diseases, the proteins according to the invention are suitable for biological research and other applications in which the detection of a member of the TGF-β/BMP family is of interest. Applications of this kind are in particular Western Blot, immunostaining of cells (e.g. for flow cytometry and microscopy) and ELISA, and the use as a tracer in imaging techniques such as CT (computer tomography), PET/CT (positron emission tomography).

### First Configuration Concepts

1. Protein having an amino acid sequence that has at least 70, 75, 80, 85, 90, 95, 96, 97, 98 or 99% identity with the sequence of SEQ ID NO. 1, or the fragments thereof, for use in diagnosing and treating primary or secondary sclerosing diseases.
2. Protein comprising sequence SEQ ID NO. 1 or SEQ ID NO. 2 for use in diagnosing and/or treating primary or secondary sclerosing diseases.
3. Protein according to either Concept 1 or Concept 2, having a length of from 232 amino acids to 801 amino acids, for use in diagnosing and/or treating primary or secondary sclerosing diseases.
4. Protein according to any of Concepts 1 to 3, characterized in that the protein is a transferrin receptor (Tfr) 2α, a transferrin receptor (Tfr) 2β or an extracellular domain of Tfr2α for use in diagnosing and/or treating primary or secondary sclerosing diseases.
5. Protein according to any of Concepts 1 to 4 for use in diagnosing and treating primary or secondary sclerosing diseases, characterized in that the protein is the human transferrin receptor (Tfr) 2α (SEQ ID NO. 3), the murine transferrin receptor (Tfr) 2α (SEQ ID NO. 4), the human transferrin receptor (Tfr) 2β (SEQ ID NO. 1) or the extracellular domains of human Tfr2α (SEQ ID NO. 1), the murine transferrin receptor (Tfr) 2β (SEQ ID NO. 2) or the extracellular domains of murine Tfr2α (SEQ ID NO. 2).
6. Fusion protein comprising at least one protein according to any of Concepts 1 to 5 for use in diagnosing and/or treating primary or secondary sclerosing diseases.
7. Protein according to any of Concepts 1 to 5 or fusion protein according to Concept 6 for use in diagnosing and/or treating primary or secondary sclerosing diseases, characterized in that the protein or fusion protein comprises at least one modification selected from proteins containing D-amino acids, pseudopeptide bonds, amino alcohols, non-proteinogenic amino acids, amino acids having modified side groups and/or circular proteins.
8. Protein according to any of Concepts 1 to 5 or 7, or fusion protein according to either Concept 6 or Concept 7 for use in diagnosing and/or treating diseases associated with increased BMP receptor activation.
9. Nucleotide sequence comprising a sequence coding for a protein according to any of Concepts 1 to 5 or 7 or a fusion protein according either Concept 6 or Concept 7 for use in diagnosing and/or treating primary or secondary sclerosing diseases.
10. Vector comprising a nucleotide sequence according to Concept 9 for use in diagnosing and/or treating primary or secondary sclerosing diseases.
11. Pharmaceutical composition comprising at least one protein according to any of Concepts 1 to 5 or 7 or a fusion protein according either Concept 6 or Concept 7 for use in diagnosing and/or treating primary or secondary sclerosing diseases.
12. Pharmaceutical composition comprising at least one protein according to any of Concepts 1 to 5 or 7 or a fusion protein according either Concept 6 or Concept 7 for use in diagnosing and/or treating diseases associated with increased BMP receptor activation.
13. Use of a protein according to any of Concepts 1 to 5 or 7 or a fusion protein according to either Concept 6 or Concept 7 in diagnosing members of the TGF-β/BMP family or in diagnosing diseases associated with increased BMP receptor activation.
14. Use of a nucleotide sequence according to Concept 9 and/or of a vector according to Concept 10 in diagnosing members of the TGF-β/BMP family or in diagnosing diseases associated with increased BMP receptor activation.

### Second Configuration: Transferrin receptor 2 inhibitors for use in the treatment of bone diseases, iron metabolism disorders, and hematopoietic disorders

The second configuration of the invention relates to transferrin receptor 2 inhibitors for use in the treatment of bone diseases, iron metabolism disorders, and hematopoietic disorders.

Osteoporosis is the most common bone disease, in which the bone density and bone quality reduces, which is associated with an increased risk of fracture. Bone loss may have various causes, and can be traced back to an increase in bone resorption by osteoclasts and inhibition of bone formation by osteoblasts. Forms of treatment to date, such as bisphosphonates or monoclonal antibodies such as denosumab (anti-RANKL antibody) aim to inhibit the osteoclasts and thus the bone resorption. Bone synthesizing, osteoanabolic therapy options which specifically promote bone synthesis exist to date only in the form of teriparatide which can be used only in severe cases of osteoporosis.

Anaemia is characterized by too low an amount of oxygen-carrying haemoglobin in the blood, and can be either hereditary or developed. The most common form of anaemia is iron deficiency anaemia which is usually caused by malnutrition or bleeding. Anaemia is usually treated etiologically, and so in the case if iron deficiency anaemia, iron is administered medicinally.

The present invention relates to the modulation or inhibition of transferrin receptor 2 for use in the treatment of bone diseases, iron metabolism disorders, and hematopoietic disorders.

Transferrin receptor 2 (Tfr2) is expressed primarily in the liver, where it regulates the iron metabolism. The loss of Tfr2 leads, in mice and humans, to iron overload as a result of downregulation of the expression of hepcidin in the liver, which then leads to increased iron resorption. In addition, Tfr2 is necessary for erythrocyte differentiation.

Embodiments of the invention relate to an inhibitor for use in the treatment of primary and/or secondary osteoporosis.

Embodiments of the invention relate to an inhibitor for use in the treatment of anaemia resulting from tumors or anaemia within the context of chronic diseases (inflammation, dialysis, diabetes, etc.). In this situation, hepcidin is upregulated, leading to iron retention in the monocytes and macrophages, via degradation of ferroportin. A soluble form of Tfr2 would act as a ligand trap (for BMPs) and thus quasi inhibit the Tfr2 signaling cascade, resulting in downregulation of the expression of hepcidin in the liver and then in increased iron resorption and better provision from the MPS.

Embodiments of the invention relate to an inhibitor for use in the treatment of myelodysplastic syndromes, beta thalassemia, renal insufficiency and anaemia of chronic disease (ACD).

In embodiments of the invention, the inhibitor interacts with at least one isoform selected form Tfr2α or Tfr2β.

In embodiments of the invention, the inhibitor is selected from an antibody or a fragment thereof, a peptide, a fusion protein, aptamer or RNA interference, for example siRNA, shRNA, miRNA, Crispr/Cas, other recombinases, etc.

Examples for types of inhibitors are set out in the following (the inhibitors of the present invention will differ, however, in that they inhibit Tfr2 and not the biological proteins that are the subject of these examples):

### Aptamer:

Pegaptanib, a pegylated anti-VEGF (Vascular endothelial growth factor) aptamer; Adamis AP, Altaweel M, Bressler NM, et al. Changes in retinal neovascularization after pegaptanib (Macugen) therapy in diabeticindividuals. Ophthalmology 2006;113(1):23-8
Anti-PDGF (Platelet derived growth factor) aptamers in tumor treatment: Pietras K, Rubin K, Sjoblom T. Inhibition of PDGF receptor signaling in tumor stroma enhances antitumor effect of chemotherapy. Cancer Res 2002;62:5476-84

### Peptide:

STI-571: Inhibitor of protein tyrosinases (c-Abl, Bcr-Abl, c-kit): Biochem Biophys Res Commun. 2003 Oct 3;309(4):709-17. STI-571: an anticancer protein-tyrosine kinase inhibitor. Roskoski R Jr.

### RNA interference:

Anti-IL-13 siRNA for treating asthma: Lively, TN, Kossen, K, Balhorn, A, Koya, T, Zinnen, S, Takeda, K et al. (2008). Effect of chemically modified IL-13 short interfering RNA on development of airway hyperresponsiveness in mice. J Allergy Clin Immunol 121: 88-94.
Anti-Hsp27 siRNA for treating pharmacoresistant prostate cancer: Liu, C, Liu, X, Rocchi, P, Qu, F, lovanna, JL and Peng, L (2014). Arginine-terminated generation 4 PAMAM dendrimer as an effective nanovector for functional siRNA delivery in vitro and in vivo. Bioconjug Chem 25: 521-532.
Recombinases: Karpinski J, Hauber I, Chemnitz J, Schäfer C, Paszkowski-Rogacz M, Chakraborty D, Beschorner N, Hofmann-Sieber H, Lange UC, Grundhoff A, Hackmann K, Schrock E, Abi-Ghanem J, Pisabarro MT, Surendranath V, Schambach A, Lindner C, van Lunzen J, Hauber J, Buchholz F. Directed evolution of a recombinase that excises the provirus of most HIV-1 primary isolates with high specificity. Nat Biotechnol. 2016 Apr;34(4):401-9
Crispr/Cas: Modzelewski AJ, Chen S, Willis BJ, Lloyd KCK, Wood JA, He L. Efficient mouse genome engineering by CRISPR-EZ technology. Nat Protoc. 2018 Jun;13(6):1253-1274.

### Antibody:

Anti-cytokine antibodies such as TNFa or II-1b for treating autoimmune diseases: Susan J. Lee, MD,a,b Javier Chinen, MD, PhD,c and Arthur Kavanaugh, M Immunomodulator therapy: Monoclonal antibodies, fusion proteins, cytokines, and immunoglobulins. J Allergy Clin Immunol. 2010, 125 (2)
Chimeric Antigen Receptor T cell therapy: Cartellieri M, Feldmann A, Koristka S, Arndt C, Loff S, Ehninger A, von Bonin M, Bejestani EP, Ehninger G, Bachmann MP. Switching CAR T cells on and off: a novel modular platform for retargeting of T cells to AML blasts. Blood Cancer J. 2016 Aug 12;6(8):e458
Anti-PD-1 antibodies in immunotherapy for various cancer types: Riley JL. Combination checkpoint blockade--taking melanoma immunotherapy to the next level. N Engl J Med. 2013 Jul 11;369(2):187-9. doi: 10.1056/NEJMe1305484. Epub 2013 Jun 2.
Anti-TGFbeta antibodies in the treatment of anaemia in MDS: Platzbecker et al. Lancet Oncology 2017
In embodiments of the invention, the inhibitor is an antibody or a fragment thereof. Within the context of the present invention, a fragment is to be understood as an antibody fragment that comprises at least one Tfr2-binding sequence. In this case, by way of example, fragments could be scFv, Fab or Fc fragments.

In embodiments of the invention, the inhibitor is a bispecific antibody. In this case, the bispecific antibody has a first affinity for Tfr2 and a second affinity for a further tissue-specific membrane protein. In embodiments of the invention, the tissue-specific membrane proteins are glycoproteins. The membrane proteins CD (cluster of differentiation) antigens are preferred.

The term "cluster of differentiation" (CD for short) denotes groups of immunophenotypical surface features of cells which can be categorized according to biochemical or functional criteria. Owing to the tissue-specific expression thereof, the CD molecules can be used to target the bispecific antibody in a tissue-specific manner. As a result, the Tfr2 can a be inhibited in a tissue-specific manner.

The invention also relates to a pharmaceutical composition comprising at least one transferrin receptor 2 inhibitor according to the invention.

In embodiments of the invention, the pharmaceutical composition is a solution, tablet or capsule. In embodiments of the invention, the inhibitor according to the invention is used similarly as a coating for implant materials, preferably metals or plastics materials, and/or implants, preferably protheses, screws or nail holes.

In an embodiment, the pharmaceutical composition is administered locally in an intraarticular or intramuscular manner, or systemically in a subcutaneous or intravenous manner, or by means of oral administration. In an embodiment, the pharmaceutical composition is in a suitable form for intraarticular, intramuscular, subcutaneous, intravenous, inhalative or oral administration.

In an embodiment, the pharmaceutical composition contains the inhibitor according to the invention in a dose of from 10 µg/kg to 100 mg/kg body weight per administration.

In a further embodiment, the pharmaceutical composition further comprises a pharmaceutically acceptable diluent or base. In an embodiment, the pharmaceutically acceptable diluent or base is an aqueous solution, preferably a buffered aqueous solution, an aqueous saline solution or an aqueous glycine solution. In an embodiment, the buffered aqueous solution is selected from a histidine-buffered aqueous solution having a pH of from pH 5.0 to pH 7.0, or a sodium succinate-, sodium citrate-, sodium phosphate-, or potassium phosphate-buffered aqueous solution. In an embodiment, the buffered aqueous solution has a concentration of from 1 mmol/I (mM) to 500 mM, preferably 1 mM to 50 mM. In a further embodiment, the pharmaceutically acceptable diluent or base comprises sodium chloride, preferably in a concentration of between 0 mM and 300 mM, particularly preferably in a concentration of 150 mM.

In an embodiment of the invention, the pharmaceutical composition furthermore comprises at least one pharmaceutically acceptable excipient. An "excipient" is understood to be a compound that adjusts physiological conditions with regard to the pH and/or the ionic strength, and/or increases the stability of the pharmaceutical composition. In an embodiment, at least one pharmaceutically acceptable excipient is selected from sodium acetate, sodium chloride, potassium chloride, calcium chloride or sodium lactate.

In embodiments of the invention, the pharmaceutical composition is sterile. The pharmaceutical composition is sterilized by means of known methods.

The present invention also relates to the use of the inhibitor according to the invention or of the pharmaceutical composition for treating bone diseases, iron metabolism disorders and hematopoietic disorders.

Embodiments of the invention relate to an inhibitor for use in the treatment of primary and/or secondary osteoporosis.

New data from our laboratory show that Tfr2 is also expressed in the bones. Tfr2-deficient mice exhibit a two-fold increase in bone mass, both bone formation and bone resorption being increased. Said data suggest that blocking Tfr2 can have positive effects both on erythropoiesis and on bone metabolism.

Embodiments of the invention relate to an inhibitor for use in the treatment of anaemia resulting from tumors or anaemia within the context of chronic diseases (inflammation, dialysis, diabetes, etc.). In this situation, hepcidin is upregulated, leading to iron retention in the monocytes and macrophages, via degradation of ferroportin. A soluble form of Tfr2 would act as a ligand trap (for BMPs) and thus quasi inhibit the Tfr2 signaling cascade, resulting in downregulation of the expression of hepcidin in the liver and then in increased iron resorption and better provision from the MPS.

Embodiments of the invention relate to an inhibitor for use in the treatment of myelodysplastic syndromes, beta thalassemia, renal insufficiency and anaemia of chronic disease (ACD).

### Second Configuration Clauses

1. A transferrin receptor 2 inhibitor for use in the treatment of bone diseases, iron metabolism disorders, and /or hematopoietic disorders.
2. Inhibitor according to Clause 1 for use in the treatment of osteoporosis.
3. Inhibitor according to Clause 1 for use in the treatment of anaemia.
4. Inhibitor according to Clause 1 for use in the treatment of myelodysplastic syndromes.
5. Inhibitor according to any of the preceding Clauses, characterized in that the inhibitor is selected from an antibody or a fragment thereof, a peptide, a fusion protein, aptamers or RNA interference.
6. Inhibitor according to any of the preceding Clauses, characterized in that the inhibitor is an antibody or a fragment thereof.
7. Inhibitor according to any of the preceding Clauses, characterized in that the inhibitor is a bispecific antibody.
8. Pharmaceutical composition comprising at least one inhibitor according to any of Clauses 1 to 7 and a pharmaceutically acceptable excipient.
9. Use of a pharmaceutical composition according to Clause 8 for treating bone diseases, iron metabolism disorders and hematopoietic disorders.

### Further embodiments

The configurations of the invention also provide the following Aspects.

### Antagonist (Antibody), eg, for Osteoporosis

1. A method of treating or preventing or reducing the risk of a bone disease or condition in a human or animal subject, the method comprising antagonising transferrin receptor 2 (Tfr2) in the subject.
2. The method of Aspect 1, wherein the method comprises inhibiting p38 MAP kinase pathway signalling in bone cells (optionally osteoblasts) by antagonising Tfr2 of the cells.
   Examples of bone cells are osteoblasts, osteocytes and osteoclasts.
3. The method of Aspect 1 or 2, wherein the method comprises upregulating Wnt expression in bone cells (optionally osteoblasts) by antagonising Tfr2 in the subject.
   Tfr2 in the subject may be cell-bound (such as wherein the cells are osteoblasts) or soluble Tfr2.
4. The method of any preceding Aspect, wherein the method comprises inhibiting sclerostin, Dkk1 and/or Activin A activity or expression in bone cells (optionally osteoblasts) by antagonising Tfr2 of the cells.
   Our research showed that Dkk1 (a Wnt antagonist) is severely downregulated.
5. The method of any preceding Aspect, wherein the method comprises inhibiting expression of Phex, Dmp1, Dkk1 and/or Sost in bone cells (optionally osteoblasts) by antagonising Tfr2 of the cells.
6. The method of any preceding Aspect, wherein the method comprises inhibiting the binding of Tfr2 to a BMP and/or transferrin in the subject.
7. The method of Aspect 6, wherein the BMP is one or more of BMP2, 4, 6 and 7.
8. The method of Aspect 7, wherein the BMP is BMP2.
9. The method of Aspect 7, wherein the BMP is BMP4.
10. The method of Aspect 7, wherein the BMP is BMP6.
11. The method of Aspect 7, wherein the BMP is BMP7.
12. The method of any preceding Aspect, wherein the disease or condition is osteoporosis.

For example, the osteoporosis is primary osteoporosis. For example, the osteoporosis is secondary osteoporosis. For example, the osteoporosis is premature osteoporosis. For example, the osteoporosis in in post-menopausal women.

In an example, the subject is a post-menopausal woman or female.
13. The method of any preceding Aspect, wherein the method is for causing one or more of the following (optionally of femur and/or vertebrae and/or spine bone):-
(a) Increased bone volume;
(b) Increased bone density;
(c) Increased trabecular number (Tb.N);
(d) Increased trabecular thickness (Tb.Th);
(e) Decreased trabecular spacing (Tb.Sp);
(f) Increased bone mineral density;
(g) Increased bone micro-mineralisation density;
(h) Increased bone mass; and
(i) Increased bone strength.

See, eg, http://microctworld.net/trabecular-thickness-tb-th-trabecular-spacing-tb-sptrabecularnumber-tb-n/ for explanations of these art-recognised terms.

Optionally the bone is skull, mandible, clavicle, ribs or long bones.

In an example, the method of the invention is for:-
causing one or more of the following (optionally of femur and/or vertebrae and/or spine bone):-
(a) Increased trabecular bone volume;
(b) Increased cortical bone density;
(c) Increased trabecular number (Tb.N);
(d) Increased trabecular thickness (Tb.Th);
(e) Decreased trabecular spacing (Tb.Sp);
(f) Increased trabecular bone micro-mineralisation density;
(g) Increased bone mass; and (h) Increased bone strength.

14. The method of any preceding Aspect, wherein the method is for increasing bone formation in the subject and/or decreasing bone resorption in the subject.
15. The method of any preceding Aspect, wherein the method is for increasing osteoblasts (or bone formation activity thereof) in the subject and/or decreasing osteoclasts (or bone resorption activity thereof) in the subject.

In an example, the method is for increasing eg, osterix-expressing osteoblasts, or increasing osterix (aka Transcription factor Sp7) expression in the subject.

Increasing osteoblasts may comprise increasing differentiation of progenitor cells into osteoblasts and/or reduced turnover of osteoblasts. Decreasing osteoclasts may comprise decreasing differentiation of progenitor cells into osteoclasts and/or increased turnover of osteoblasts.
16. The method of any preceding Aspect, wherein the method is for increasing bone turnover in the subject.
17. The method of any preceding Aspect, wherein the method is for increasing pro-collagen type I N-terminal peptide (P1NP) in the subject and/or increasing C-terminal telopeptide of type I collagen (CTX) in the subject.

Optionally, the method is for increasing osteocalcin, bone-specific alkaline phosphatase or OB or NTX or DPD in the subject.
18. The method of any preceding Aspect, wherein the method is for increasing osteocytes in the subject.
19. The method of any preceding Aspect, wherein the method comprises administering a Tfr2 antagonist to the subject, optionally wherein the antagonist is an anti-Tfr2 antibody or antibody fragment that specifically binds to Tfr2.

For example, the antagonist is an antibody that specifically binds to Tfr2-ECD (eg, human Tfr2ECD).

In any aspect of the invention, in an example the Tfr2 is human Tfr2.
20. The method of Aspect 19, wherein the antibody or fragment competes with an antibody selected from 1B1 (MyBioSource, MBS833691), 3C5 (Abnova, H00007036-M01), CY-TFR (Abnova, MAB6780) and B-6 (Santa Cruz Biotechnology, sc-376278), 353816 (R&D Systems, MAB3120) and 9F8 1C11 (Santa Cruz Biotechnology, sc-32271) for binding to Tfr2 as determined by surface plasmon resonance (SPR).

In an example, SPR is carried out under the following conditions:-
- Flow: 50 µl/min
- Contact time: 220 sec
- Running buffer: HBS-P
- 37°C
- Regeneration: 1) 120 s Gly-HCl (pH 1.5), 2) 120 s 4 M MgCl2, 3) 60 s 5 M NaCl + 50 mM NaOH, 4) 60 s 100 mM NaOH

In an example, SPR is carried by Biacore^{™}, eg, using Biacore T200 and analysed using Evaluation software 3.1.
21. The method of any preceding Aspect, wherein the method comprises administering a sclerostin, Dkk1 or Activin A antagonist to the subject, optionally wherein the antagonist is an anti-sclerostin antibody or antibody fragment that specifically binds to sclerostin, Dkk1 or Activin A.
22. The method of Aspect 21 wherein a multi-specific antibody or fragment is administered to the subject, wherein the antibody or fragment specifically binds to Tfr2 (eg, human Tfr2) and sclerostin, Dkk1 or Activin A.

In an example, the antibody specifically binds to Tfr2 (eg, human Tfr2) and sclerostin (eg, human sclerostin).

In an example, the antibody specifically binds to Tfr2 (eg, human Tfr2) and Dkk1 (eg, human Dkk1).

In an example, the antibody specifically binds to Tfr2 (eg, human Tfr2) and Activin A (eg, human Activin A).

In an example, the antibody specifically binds to Tfr2 (eg, human Tfr2) and CD63 (eg, human CD63).

In an example, the antibody specifically binds to Tfr2 (eg, human Tfr2) and Syndecan-3 (eg, human Syndecan-3).

In an example, the antibody specifically binds to Tfr2 (eg, human Tfr2) and FGFR1 (eg, human FGFR1).

In an example, the antibody specifically binds to Tfr2 (eg, human Tfr2) and LIFR (eg, human LIFR).

In an example, the antibody specifically binds to Tfr2 (eg, human Tfr2) and CD47 (eg, human CD47).

In an example, the antibody specifically binds to Tfr2 (eg, human Tfr2) and CX3CR1 (eg, human CX3CR1).

In an example, the antibody specifically binds to Tfr2 (eg, human Tfr2) and CD68 (eg, human CD68).

In an example, the antibody specifically binds to Tfr2 (eg, human Tfr2) and osteocalcin (eg, human osteocalcin).

In an example, the antibody specifically binds to Tfr2 (eg, human Tfr2) and DMP-1 (eg, human DMP-1).

In an example, the antibody specifically binds to Tfr2 (eg, human Tfr2) and osteoadherin (eg, human osteoadherin).

In an example, the antibody specifically binds to Tfr2 (eg, human Tfr2) and alkaline phosphatase (eg, human alkaline phosphatase).

### Agonist for HO, FOP etc

23. A method of treating or preventing a bone disease or condition in a human or animal subject, the method comprising agonising transferrin receptor 2 (Tfr2) in the subject.

The method may, therefore reduce the risk of the disease or condition in the subject.
24. The method of Aspect 23, wherein the method comprises stimulating p38 MAP kinase pathway signalling in bone cells (optionally osteoblasts) by agonising Tfr2 of the cells.
25. The method of Aspect 23 or 24, wherein the method comprises downregulating Wnt expression in bone cells (optionally osteoblasts) by agonising Tfr2 of the cells.
26. The method of any one of Aspects 23 to 25, wherein the method comprises stimulating sclerostin activity or expression in bone cells (optionally osteoblasts) by agonising Tfr2 of the cells.
27. The method of any one of Aspects 23 to 26, wherein the method comprises promoting BMP binding of Tfr2.
28. The method of Aspect 27, wherein the BMP is one or more of BMP2, 4, 6 and 7.
29. The method of Aspect 28, wherein the BMP is BMP2.
30. The method of Aspect 28, wherein the BMP is BMP4.
31. The method of Aspect 28, wherein the BMP is BMP6.
32. The method of Aspect 28, wherein the BMP is BMP7.
33. The method of any one of Aspects 23 to 32, wherein the method comprises administering a Tfr2 agonist to the subject, optionally wherein the agonist is an anti-Tfr2 antibody or antibody fragment that specifically binds to Tfr2 (eg, human Tfr2).
34. The method of any one of Aspects 23 to 33, wherein the method comprises administering a sclerostin, Dkk1 or Activin A agonist to the subject, optionally wherein the agonist is an antisclerostin, Dkk1 or Activin A antibody or antibody fragment that specifically binds to sclerostin, Dkk1 or Activin A.

In an example, the agonist antibody is romosozumab or comprises a sclerostin binding site thereof.
35. The method of Aspect 34, wherein a multi-specific antibody or fragment is administered to the subject, wherein the antibody or fragment specifically binds to Tfr2 and sclerostin, Dkk1 or Activin A

### BMP trap for HO, FOP etc

36. A method of treating or preventing a disease or condition in a human or animal subject, the method comprising administering a BMP-binding agent to the subject, wherein the agent competes with soluble Tfr2-ECD for binding to the BMP, and wherein the disease or condition is mediated by said BMP.

The method may, therefore reduce the risk of the disease or condition in the subject.

In an example, the agent is a protein agent, eg, comprises one or more polypeptides, such as an Fc fusion polypeptide. In an example, the agent is a BMP trap, such as a trap that comprises a binding site for the BMP fused to a half-life-extending moiety (eg, a PEG, serum albumin, an antibody Fc or domain, or an anti-serum albumin binding moiety).

Herein, for "Tfr2-ECD-Fc" the Tfr2 may be N- or C-terminal to the Fc. For example, in N- to C-direction the Tfr2-ECD-Fc comprises Fc and the ECD.
37. The method of Aspect 36, wherein the agent is a BMP trap.
38. The method of Aspect 36 or 37, wherein the BMP is a BMP2.
39. The method of Aspect 36 or 37, wherein the BMP is a BMP4.
40. The method of Aspect 36 or 37, wherein the BMP is a BMP6.
41. The method of Aspect 36 or 37, wherein the BMP is a BMP7.
42. The method of any one of Aspects 36 to 41, wherein the soluble Tfr2-ECD is comprised by a fusion protein, wherein the ECD is fused (optionally via a linker, such as a IEGR linker) to an antibody Fc region.

Optionally, the soluble Tfr2-ECD is comprised by a fusion protein, wherein the ECD is fused (optionally via a linker) to a human antibody IgG Fc region.

In an example, the Fc herein is a human Fc. In an example, the Fc herein is a human gamma Fc (eg, a gamma-1, gamma-2, gamma-3 or gamma-4). In an example, the Fc herein is a human alpha Fc. In an example, the Fc herein is a human delta Fc. In an example, the Fc herein is a human epsilon Fc. In an example, the Fc herein is a human mu Fc.
43. The method of Aspect 42, wherein the fusion protein comprises a Tfr2-ECD-Fc dimer.
44. The method of any one of Aspects 36 to 43, wherein the agent binds to one, more or all of BMP2, 4, 6 and 7 more strongly than the binding of holo-tranferrin and/or BMPR to said BMP(s).
45. The method of any one of Aspects 36 to 44, wherein the agent binds to BMP2 more strongly than the binding of holo-tranferrin and/or BMPRIA to said BMP.

Binding strength may be KD as determined by SPR, for example.
46. The method of any one of Aspects 36 to 45, wherein the agent binds to BMP4 more strongly than the binding of holo-tranferrin to said BMP.
47. The method of any one of Aspects 36 to 46, wherein the agent binds to BMP6 more strongly than the binding of holo-tranferrin to said BMP.
48. The method of any one of Aspects 36 to 47, wherein the agent binds to BMP7 more strongly than the binding of holo-tranferrin to said BMP.
49. The method of any one of Aspects 36 to 48, wherein the agent comprises a Tfr2-ECD which is comprised by a fusion protein, wherein the ECD is fused (optionally via a linker, such as a IEGR linker) to an antibody Fc region.

Optionally, the soluble Tfr2-ECD is comprised by a fusion protein, wherein the ECD is fused (optionally via a linker) to a human antibody IgG Fc region.

In an example, the Fc herein is a human Fc. In an example, the Fc herein is a human gamma Fc (eg, a gamma-1, gamma-2, gamma-3 or gamma-4). In an example, the Fc herein is a human alpha Fc. In an example, the Fc herein is a human delta Fc. In an example, the Fc herein is a human epsilon Fc. In an example, the Fc herein is a human mu Fc.
50. The method of Aspect 49, wherein the fusion protein comprises a Tfr2-ECD-Fc dimer.
51. The method of any one of Aspects 36 to 50, wherein the agent comprises Tfr2 extracellular domain (Tfr2-ECD), optionally wherein the agent is comprised by a fusion protein comprising a second moiety.
52. The method of Aspect 51 wherein the second moiety is a half-life-extending moiety for enhancing half-life of the trap in a subject.
53. The method of Aspect 51 or 52 wherein the second moiety is selected from an antibody domain (eg, a Fc region), a polyethylene glycol (PEG) moiety, serum albumin or an antiserum albumin binding moiety (optionally an antibody fragment or domain such as a scFv, Fab or domain antibody (or Nanobody^{™})).
54. The method of any one of Aspects 36 to 53, wherein the disease or condition is a bone disease or condition.

### Specific Indications

55. The method of any preceding Aspect, wherein the disease or condition is a sclerosing disease or condition.

Optionally, the sclerosing disease is a sclerosing disease of the skeleton. Optionally, the sclerosing disease is a sclerosing disease outside the skeleton.

Optionally, the subject has received or is undergoing steroid treatment, NSAID treatment, ressection or irradiation treatment. Optionally, the subject is administered (simultaneously or sequentially with the Tfr2 agonism) steroid treatment, NSAID treatment, ressection or irradiation treatment.
56. The method of any preceding Aspect, wherein the disease or condition is a disease or condition comprising pathological bone formation.
57. The method of Aspect 55 or 56, wherein the disease or condition is an ossification disease or condition, optionally a heterotypic ossification (HO) disease or condition, or *fibrodysplasia ossificans progressive* (FOP) disease or condition.
58. The method of Aspect 55 or 56, wherein the disease or condition is selected from heterotypic ossification (HO) (optionally HO of muscle), Van Buchem disease, Sclerosteosis and *fibrodysplasia ossificans progressive* (FOP).

FOP is also known as Fibrodysplasia ossificans multiplex progressiva, Myositis ossificans progressiva oder Münchmeyer-Syndrome.

Optionally herein, HO is trauma-potentiated HO, eg, wherein the trauma is a blast injury or hip surgery or knee surgery.

In an example, the method treats or prevents a bone disease of the skull, mandible, clavicle, ribs or long bones.
59. The method of any one of Aspects 36 to 58, wherein the method comprises administering a retinoic acid receptor gamma (RAR-γ) agonist (optionally palovarotene) to the subject simultaneously or sequentially with a Tfr2 agonist or the trap.

Optionally, the antagonist, agonist, agent, trap, antibody or fragment is administered to the subject intravenously, subcutaneously or intramuscularly.
60. The method of any one of Aspects 36 to 59, wherein the method comprises administering a BMP signalling antagonist to the subject simultaneously or sequentially with a Tfr2 agonist or the trap.
61. The method of any one of Aspects 36 to 60, wherein the method inhibits cartilage formation in the subject.
62. The method of any one of Aspects 36 to 61, wherein the method inhibits chondrocyte formation in the subject.

### Antibody

63. An anti-Tfr2 antibody or fragment that specifically binds Tfr2 for use in the method of any preceding Aspect, or for treating or preventing a bone disease or condition in a human.
64. The antibody or fragment of Aspect 63, wherein the antibody or fragment is a human antibody or fragment.

In an example, the antibody or fragment comprises one or more Tfr2 binding sites, wherein each binding site comprises a human VH domain (that is optionally paired with a human VL domain).

In an example, the antibody or fragment comprises one or more Tfr2 binding sites, wherein each binding site comprises a human VL domain (that is optionally paired with a human VH domain).

Each VH domain is, for example, encoded by a nucleotide sequence that is a recombinant of a human VH, DH and JH gene segment. For example, the VH is selected from the VH gene segment disclosed in Table 7. Additionally or alternatively, for example, the DH is selected from the D gene segment disclosed in Table 7. Additionally or alternatively, for example, the JH is selected from the J gene segment disclosed in Table 7. For example the VH, DH and JH are selected from the gene segments disclosed in Table 7. In an example, the VH is a VH1 family VH gene segment, optionally in combination with a JH1, JH2, JH3, JH4, JH5 or JH6; eg, a VH1 in combination with a JH4, or a VH1 in combination with a JH6. In an example, the VH is a VH2 family VH gene segment, optionally in combination with a JH1, JH2, JH3, JH4, JH5 or JH6; eg, a VH2 in combination with a JH4, or a VH2 in combination with a JH6. In an example, the VH is a VH3 family VH gene segment, optionally in combination with a JH1, JH2, JH3, JH4, JH5 or JH6; eg, a VH3 in combination with a JH4, or a VH3 in combination with a JH6.

Each VL domain is, for example, encoded by a nucleotide sequence that is a recombinant of a human VL and JL gene segment. For example, the VL is selected from the VL gene segment disclosed in Table 8. Additionally or alternatively, for example, the JL is selected from the J gene segment disclosed in Table 8. For example the VL and JL are selected from the gene segments disclosed in Table 8. In an example, the VL is a Vκ1 family gene segment, optionally in combination with a Jκ1, Jκ2, Jκ3, Jκ4 or Jκ5; eg, a Vκ1 in combination with a Jκ1, or a Vκ1 in combination with a Jκ4.

Additionally or alternatively, the inhibitor, Trf2-ECD-Fc, antibody or fragment comprises a heavy chain constant region disclosed in Table 4. Additionally or alternatively, the inhibitor, Trf2-ECD-Fc, antibody or fragment comprises a lambda light chain constant region disclosed in Table 4; alternatively, the inhibitor, Trf2-ECD-Fc, antibody or fragment comprises a kappa light chain constant region disclosed in Table 4. In an example the VL is a lambda VL fused to a lambda constant region. In another example, the VL is a kappa VL fused to a kappa constant region. In another example, the VL is a kappa VL fused to a lambda constant region.

Additionally or alternatively, the inhibitor, Trf2-ECD-Fc, antibody or fragment comprises mammalian pattern glycosylation, eg, hamster, mouse or human glycosylation. For example, the inhibitor, Trf2-ECD-Fc, antibody or fragment is an expression product of a HEK293 cell. For example, the inhibitor, Trf2-ECD-Fc, antibody or fragment is an expression product of a CHO cell. For example, the inhibitor, Trf2-ECD-Fc, antibody or fragment is an expression product of a Cos cell. For example, the inhibitor, Trf2-ECD-Fc, antibody or fragment is an expression product of a *Picchia* cell. For example, the inhibitor, Trf2-ECD-Fc, antibody or fragment is an expression product of a *E coli* cell.
65. The antibody or fragment of Aspect 63 or 64, wherein the antibody or fragment competes with a reference antibody for binding to human Tfr2 (optionally human Tfr2-ECD) as determined by SPR, wherein the reference antibody is selected from 1B1 (MyBioSource, MBS833691), 3C5 (Abnova, H00007036-M01), CY-TFR (Abnova, MAB6780) and B-6 (Santa Cruz Biotechnology, sc-376278), 353816 (R&D Systems, MAB3120) and 9F8 1C11 (Santa Cruz Biotechnology, sc-32271).
66. A pharmaceutical composition comprising the antibody, fragment, dimer or monomer of any one of Aspects 63 to 65 and a pharmaceutically acceptable diluent, excipient or carrier.
67. The composition of Aspect 66, further comprising an anti-sclerostin antibody (optionally romosozumab), anti-Dkk1 antibody or anti-Activin A antibody (eg, REGN-2477), or fragment thereof.

In an example, the antibody is an anti-sclerostin antibody. In an example, the antibody is romosozumab. In an example, the antibody is an anti-Dkk1 antibody. In an example, the antibody is an anti-Activin A antibody. In these examples, the disease or condition may be FOP.

### Fc Fusion

68. A Tfr2-ECD-Fc dimer, optionally for use in the method of any preceding Aspect, or for treating or preventing a bone disease or condition in a human.

In an embodiment, the dimer comprises first and second copies of a polypeptide chain, wherein each polypeptide chain comprises (in N- to C- terminal direction) the Fc and ECD. In another embodiment, the dimer comprises first and second copies of a polypeptide chain, wherein each polypeptide chain comprises (in N- to C- terminal direction) the ECD and Fc.

The invention also provides a Tfr2-ECD-Fc monomer. In an example, the monomer comprises a polypeptide chain, wherein the chain comprises (in N- to C-terminal direction) the Fc and ECD. In an example, the monomer comprises a polypeptide chain, wherein the chain comprises (in N-to C-terminal direction) the ECD and Fc.

In an example, each polypeptide chain of the monomer, dimer, protein or inhibitor of the invention comprises or consists of SEQ ID NO: 10, or a sequence that has at least 70, 75, 80, 85, 90, 95, 96, 97, 98 or 99% identity with SEQ ID NO: 10.

In an example, each polypeptide chain of the monomer, dimer, protein or inhibitor of the invention comprises or consists of SEQ ID NO: 11, or a sequence that has at least 70, 75, 80, 85, 90, 95, 96, 97, 98 or 99% identity with SEQ ID NO: 11.

In an example, each polypeptide chain of the monomer, dimer, protein or inhibitor of the invention comprises or consists of SEQ ID NO: 12, or a sequence that has at least 70, 75, 80, 85, 90, 95, 96, 97, 98 or 99% identity with SEQ ID NO: 12.

Optionally, the dimer or monomer is an expression product isolated from a mammalian, CHO, HEK293 or Cos cell.
69. A Tfr2-ECD monomer, optionally for use in the method of any preceding Aspect, or for treating or preventing a bone disease or condition in a human.
70. The dimer of Aspect 68 or the monomer of Aspect 69, herein the ECD and Fc are human.
71. The dimer or monomer of any one of Aspects 68 to 70, wherein the Fc is a human gamma Fc.
72. The antibody or fragment of Aspects 68 to 71, wherein the Tfr2-ECD is Tfr2α-ECD.
73. The antibody or fragment of Aspects 68 to 71, wherein the Tfr2-ECD is Tfr2β-ECD.
74. The dimer or monomer of any one of Aspects 68 to 73, wherein the disease or condition is pathological bone formation.
75. The dimer or monomer of any one of Aspects 68 to 74, wherein the disease or condition is selected from an ossification disease or condition, optionally a heterotypic ossification (HO) disease or condition and a *fibrodysplasia ossificans progressive* (FOP) disease or condition. 76. The dimer or monomer of Aspect 75, wherein the disease or condition is BMP-2-induced HO.
   77. The dimer or monomer of Aspect 75, wherein the disease or condition is BMP-4-induced HO.
   78. The dimer or monomer of Aspect 75, wherein the disease or condition is BMP-6-induced HO.
   79. The dimer or monomer of Aspect 75, wherein the disease or condition is BMP-7-induced HO.
   80. A pharmaceutical composition comprising the antibody, fragment, dimer or monomer of any one of Aspects 63 to 79 and a pharmaceutically acceptable diluent, excipient or carrier.
   81. The composition of Aspect 80, further comprising a retinoic acid receptor gamma (RAR-γ) agonist (optionally polovarotene).
   82. The composition of Aspect 80 or 81, further comprising a BMP antagonist, optionally an antiBMP antibody or fragment.
   83. The composition of Aspect 82, wherein the BMP is BMP2, 4, 6 or 7.

Examples of suitable anti-BMP6 antibodies and fragments are disclosed in WO2016098079, US20160176956A1 and WO2017191437.
84. The method, antibody, fragment, dimer, monomer or composition of any preceding Aspect, wherein the Tfr2 is Tfr2α.
85. The method, antibody, fragment, dimer, monomer or composition of any one of Aspects 1 to 83, wherein the Tfr2 is Tfr2β.
86. The method, antibody, fragment, dimer, monomer or composition of any preceding Aspect for
   (a) causing increased trabecular bone volume (optionally of femur and/or vertebrae and/or spine bone;
   (b) causing increased cortical bone density (optionally of femur and/or vertebrae and/or spine bone;
   (c) causing increased trabecular number (Tb.N) (optionally of femur and/or vertebrae and/or spine bone;
   (d) causing increased trabecular thickness (Tb.Th) (optionally of femur and/or vertebrae and/or spine bone;
   (e) causing separation (Tb.Sp) (optionally of femur and/or vertebrae and/or spine bone.
   (f) causing increased trabecular bone micro-mineralisation density (optionally of femur and/or vertebrae and/or spine bone;
   (g) causing increased bone strength (optionally of femur and/or vertebrae and/or spine bone;
   (h) increasing bone formation in the subject and/or decreasing bone resorption in the subject;
   (i) increasing osteoblasts (or bone formation activity thereof) in the subject and/or increasing osteoclasts (or bone resorption activity thereof) in the subject;
   (j) increasing bone turnover in the subject;
   (k) increasing pro-collagen type I N-terminal peptide (P1NP) in the subject and/or increasing Cterminal telopeptide of type I collagen (CTX) in the subject;
   (l) increasing osteocytes in the subject;
   (m)treating or preventing a sclerosing disease or condition in the subject, optionally an ossification disease or condition, optionally a heterotypic ossification (HO) disease or condition, or *fibrodysplasia ossificans progressive* (FOP) disease or condition;
   (n) treating or preventing pathological bone formation in the subject; or
   (o) treating or preventing heterotypic ossification (HO) (optionally HO of muscle, or traumapotentiated HO, eg, wherein the trauma is a blast injury or hip surgery or knee surgery), Van Buchem disease, Sclerosteosis and *fibrodysplasia ossificans progressive* (FOP) in the subject.

In an example, the Tfr2 inhibitor (eg, antibody) is for increasing bone turnover in a subject. In an example, the Tfr2 inhibitor (eg, antibody) is for increasing bone formation (BFR/BS) in a subject. In an example, the Tfr2 inhibitor (eg, antibody) is for increasing bone resorption (Oc.S/BS) in a subject In an example, the Tfr2 inhibitor (eg, antibody) is for increasing or maintaining bone volume in a subject, eg, as determined by micro computed tomography. In an example, the Tfr2 inhibitor (eg, antibody) is for increasing or maintaining trabecular bone volume (BV/TV) in a subject, eg, as determined by micro computed tomography. In an example, the Tfr2 inhibitor (eg, antibody) is for increasing or maintaining bone stiffness in a subject, eg, as determined by a three-point flexural test. In an example, the Tfr2 inhibitor (eg, antibody) is for increasing or maintaining bone break resistance in a subject, eg, as determined by a three-point flexural test. In an example, the Tfr2 inhibitor (eg, antibody) is for increasing or maintaining bone strength in a subject, eg, as determined by a three-point flexural test. Optionally, the flexural test comprises applying force to the bone. In an example, any of these determinations is made in a surrogate non-human animal model, eg, a non-human primate, pig, rodent, rat or mouse.

In any configuration herein, optionally the protein or inhibitor of the invention comprises an antibody Fc region comprising an antibody CH2 domain and CH3 domain. For example, the Fc region is a gamma-1, gamma-2, gamma-3, gamma-4 Fc, mu, delta, epsilon or alpha Fc region. Preferably, the Fc region is a gamma-1 Fc region. Preferably, the Fc region is a human Fc region.

In any configuration herein, optionally the composition, protein or inhibitor of the invention is comprised by a medical device or container (eg, a sterile container or a container that is closed and contains sterile contents). Optionally, the composition, protein, inhibitor, device or container is in combination with a label or instructions for use to treat and/or prevent a sclerosing disease, bone disease, iron metabolism disorder or a hematopoietic disorders in a human; optionally wherein the label or instructions comprise a marketing authorisation number (optionally an FDA or EMA authorisation number); optionally wherein the device is or comprises an IV or injection device that comprises the protein or inhibitor, eg, an antibody or fragment.

Optionally, any protein, inhibitor, agent, antibody or fragment comprises a bispecific format selected from DVD-Ig, mAb², FIT-Ig, mAb-dAb, dock and lock, SEEDbody, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, minibody, knobs-in-holes, knobs-in-holes with common light chain, knobs-in-holes with common light chain and charge pairs, charge pairs, charge pairs with common light chain, in particular mAb², knob-in-holes, knob-in-holes with common light chain, knobs-in-holes with common light chain and charge pairs and FIT-Ig, e.g. mAb² and FIT-Ig.

In one embodiment, the bispecific format is selected from DVD-Ig, mAb², FIT-Ig, mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, Triple body, Miniantibody, minibody, TriBi minibody, scFv-CH₃ KIH, scFv-CH-CL-scFv, F(ab')₂-scFv, scFv-KIH, Fab-scFv-Fc, tetravalent HCab, ImmTAC, knobs-in-holes, knobs-in-holes with common light chain, knobs-inholes with common light chain and charge pairs, charge pairs, charge pairs with common light chain, DT-IgG, DutaMab, IgG(H)-scFv, scFv-(H)IgG, IgG(L)-scFv, scFv-(L)IgG, IgG(L,H)-Fv, IgG(H)-V, V(H)-IgG, IgG(L)-V, V(L)-lgG, KIH IgG-scFab, 2scFv-IgG, IgG-2scFv, scFv4-Ig and zybody.

In one embodiment, the bispecific format is selected from DVD-Ig, FIT-lg, mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, Triple body, Miniantibody, minibody, TriBi minibody, scFv-CH₃ KIH, scFv-CH-CL-scFv, F(ab')₂-scFv, scFv-KIH, Fab-scFv-Fc, tetravalent HCab, ImmTAC, knobs-in-holes, knobs-in-holes with common light chain, knobs-inholes with common light chain and charge pairs, charge pairs, charge pairs with common light chain, DT-IgG, DutaMab, IgG(H)-scFv, scFv-(H)IgG, IgG(L)-scFv, scFv-(L)IgG, IgG(L,H)-Fv, IgG(H)-V, V(H)-IgG, IgG(L)-V, V(L)-lgG, KIH IgG-scFab, 2scFv-IgG, IgG-2scFv, scFv4-Ig and zybody, for example DVD-Ig, FIT-Ig, mAb-dAb, dock and lock, SEEDbody, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, minibody, knobs-in-holes, knobs-in-holes with common light chain, knobs-in-holes with common light chain and charge pairs, charge pairs, charge pairs with common light chain, in particular knob-in-holes, knob-in-holes with common light chain, knobs-in-holes with common light chain and charge pairs and FIT-Ig, e.g. FIT-Ig.

In one embodiment, the bispecific format is selected from DVD-Ig, mAb², mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, Triple body, Miniantibody, minibody, TriBi minibody, scFv-CH₃ KIH, scFv-CH-CL-scFv, F(ab')₂-scFv, scFv-KIH, Fab-scFv-Fc, tetravalent HCab, ImmTAC, knobs-in-holes, knobs-in-holes with common light chain, knobs-inholes with common light chain and charge pairs, charge pairs, charge pairs with common light chain, DT-IgG, DutaMab, IgG(H)-scFv, scFv-(H)IgG, IgG(L)-scFv, scFv-(L)IgG, IgG(L,H)-Fv, IgG(H)-V, V(H)-IgG, IgG(L)-V, V(L)-lgG, KIH IgG-scFab, 2scFv-IgG, IgG-2scFv, scFv4-Ig and zybody, for example DVD-Ig, mAb², mAb-dAb, dock and lock, SEEDbody, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, minibody, knobs-in-holes, knobs-in-holes with common light chain, knobs-in-holes with common light chain and charge pairs, charge pairs, charge pairs with common light chain, in particular mAb², knob-in-holes, knobs-in-holes with common light chain and charge pairs, and knob-in-holes with common light chain, e.g. mAb².

In one embodiment, the bispecific format is selected from DVD-Ig, mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, Triple body, Miniantibody, minibody, TriBi minibody, scFv-CH₃ KIH, scFv-CH-CL-scFv, F(ab')₂-scFv, scFv-KIH, Fab-scFv-Fc, tetravalent HCab, ImmTAC, knobs-in-holes, knobs-in-holes with common light chain, knobs-in-holes with common light chain and charge pairs, charge pairs, charge pairs with common light chain, DTIgG, DutaMab, IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv, scFv4-Ig and zybody, for example DVD-Ig, mAb-dAb, dock and lock, SEEDbody, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, minibody, knobs-in-holes, knobs-in-holes with common light chain, knobs-in-holes with common light chain and charge pairs, charge pairs, charge pairs with common light chain, in particular knob-in-holes, knobs-in-holes with common light chain and charge pairs, and knob-in-holes with common light chain.

In an example, the subject is a human. In an alternative, the subject is a non-human animal. In an example, the subject is an adult human. In an example, the subject is a paediatric human.

For example, the % identity is at least 85%. For example, the identity is at least 90%. For example, the identity is at least 95%.

In an example, the heavy chain of the antibody or fragment of the invention is a human gamma1, gamma-2, gamma-3, gamma-4, mu, delta, epsilon or alpha isotype, preferably a gamma isotype (eg, an IgG4 isotype). In an example, the light chain of the antibody or fragment of the invention comprises a human kappa constant region. Alternatively, in an example, the light chain of the antibody or fragment of the invention comprises a human lambda constant region. Optionally, the antibody of the invention comprises a human IgG4 constant region. Optionally, the antibody of the invention comprises a human IgG1 constant region.

Optionally, the antibody is a 4-chain antibody comprising a dimer of a heavy chain associated with a dimer of a light chain.

In one embodiment, the antibody or fragment is a human antibody or fragment. In one embodiment, the antibody or fragment is a fully human antibody or fragment. In one embodiment, the antibody or fragment is a fully human monoclonal antibody or fragment.

in one embodiment, the antibody or fragment is a humanised antibody or fragment. In one embodiment, the antibody or fragment is a humanised monoclonal antibody or fragment.

In an example, the antibody or fragment of the invention binds to human Tfr2 (or sclerostin, Activin A or other recited antigen) with a Ka of eg, 5x10⁶ M⁻¹ x s⁻¹ ; or about 5x10⁶ M⁻¹ x s⁻¹. In an example, the antibody or fragment of the invention binds to with a Kd of eg, 4 or 5 s⁻¹; or about 4 or 5 s⁻¹. In an example, the antibody or fragment of the invention binds with a KD of eg, 0.07 or 0.14 nM; or about 0.07 or 0.14 nM. In an embodiment, the fragment is a Fab fragment. In an embodiment, the fragment is a scFv.

Preferably, an antibody or a fragment thereof that specifically binds to a Tfr2 (eg, human Tfr2) does not cross-react with other antigens (but may optionally cross-react with different Tfr2 species, e.g., rhesus, cynomolgus, or murine; and or may optionally cross-react with different Tfr2s). An antibody or a fragment thereof that specifically binds to a Tfr2 antigen can be identified, for example, by immunoassays, BIAcore^{™}, or other techniques known to those of skill in the art. An antibody or a fragment thereof binds specifically to a Tfr2 antigen when it binds to a hBMP6 antigen with higher affinity than to any cross-reactive antigen as determined using experimental techniques, such as radioimmunoassays (RIA) and enzyme-linked immunosorbent assays (ELISAs). Typically, a specific or selective reaction will be at least twice background signal or noise and more typically more than 10 times background. See, e.g. Paul, ed., 1989, Fundamental Immunology Second Edition, Raven Press, New York at pages 332-336 for a discussion regarding antibody specificity.

In one embodiment, if the antibody recognises a linear Tfr2 epitope, short peptides based on the antigen sequence can be produced and binding of the antibody to these peptides can be assessed using standard techniques.

In one embodiment, limited proteolytic digestion and mass spectrophotometry can be used to identify binding epitopes.

In one embodiment, the contact residues of the epitope are identified by X-ray crystallography. In one embodiment, the contact residues of the epitope are identified by cryo-electro microscopy. In one embodiment, the contact residues of the epitope are identified by a combination of limited proteolytic digestion and mass spectrometry.

Any feature of one configuration, example, embodiment or Aspect disclosed herein is combinable *mutatis mutandis* with any other configuration, example, embodiment or Aspect herein.

In order to implement the invention, it is also expedient to combine the above embodiments and features of the claims. The invention provides uses described herein and also corresponding methods, such as methods of diagnosis, treatment or prophylaxis in a subject.

### Examples

### Example 1: First Configuration Embodiments

The invention will be explained in greater detail in the following, with reference to some embodiments and accompanying Figures 1 to 4. In this case, the embodiments are intended to describe the invention but without having a limiting effect.

### Preparation of the Tfr2 extracellular domains (Tfr2-ECD)

The nucleic acid sequence of the entirety of the murine extracellular domains (ECD, aa 103-798) of Tfr2, including a 6x His tag, is carried out by Genscript (Germany). The recombinant His-Tfr2-ECD is expressed in Sf9 insect cells using the baculovirus expression system (pOCC211-Tfr2-ECD). Cell culture supernatants are collected and purified using a HisTrap column. After the step of washing using phosphate-buffered saline solution (PBS), the His-Tfr2ECD-protein is eluted with imidazole by means of PBS.

### Surface plasmon resonance measurement (SPR)

The interactions between Tfr2-ECD and BMPs (BMP-2, -4, -6, -7 by R&D Systems) and BMP receptors (BMPR-IA, BMPR-II by R&D Systems) are analyzed using Biacore^{™} T100 (GE Healthcare).

For this purpose, Tfr2-ECD is immobilized on a Series S Sensor Chip C1 (GE Healthcare), by means of coupling the amino groups at 25°C. The carboxyl groups on the chip surface are activated for 7 minutes using a mixture of 196 mM 1-ethyl-3-(3-dimethylaminopropyl) carbodiimide-hydrochloride and 50 mM n-hydroxysuccinimide at a flow rate of 10 µl/min. Subsequently, 5 µg/ml Tfr2-ECD, diluted with sodium acetate buffer (pH 4.5), is injected at a flow rate of 5 µl/min up to a relative occupancy of 200 RU. Non-reacted groups are inactivated by injecting 1 M thanolamine-HCI (pH 8.5) for 7 minutes at a flow rate of 10 µl/min. In order to prepare a reference surface, the same procedure is carried out but without Tfr2-ECD being injected.

The binding assays are carried out at 37°C at a flow rate of 30 µl/min. Each analyte is diluted by a running buffer (HBS-P, pH 7.4 together with 50 nM FeCl₃). BMPs are used in a concentration of 50 nM and BMP receptors are used in a concentration of 200 nM. The binding assays are carried out by means of analyte injection for 300 s over the Tfr2-ECD surface, followed by dissociation for 1000 s. The values of the binding level are read out, relative to the baseline, 10 s before the end of the injection and are corrected with respect to the molar mass. Following the dissociation for 1000 s, the chip surface is regenerated for 60 s by means of HBS-P together with 5 M NaCl and 50 mM NaOH and is stabilized for 1000 s. The binding parameters are determined using the Biacore^{™} T100 evaluation software 2.03.

Fig. 2 shows the binding of the protein according to the invention to various BMP ligands (BMP2, BMP-4, BMP-6, BMP-7).

### BMP-2-competitive ELISA (enzyme linked immunosorbent assay)

In order to carry out the BMP-2-competitive ELISA, the Duo Set BMP-2 ELISA kit by R&D Systems is used. After the plate has been coated, overnight, with BMP-2 capture antibodies, 1.5 ng/ml BMP-2 together with increasing concentrations of Tfr2-ECD or BMPR-IA (positive control, R&D Systems) is added to the assay. Following incubation for 1 hour at room temperature and intensive washing, the detection antibody is added according to the manufacturer's specifications and the amount of BMP-2 not bound to Tfr2-ECD or BMPR-IA that is bound to the capture and detection antibody is quantified.

Fig. 3 shows the binding of the protein according to the invention to BMPs, in particular BMP-2. As the concentration of the protein according to the invention increased, the signal of the BMP-2 detection antibody reduced, despite the BMP-2 concentration remaining the same. The progression was comparable to the binding of BMP-2 to the BMP receptor I (BMPR-I).

### Mouse model of heterotopic ossification (HO)

Male and female C57BL/6 mice are used for the HO model. The HO is triggered by injecting BMP-2 into the muscle (Wosczyna et al. 2012).

All the mice are fed with standard feed and water *ad libitum* and are kept in groups of five mice per cage. The mice are exposed to a 12-hour light/dark cycle and air cooling to 23°C (no special pathogen-free room). Enrichment is provided in the form of cardboard houses and bedding material. The mice are randomly divided into the different treatment groups and the assays are subsequently carried out as blind experiments.

The HO is examined by means of treatment using 2.5 µl of a 1 mg/ml recombinant BMP-2 solution (Thermo Fisher Scientific) or 2.5 µl of a 1 mg/ml Tfr2-ECD mixed with 47.5 µl Matrigel (BD Bioscience) at 0°C. For combination treatment, 2.5 of a 1mg/ml recombinant BMP-2 solution is mixed with 2.5 µl of a 1 mg/ml Tfr2-ECD and 45 µl Matrigel. The Matrigel mixtures are injected into the *musculus tibialis anterior* of 10-week-old female wild-type mice. The legs are examined after two weeks.

### µCT (microtomography) and bone micromineralization density

The bone microarchitecture is analyzed using vivaCT40 (Scanco Medical, Switzerland). The entire lower leg bones are measured at a resolution of 10.5 µm using X-rays of 70 kVp, 114 mA and at an integration time of 200 s. Predefined scripts from Scanco are used to analyze the bone (#1).

The mouse model of HO develops a BMP-2-induced ossification of the muscle tissue. Fig. 4 shows the inhibition of the ossification or HO in mice (C57BL/6 mice) by Tfr2-ECD, by means of binding of BMP-2.

### Statistical analysis

The data are specified as the average value ± standard deviation (SD). Graphs and statistics are created using Graphpad Prism 6.0-Software. The normality of the data is determined by means of the Kolmogorow-Smirnov Test. In the case of normal distribution, statistical evaluations are carried out by means of two-sample comparison, using the Student's T-Test two-sample test. A one-way analysis of variance (ANOVA) is used for experiments having more than two groups. A two-way ANOVA comprising a Bonferroni post hoc test is used to analyze the treatment effects. If data do not correspond to the normal distribution, the Mann-Whitney U test and the Wilcoxon signed-rank test are used for the data analysis.

### Example 2: Second Configuration Embodiments

In an embodiment, the bone measurements of Tfr2-deficient mice, taken by means of micro computed tomography, show that the loss of Tfr2 leads to a higher trabecular bone volume (bone volume/total volume, BV/TV) (Fig. 5) and a thicker cortical bone in mice (Fig. 6). In addition, the quality of the bone is better in the absence of Tfr2 (stiffness parameter) (Fig. 7) than compared with wild-type mice (WT). The mechanical properties such as the break resistance of the bone were determined by means of a three-point flexural test in which the force that has to be applied in order to break the bone is determined.

The studies of Tfr2-deficient mice further show, in Fig. 8 and Fig. 9, that the deletion of Tfr2 leads to a higher bone turnover, i.e. to a higher rate of bone formation (bone formation rate per bone surface (BFR/BS) (Fig. 8) and to a higher rate of bone resorption (osteoclast surface per bone surface (Oc.S/BS) (Fig. 9).

### Example 3: Transferrin receptor 2 controls bone mass and pathological bone formation via BMP and Wnt signaling

### Abstract

Transferrin receptor 2 (Tfr2) is mainly expressed in the liver and controls iron homeostasis. Here, we identify Tfr2 as a regulator of bone homeostasis that inhibits bone formation. Mice lacking Tfr2 display increased bone mass and mineralization independent of iron homeostasis and hepatic Tfr2. Bone marrow transplantation experiments and studies of cell-specific Tfr2 knockout mice demonstrate that Tfr2 impairs BMP-p38MAPK signaling and decreases expression of the Wnt inhibitor sclerostin specifically in osteoblasts. Reactivation of MAPK or overexpression of sclerostin rescues skeletal abnormalities in Tfr2 knockout mice. We further show that the extracellular domain of Tfr2 binds BMPs and inhibits BMP-2-induced heterotopic ossification by acting as a decoy receptor. These data indicate that Tfr2 limits bone formation by modulating BMP signaling, possibly through direct interaction with BMP either as a receptor or as a co-receptor in a complex with other BMP receptors. Finally, the Tfr2 extracellular domain may be effective in the treatment of conditions associated with pathological bone formation.

Iron is indispensable for red blood cell production, bacterial defense, and cellular respiration', however, iron excess is cytotoxic. Therefore, systemic iron levels are maintained in a narrow range to avoid iron deficiency and anemia, or iron overload leading to multi-organ damage. Amongst other organs, bone is highly susceptible to changes in iron homeostasis. Bone mineral density is negatively associated with systemic iron concentrations² and patients suffering from hereditary hemochromatosis, a disorder characterized by iron overload, develop premature osteoporosis³. Despite these observations, the relationship between iron homeostasis and bone turnover remains largely unexplored.

Systemic iron concentrations are maintained by balancing dietary iron absorption and iron recycling from the reticuloendothelial system'. Hepcidin is a hepatic peptide hormone and key regulator of iron homeostasis⁴. By binding to ferroportin, an iron exporter, hepcidin causes the internalization and degradation of ferroportin, thereby limiting iron export into the circulation. Dysregulation of this mechanism leads to iron overload. Accordingly, mutations in the gene encoding hepcidin or hepcidin-regulating genes cause hereditary hemochromatosis⁵.

Transferrin receptor 2 (Tfr2) is a key regulator of hepcidin. Similar to humans, mice with global or liver-specific deletion of *Tfr2* accumulate iron in the liver⁶⁻⁹. Tfr2 is proposed to control iron homeostasis by regulating hepcidin expression and has two isoforms: Tfr2α, which represents the full-length protein and regulates iron homeostasis in the liver, and Tfr2β, which lacks the intracellular and transmembrane domains and plays an important role in iron efflux in the spleen⁸. To date, the mechanisms whereby Tfr2 senses and regulates systemic iron concentrations remain incompletely understood. However, holo-transferrin can bind Tfr2 and prolong its half-life¹⁰. Thus, Tfr2 has been postulated to sense circulating iron and activate hepcidin in response to elevated transferrin saturation.

Tfr2-deficient hepatocytes have reduced bone morphogenetic protein (BMP) and p38MAPK/ERK signaling¹¹⁻¹³, implicating these pathways in its signal transduction. Although BMP signaling is mostly known for its critical role in bone development and postnatal bone homeostasis¹⁴, it has also emerged as an important regulator of iron homeostasis. Deficiency of several components of the BMP pathway *(Bmpr1a, Bmpr2, Acvr1, Acvr2a, Smad4, Bmp2, Bmp6*) or their pharmacological inhibition result in iron overload¹⁵⁻²⁰. Moreover, hemojuvelin, another regulator of hepcidin expression, has been identified as a hepatic BMP co-receptor¹⁶, further linking BMP signaling to iron homeostasis. Importantly, activating mutations in one of the BMP receptors that controls iron homeostasis, *ACVR1,* cause a rare human disorder, fibrodysplasia ossificans progressiva (FOP), which is characterized by excessive heterotopic ossification²¹. Thus, balancing BMP signaling is necessary to maintain bone and iron homeostasis in a physiological range.

Recent evidence indicates Tfr2 is not restricted to the liver, but is also expressed in erythroid progenitors to ensure their proper differentiation^{8,22,23}. As BMP signaling has a critical role in the skeleton^{14,24}, we hypothesized that Tfr2 may possess additional extrahepatic functions and regulate bone homeostasis. Here, we demonstrate that Tfr2 is a novel negative regulator of bone turnover. By binding BMP ligands, Tfr2 activates p38MAPK signaling in osteoblasts to induce expression of the Wnt inhibitor sclerostin and limit bone formation. Finally, by taking advantage of the BMP-binding property of the Tfr2 extracellular domain, we show that this protein fragment effectively inhibits heterotopic ossification in two preclinical models, suggesting it may also be efficacious to treat disorders of pathological bone formation.

### Results

### Tfr2-deficiency leads to high bone mass

To investigate whether the iron-sensing receptor Tfr2 regulates bone homeostasis, we studied *Tfr2*^{*-*/*-*} mice, which are iron overloaded. Consistent with previous reports^{8,25}, the transferrin saturation, serum iron and ferritin concentrations, and iron content in the liver were increased in *Tfr2*^{*-*/*-*} mice compared to wild-type (WT) mice (Suppl. Fig 10a-d). In addition, atomic absorptiometry revealed a higher iron content in the cortical bone of *Tfr2*^{*-*/*-*} mice (Suppl. Fig. 10e). As iron overload is associated with bone loss³, we expected a decreased bone volume in *Tfr2*^{*-*/*-*} mice. However, in contrast to the low bone mass phenotype of mice with diet-induced iron overload and in different mouse models of hemochromatosis, including *Hfe*^{-/-} mice²⁶ and *Fpn^{C326S}* mutant mice²⁷ (Suppl. Fig. 11a-c), *Tfr2*^{*-*/*-*} mice displayed a 1.5-3-fold higher trabecular bone volume in the femur and the vertebrae and a 1.5-fold higher cortical bone density compared to WT controls (Fig. 10a-b). High bone mass was independent of sex and declined with age (Suppl. Fig. 12a-b). At a structural level, *Tfr2*^{*-*/*-*} vertebrae had increased trabecular number (Tb.N) and thickness (Tb.Th) and decreased separation (Tb.Sp) (Fig. 10c-e). Furthermore, *Tfr2*^{*-*/*-*} mice had increased trabecular bone micro-mineralization density (Suppl. Fig. 12c), which together with the increased bone volume enhanced bone strength (Fig. 10f).

We performed dynamic and static histomorphometry to determine whether the high bone mass phenotype was a consequence of increased bone formation or decreased bone resorption. *Tfr2* deficiency resulted in an increase in both osteoblast and osteoclast parameters. The bone formation rate and the serum concentration of the bone formation marker pro-collagen type I Nterminal peptide (P1NP) were elevated more than two-fold in *Tfr2*^{*-*/*-*} mice, and the number of osteoclasts and serum concentration of the bone resorption marker C-terminal telopeptide of type I collagen (CTX) were similarly increased (Fig. 10g-l). The high bone turnover was present in both males and females at all ages studied (Suppl. Fig. 12d-e). Interestingly, *Tfr2*^{*-*/*-*} mice were not protected from ovariectomy-induced bone loss, but lost even more bone than WT mice (Fig. 10m). Taken together, these data demonstrate that Tfr2 does not only control iron homeostasis, but also bone turnover.

### High bone mass in Tfr2-deficient mice is independent of hepatic iron status or Tfr2 expression in the liver

As *Tfr2*^{*-*/*-*} mice have iron overload and high bone mass, whereas iron overload is commonly associated with decreased bone mass^{3,28}, we investigated whether abnormal iron metabolism contributes to the skeletal phenotype in *Tfr2*^{*-*/*-*} mice. Thus, *Tfr2*^{*-*/*-*} mice received an iron-free diet for 8 weeks from weaning or were treated with the iron-chelator deferoxamine for three weeks from 10 weeks of age. Despite successful iron depletion by both regimens, bone mass remained elevated in *Tfr2*^{*-*/*-*} mice (Fig. 11a-d), indicating the high bone mass phenotype in *Tfr2*^{*-*/*-*} mice is independent of the hepatic iron status.

To corroborate these findings, we studied two distinct *Tfr2* mutant mouse models, which globally lack *Tfr2*β but have contrasting *Tfr2*α expression that results in divergent abnormalities of iron homeostasis⁸: *Tfr2* knock-in mice (KI) globally lack *Tfr2*βbut have normal *Tfr2*α and thus normal iron parameters. By contrast, hepatocyte-specific *Tfr2* knock-out (LCKO) mice globally lack *Tfr2*β*,* and have *Tfr2*α deficiency restricted to the liver, but are severely iron-overloaded. Both models had comparable bone volume fractions compared to controls (Fig. 11e-f), indicating that neither *Tfr2*β nor hepatic *Tfr2*α play a role in the control of bone homeostasis.

### Tfr2-deficiency in osteoblasts drives the high bone mass phenotype

To explore whether Tfr2 regulates bone mass directly via its expression in skeletal cells, we determined expression of *Tfr2*α and *Tfr2*β in various mouse tissues. As expected, *Tfr2*α was predominantly expressed in the liver with the next highest expression in femoral cortical bone (Suppl. Fig. 13a). *Tfr2*β mRNA expression was also detected in femoral cortical bone, although at a much lower level (C_{T} value spleen (positive control): 26, C_{T} value bone: 32). Using an antibody that binds to the extracellular domain of Tfr2 and thus detects both *Tfr2*α and *Tfr2*β isoforms, we confirmed expression of Tfr2 in vertebral bone sections, showing Tfr2-positive osteoclasts, osteoblasts, and osteocytes (Fig. 12a, d). Staining of bone sections from *Tfr2*^{*-*/*-*} mice showed no non-specific binding of the Tfr2 antibody (Suppl. Fig. 12b). Osteoclasts and osteoblasts differentiated from the bone marrow of WT mice both expressed *Tfr2*α and *Tfr2*βmRNA transcripts *ex vivo* but expression of *Tfr2*β was very low in each cell type (data for *Tfr2*β not shown). *Tfr2*α was readily detectable in osteoclasts and osteoblasts, with peak levels of expression in mature osteoclasts (day 7/7) and in immature osteoblasts (day 7/21) (Fig. 12b, e). Immunocytochemistry confirmed Tfr2 expression in osteoclasts and osterix-expressing osteoblasts that were differentiated *ex vivo* (Fig. 12c, f). Subcellular fractioning of osteoblasts further localized the majority of Tfr2 to the membrane fraction (Fig. 12g). A low signal was also detected in the cytoplasm.

To determine if Tfr2 in osteoclasts or in osteoblasts regulates bone turnover, we performed reciprocal bone marrow transplantations. In WT and *Tfr2*^{*-*/*-*} mice, bone marrow transplantation had no effect on vertebral or femoral bone volume irrespective of donor genotype (Fig. 12h), suggesting that Tfr2 deficiency in the hematopoietic compartment is not responsible for the high bone mass phenotype in *Tfr2*^{*-*/*-*} mice. Consistent with these findings, deletion of *Tfr2* specifically in the myeloid lineage (*Lysm-cre*) and in mature osteoclasts (*Ctsk-cre*) did not affect bone volume at the spine (Fig. 12i). Femoral bone volume, however, was decreased in *Tfr2*^{*f*/}*^{f};Lysm-cre* mice, but not in *Tfr2*^{*f*/}*^{f};Ctsk-cre* mice (Suppl. Fig. 20a). By contrast, deletion of *Tfr2* in osteoblast progenitors, in which *Tfr2* expression is highest, increased bone mass at the femur and spine (Fig. 12j), increased trabecular number, and decreased trabecular separation (Fig. 12k). Bone formation was increased in *Tfr2*^{*f*/}*^{f};Osx-cre,* as reflected by higher serum P1NP levels (Fig. 12I) and a higher bone formation rate (Fig. 12m). Finally, deletion of *Tfr2* in osteoblasts did not change osteoclast numbers, but tended to increase serum levels of CTX (Fig. 12n-o). Consistent with published *Tfr2*^{*f*/}*^{f};Lysm-cre* mice²⁹, *Tfr2*^{*f*/}*^{f};Ctsk-cre* and *Tfr2*^{*f*/}*^{f};Osx-cre* mice also showed a normal liver iron content (Suppl. Fig. 20b-c). Taken together, these data indicate that Tfr2 predominantly in osteoblasts regulates bone formation, but does not contribute to systemic iron homeostasis.

### Tfr2-deficiency in osteoblasts attenuates BMP-MAPK signaling and results in decreased expression of Wnt inhibitors

As the data indicate a direct role for Tfr2 in osteoblasts, we performed genome-wide RNA sequencing analysis using primary osteoblasts from WT and *Tfr2*^{*-*/*-*} mice to identify signaling pathways affected by deletion of *Tfr2.* A total of 5,841 differentially expressed genes (DEGs) were identified. We performed gene ontology analysis to determine the biological processes affected by *Tfr2* deficiency. Genes upregulated in *Tfr2*^{/*-*} osteoblasts belonged to the biological processes of negative regulation of protein secretion and muscle systems. By contrast, genes involved in ossification, extracellular matrix organization, negative regulation of Wnt signaling, and Smad phosphorylation were downregulated in *Tfr2*^{*-*/*-*} osteoblasts (Suppl. Fig. 21a). These data are consistent with molecular function and cellular component analyses, which revealed underrepresentation of genes involved in glycosaminoglycan and heparin binding, and BMP receptor binding as well as proteinaceous extracellular matrix and collagen formation (Suppl. Fig. 21a). Gene set enrichment analysis further demonstrated underrepresentation of genes involved in late osteoblastic differentiation and Wnt signaling, the latter associated with marked suppression of the Wnt inhibitor Dickkopf-1 (*Dkk1*) (Suppl. Fig. 21b-c). The complete list of significantly enriched gene sets is provided in the Suppl.
Table 2.

*Dkk1* and Sost (encoding the Wnt inhibitor sclerostin) were among the 25 most downregulated genes in *Tfr2*^{*-*/*-*} osteoblasts *ex vivo* (Fig. 13a). Reduced expression of the two Wnt inhibitors was verified by qPCR and is consistent with increased expression of the Wnt target genes (*Axin2, Lef1, Cd44)* (Suppl. Fig 21c). *Dkk1* and Sost mRNA levels were also down-regulated in osteoblasts obtained from *Tfr2*^{*f*/}*^{f};Osx-cre* mice (Fig. 13b). Furthermore, expression of the osteocyte-associated genes *Phex* and *Dmp1* was decreased (Suppl. Fig 21c). Importantly, impaired expression of osteocytic markers was not due to reduced osteocyte number in *Tfr2*^{*-*/*-*} bone (WT: 8.73±2.74 vs *Tfr2*^{*-*/}*⁻:* 9.77±0.85 osteocytes/bone volume fraction). Low concentrations of sclerostin and Dkk1 were further detected in the serum of *Tfr2*^{*-*/*-*} mice (Fig. 13c), along with a greater proportion of osteoblasts/osteocytes with high expression of β-catenin and axin-2 (Fig. 13d). Finally, increased Wnt signaling was demonstrated in *Tfr2*^{*-*/*-*} osteoblasts differentiated for 7 days using Western blot analysis of β-catenin (Fig. 13e).

Deep sequencing analysis also suggested decreased BMP signaling in *Tfr2*^{*-*/*-*} osteoblasts, and recent studies indicate that *Sost* and *Dkk1* are downstream targets of BMP signaling^{30,31}. Thus, we analyzed BMP target genes as well as the basal canonical (Smad) and non-canonical (MAPK) BMP signaling pathways in *Tfr2*^{*-*/*-*} and WT osteoblasts. *Smad6* and *Id1* were significantly downregulated in *Tfr2*^{*-*/*-*} osteoblasts, while *Id2* was not different (Suppl. Fig. 21c). Both, Smad 1/5/8 phosphorylation as well as ERK and p38 activation were decreased in *Tfr2*^{*-*/*-*} compared to WT osteoblasts (Fig. 13e). Moreover, activation of BMP signaling following BMP-2 treatment demonstrated that Smad activation was delayed in *Tfr2*^{*-*/*-*} osteoblasts, whereas activation of p38MAPK and ERK was persistently impaired (Fig. 13f-g). The reduced activation of noncanonical BMP signaling in *Tfr2*^{-/-} osteoblasts was not restricted to BMP-2, but was also observed after BMP-4 and to a lesser extent BMP-6 stimulation (Fig. 13h, Suppl. Fig. 21d). Overall, *Tfr2* deficiency in osteoblasts results in impaired BMP signaling and increased activation of the Wnt pathway.

### Reactivation of MAPK signaling or overexpression of sclerostin rescues high bone mass in Tfr2-deficiency

We next investigated the mechanisms underlying the Tfr2-mediated regulation of Sost expression, as this may be a major driver of the Tfr2-dependent effects on bone. Using *Tfr2*^{*-*/*-*} osteoblasts *in vitro,* we confirmed the lack of induction of Sost expression after stimulation with BMP-2, BMP-4, and BMP-7 (Fig. 14a). Conversely, overexpression of *Tfr2* in WT and *Tfr2*^{*-*/*-*} osteoblasts markedly increased Sost, in particular following stimulation with BMP-2 (Fig. 14b, Suppl. Fig. 22a). To test the impact of sclerostin in producing the high bone mass in *Tfr2*^{*-*/*-*} mice, we crossed *Tfr2*^{*-*/*-*} mice with mice overexpressing human SOST in late osteoblasts/osteocytes (under the *Dmp1-*8kb-promoter). Overexpression of SOST significantly reduced the vertebral trabecular bone volume in *Tfr2*^{*-*/*-*} mice (Fig. 14c) and normalized the bone formation rate (Fig. 14d). The osteoclast-covered bone surface was higher in *Tfr2*^{*-*/*-*} and WT mice overexpressing SOST in osteoblasts/osteocytes compared to mice with normal Sost expression (Suppl. Fig. 22b).

Previous studies indicated that BMPs stimulate Sost expression via the BMP-Smad and BMPMAPK pathways^{30,31}. Because pERK and pp38 were most markedly reduced in *Tfr2*^{*-*/*-*} osteoblasts, and neither Smad1 nor Smad4 overexpression in *Tfr2*^{*-*/*-*} osteoblasts restored Sost mRNA levels (Suppl. Fig. 22c-f), we reactivated the MAPK pathways using anisomycin to rescue Sost expression. Treatment with anisomycin induced ERK and p38 phosphorylation (Suppl. Fig. 22g), and increased Sost mRNA expression in *Tfr2*^{*-*/*-*} and WT osteoblasts: 19-fold in *Tfr2*^{*-*/*-*} osteoblasts and 14-fold in WT osteoblasts at 100 nM anisomycin (Fig. 14e). Similarly, treatment of *Tfr2*^{*-*/*-*} mice with anisomycin for 3 weeks increased serum levels of sclerostin (Fig. 14f), reduced osteoblast numbers (Fig. 14g) and decreased bone volume back to WT levels (Fig. 14h). Osteoclast numbers were not altered by anisomycin treatment (Suppl. Fig. 22h). Finally, we investigated which specific MAPK pathway, ERK or p38, regulates Sost expression. Only the overexpression of *Mapk14* (encoding for p38α), but not *Mapk1* (encoding for ERK2), restored reduced Sost levels in *Tfr2*^{*-*/*-*} osteoblasts (Fig. 20i-j). Thus, Tfr2 controls bone mass by inducing Sost expression via the p38MAPK signaling pathway.

### Tfr2 is a novel interaction partner of BMPs

Finally, we asked how Tfr2 can lead to impaired BMP-MAPK signal transduction in osteoblasts and therefore explored whether Tfr2 can act as a BMP receptor. We generated a protein fragment containing the extracellular domain of Tfr2 (Tfr2-ECD), confirmed its presence using SDS-PAGE and Western blot (Suppl. Fig. 23a), and performed surface plasmon resonance (SPR) analysis. Tfr2-ECD immobilized on the sensor chip bound BMPs-2, 4, 6 and 7 more avidly than holotransferrin (Tf) (Fig. 21a, Suppl. Fig. 23b), the only known Tfr2 ligand¹⁰. BMPs-2, 4, and 6 also bound to Tfr2-ECD at high salt concentrations, which were used to reduce non-specific binding, even though at a lower level (Suppl. Fig. 23c). Tfr2-BMP binding was further verified using the inverse approach using Tfr2-ECD as an analyte and BMP-2 or BMP-4 immobilized on the sensor chip (Suppl. Fig. 23d-e). Using this approach, we determined K_{d} values for Tfr2/BMP-2 (488.0 ± 37.0 nM) and Tfr2/BMP-4 (409.1 ± 39.0 nM) binding via steady state analysis. Holo-Tf bound to Tfr2 at micromolar concentrations suggesting a K_{d} value in the micromolar range (Suppl. Fig. 23f). Holo-Tf and BMP-2 did not compete for binding to Tfr2, as the sequential injection of either BMP2 followed by holo-Tf, or holo-Tf followed by BMP-2 did not change the initial binding response (Suppl. Fig. 23g-h). Interestingly, the concomitant injection of BMP-2 and holo-Tf led to a much stronger binding response to Tfr2 than either analyte alone (Fig. 15b). As BMPs normally signal through a receptor complex consisting of the type I and type II BMP receptors, we tested whether BMPRs bind to Tfr2-ECD. Both BMPR-IA and BMPR-II had a binding response weaker than BMPs (Fig. 15a, Suppl. Fig. 23i). The physical interaction of Tfr2 and BMPR-IA was further investigated using a cell system in which they were both overexpressed. Their interaction was confirmed by co-immunoprecipitation and was not affected by the presence of BMP-2 (Suppl. Fig. 23j). BMP-2 binding to the Tfr2-ECD was further verified using a competitive sandwich ELISA with BMPR-IA as a control (Fig. 15c). Additional SPR experiments revealed that BMPR-IA competes with Tfr2 for BMP-2 binding, as adding increasing concentrations of BMPR-IA reduced the binding of BMP-2 to Tfr2-ECD (Fig. 15d). Of note, high nanomolar concentrations of BMPR-IA were required for competing with Tfr2/BMP-2.

We validated the BMP ligand binding property of Tfr2-ECD *in vivo* using a heterotopic ossification model. In this model, BMP-2 is injected into the anterior tibialis muscle of mice, which leads to muscular ossification³². While BMP-2 alone led to heterotopic ossification of the muscle in WT mice, the addition of Tfr2-ECD completely abrogated this effect (Fig. 15e, Suppl. Fig 24a-b), suggesting that Tfr2 binds BMP-2 and prevents it from binding to its cognate BMPR. Similar experiments in *Tfr2*^{*-*/*-*} mice demonstrated increased heterotopic ossification following BMP-2 injection as compared to WT mice, which was significantly inhibited by co-application of Tfr2-ECD (Fig. 15e, Suppl. Fig 25a-b). Thus, in addition to confirming functional BMP-binding activity of the Tfr2-ECD *in vivo,* these data emphasize the role of Tfr2 as a negative regulator of ossification in a BMP-dependent context.

**Tfr2-ECD potently inhibits heterotopic ossification in two distinct preclinical models** Due to the robust effect of the Tfr2-ECD to diminish BMP-2-induced heterotopic ossification, we compared Tfr2-ECD with palovarotene, a selective retinoic acid receptor-agonist that indirectly inhibits BMP signaling³³ and is currently under clinical investigation for the treatment of FOP. Tfr2ECD was either used as a single local treatment into the muscle or as a systemic treatment (i.p. injections every other day). Both regimens reduced BMP-2-induced heterotopic ossification in WT mice after two weeks with similar efficacy to daily palovarotene administration (Fig. 15f). Investigation of the chondrogenic phase of heterotopic ossification at day 8 in WT mice revealed that both systemic Tfr2-ECD and palovarotene treatment suppressed the number of chondrocytes and the production of cartilage (Suppl. Fig. 24c-d). No adverse effects of systemic Tfr2-ECD treatment were observed on blood counts, iron parameters, bone homeostasis or the gross morphology of internal organs. Finally, we tested both compounds in a model of trauma-potentiated heterotopic ossification, a frequent complication after trauma, blast injuries, or hip replacement surgeries. A single dose of Tfr2-ECD inhibited new bone formation in the muscle comparable to palovarotene (Fig. 15g). Daily treatment with ibuprofen, a frequent treatment of heterotopic ossification after hip surgeries³⁴, did not prevent trauma-induced heterotopic ossification (Fig. 15g). These data indicate that Tfr2-ECD is a potent inhibitor of heterotopic ossification and represents a potential new therapeutic strategy for treating disorders of excessive bone formation.

### Discussion

Using a series of genetically modified mice and *in vitro* analyses, these studies identify a new role for Tfr2 as a modulator of BMP and Wnt signaling in osteoblasts. Tfr2 interacts with BMP ligands and receptors, activates p38MAPK signaling, and induces expression of the Wnt inhibitor *Sost*. This in turn blocks canonical Wnt signaling, thereby limiting bone formation and bone mass accrual (Fig. 14k). Further, exploiting the BMP-binding property of the Tfr2-ECD in form of a decoy receptor shows promise as a novel therapeutic strategy to prevent heterotopic ossification (Fig. 15h), which is of particular interest as there are currently no specific treatments for congenital or trauma-induced heterotopic ossification.

Besides its well-known function in the regulation of systemic iron levels⁶⁻⁹, Tfr2 ensures proper erythropoiesis^{8,22,23}. Our study has now identified a novel extrahepatic role of Tfr2, control of bone mass via direct actions in osteoblasts, even though minor effects in myeloid cells including early osteoclasts cannot be excluded. This appears to be a unique property of Tfr2 among the other iron-regulating proteins, as all other investigated mouse models of hemochromatosis display low bone mass. Accordingly, others have shown low bone mass in patients with *HFE*-dependent hemochromatosis³ and in *Hfe-* and hepcidin-deficient mice^{35,36}. In both cases, suppressed bone formation was proposed as the main underlying mechanism of low bone mass^{35,37}. However, as both *Hfe*-and hepcidin-deficient mice are iron-overloaded, it is unclear whether the low bone mass is an indirect result of the negative effects of iron overload, or whether *Hfe* and hepcidin exert direct actions in bone cells. Importantly, the high bone mass in *Tfr2*-deficient mice was independent of the iron status and the hepatic function of Tfr2, indicating Tfr2 has distinct roles in osteoblasts (i.e. control of matrix production) and hepatocytes (i.e. regulation of hepcidin expression and systemic iron homeostasis).

Even though Tfr2 has been known as a regulator of iron homeostasis for over 15 years, its mechanisms of action have remained elusive. Decreased levels of Smad1/5/8 and MAPK/ERK signaling in *Tfr2*-deficient hepatocytes suggested that BMP signaling may be involved^{11,13,38}, but it remained unclear how Tfr2 activates BMP signaling. Previous studies in hepatocytes suggested that Tfr2 forms a ternary complex with Hfe and hemojuvelin to activate hepcidin expression¹². Our data, however, provide *in vitro* and *in vivo* evidence, which demonstrates that Tfr2 can bind BMPs directly and activate downstream signaling. Binding of BMP-2 to Tfr2 was more than 10-fold higher than that of holo-Tf, the only known ligand for Tfr2¹⁰. Compared to BMP-BMPR interactions^{39.40}, BMP-Tfr2 binding was markedly lower, suggesting that Tfr2 may act to fine-tune BMP signaling. As our studies also showed a direct interaction of Tfr2 with BMPRs, it remains to be investigated whether Tfr2 binds BMPs alone or within a multi-receptor complex with BMPRs and/or other BMP co-receptors. Despite these first indications of Tfr2 being a BMP (co)-receptor, additional experiments will be required to define accurate binding affinities that account for stoichiometry, the possibility of receptor dimerization or oligomerization, and Tfr2-ECD purity. Interestingly, the combination of holo-Tf and BMP-2 bound much more avidly to Tfr2 than either holo-Tf or BMP-2 alone, suggesting that holo-Tf may exhibit significant Tfr2 binding only in the presence of BMPs. This may be of particular importance as hepatic endothelial cells have been identified as the main producers of BMP-2 and BMP-6 that act locally on hepatocytes to control hepcidin expression and iron homeostasis^{41,42}. While hemojuvelin has been recognized to transmit the signal of BMP-6 to modulate hepcidin expression, BMP-6 can still induce hepcidin expression in hemojuvelin knock-out mice⁴³, suggesting that other receptors must be involved. Thus, the newly identified BMP-binding properties of Tfr2 may represent the missing link in the regulation of hepcidin via BMPs.

Our study further showed that BMP downstream signaling, in particular the BMP-p38MAPK pathway, is impaired in *Tfr2*^{*-*/*-*} osteoblasts resulting in reduced expression of the canonical Wnt inhibitors *Dkk1* and *Sost*, which are both potent negative regulators of bone formation⁴⁴⁻⁴⁶. Recent work has shown that BMP-2 stimulates expression of *Dkk1* and *Sost* by activating BMPdependent Smad signaling and, in the case of *Dkk1,* also through MAPK signaling via ERK and p38^{30,47}. More recent studies, including our own show that *Sost* expression is also induced by p38MAPK signaling in osteoblasts^{30,48}. Accordingly, anisomycin treatment, which activates all three MAPKs⁴⁹, rescued *Sost* expression and restored bone mass in *Tfr2*^{*-*/*-*} mice. Similar to the phenotype of *Tfr2*^{*-*/*-*} mice and counterintuitive to the supportive actions of BMP signaling on osteoblastic bone formation, targeted disruption of *Bmpr1a* or *Acvr1* in osteoblasts impairs expression of *Sost* and results in high bone mass^{47,50}. In addition, treatment of Bmpr1a-deficient calvaria with recombinant sclerostin *ex vivo* restored normal bone morphology⁴⁷, similarly as overexpression of *SOST* in *Tfr2*^{*-*/*-*} mice reduced bone volume back to WT levels. However, *Tfr2*deficient mice do not fully phenocopy the skeletal phenotype of *Bmpr1a-* or *Sost*-deficient mice. Considering osteoblast/osteocyte-specific knock-out strains, deletion of all three genes leads to high bone mass. However, while *Bmpr1a*-conditional knock-out mice have a low bone turnover^{30,47,51}, *Tfr2*-conditional knock-out mice have a high bone formation rate and normal osteoclast parameters, and *Sost*-conditional knock-out mice have a high bone formation rate⁵². Osteoclast parameters have not been reported in *Sost*-conditional knock-out mice, but are normal in *Sost*^{*-*/*-*} mice⁴⁴. While an increase in bone formation appears the predominant mechanism of high bone mass in *Tfr2-* and *Sost*-conditional knock-out mice, the main driver of high bone mass in Bmpr1a-conditional knock-out mice appears to be reduced osteoclastogenesis due to a low RANKL-to-OPG ratio in osteoblasts^{30,47}. This mechanism was reported to be independent of Wnt signaling, as overexpression of *Sost* did not rescue the osteoclast phenotype in *Bmpr1a*conditional knock-out mice⁴⁷. By contrast, *Tfr2*^{*-*/*-*} mice have elevated osteoclast numbers and an increased RANKL-to-OPG ratio (WT: 0.225±0.046, *Tfr2*^{*-*/}: 0.696±0.120, n=4, p=0.0003), but similar to Bmpr1a-conditional knock-out mice, this phenotype was not rescued by *Sost* overexpression. Interestingly, deficiency of *Bmpr2* in osteoblasts results in high bone mass accompanied by a high bone formation rate and normal bone resorption⁵³, suggesting that Tfr2 shares more similarities with Bmpr2 than Bmpr1a. Finally, *Bmpr1a*-conditional knock-out mice have disorganized bone matrix, leading to reduced bone strength^{54,55}. This is in contrast to *Tfr2*^{*-*/*-*} and *Sost*^{*-*/*-*} mice, which both have normal bone matrix organization and increased bone strength⁴⁴. Taken together, despite similarities, which propose Tfr2 acts in similar way or even in conjunction with BMPRs, additional pathways appear to mediate its effects on bone independent of BMP signaling. In sum, Tfr2 is clearly a critical regulator of Sost expression in osteoblasts and provides another link between BMP and Wnt signaling.

Finally, we show that the ability of the Tfr2-ECD to bind BMPs and act as a decoy receptor reduces heterotopic ossification in two distinct preclinical models. Heterotopic ossification is a serious and common medical complication after blast injuries, such as found in soldiers and civilians, burn victims, and recipients of total hip endoprostheses. Up to 30% of patients undergoing hip replacement surgery and 50% of severely wounded soldiers develop heterotopic ossification^{56,57}. Extensive heterotopic ossification is also a hallmark of FOP, a rare human disease caused by an activating mutation in the BMP type I receptor *ACVR1²¹.* Since the identification of this mutation, BMP signaling has been implicated in the pathogenesis of heterotopic ossification. To date, therapeutic options for FOP and trauma-induced heterotopic ossification are limited. Radiation and non-steroidal anti-rheumatic drugs are frequently used to inhibit surgery-induced heterotopic ossification with varying success³⁴. In our study, ibuprofen did not significantly reduce heterotopic ossification. In FOP, glucocorticoids are used to reduce inflammation during flare-ups. However, they do not block progressive ossification. Rapamycin, anti-activin antibodies, and palovarotene have recently been shown to reduce heterotopic ossification in preclinical models of FOP via different mechanisms^{33,58-60}. Palovarotene indirectly interferes with the BMP pathway and is currently the only drug under clinical investigation. Both, local and systemic treatment with Tfr2ECD inhibited heterotopic ossification to a similar extent as palovarotene. Systemic treatment with Tfr2-ECD did not show adverse effects on iron or bone metabolism within the 2 week treatment period, which is of relevance as mice lacking *Tfr2*β*,* which has a similar structure as Tfr2-ECD, have increased iron levels in the spleen⁸ and therefore, differences in iron metabolism may have been anticipated. In the future, longer and more extensive pharmacological studies are required to conclusively address the safety profile of Tfr2-ECD.

Taken together, we have uncovered Tfr2 as a novel regulator of bone mass via modulating the BMP-p38MAPK-Wnt signaling axis and identified Tfr2-ECD as a promising therapeutic option to treat heterotopic ossification and disorders of excessive bone formation.

### Methods

### Mice

Generation of *Tfr2*^{*-*/*-*} mice and *Tfr2* knock-in (*Tfr2-*KI) mice, which only lack the *Tfr2β* isoform, were previously described⁸. Conditional *Tfr2* knock-out mice were generated on the background of the *Tfr2*-KI mouse (129X1 /svJ), thereby producing cell-type specific *Tfr2α* knock-out mice which also lack *Tfr2β* globally. Liver-specific *Tfr2*-knock-out mice (LCKO) were generated using the albumincre (sv129 background). To delete *Tfr2* in osteoblast precursors the doxycycline-repressible osterix-cre (*Osx*-*cre*) was used⁶¹. Breeding pairs and mice up to the age of 5 weeks were kept on doxycycline (0.5 g/l). For the deletion of *Tfr2α* in mature osteoclasts the cathepsin K cre (*Ctskcre*) was used⁶². Lysozyme M (*Lysm-cre*) was used for deletion of *Tfr2* in early osteoclasts⁶³. *Tfr2*^{*f*/*f*};*Osx-cre, Tfr2*^{*f*/}*^{f};Lysm-cre,* and *Tfr2*^{*f*/}*^{f};Ctsk-cre* mice were on a mixed sv129/C57BL/6 background. Littermates were used as controls.

To obtain *Tfr2*-deficient mice with an overproduction of human sclerostin, *Tfr2*^{*-*/*-*} mice were crossed with *Dmp1-SOST* transgenic mice to obtain *Tfr2*^{*-*/*-*};*Dmp1-SOST*^{*+*/tg} mice⁶⁴. The production of ferroportin knock-in mice with a point mutation (C326S) and *Hfe* knock-out mice were described previously^{26,27}. All mice were routinely genotyped using standard PCR protocols.

### In vivo experiments

All animal procedures were approved by the institutional animal care committee and the Landesdirektion Sachsen. All mice were fed a standard diet with water *ad libitum* and were kept in groups of 5 animals per cage. Mice were exposed to a 12 h light/dark cycle and an airconditioned room at 23 °C (no specific pathogen free room). Enrichment was provided in forms of cardboard houses and bedding material. Mice were randomly assigned to treatment groups and the subsequent analyses were performed in a blinded-fashion.

*Bone phenotyping:* Male and female *Tfr2*^{*-*/*-*} and wild-type mice at 10-12 weeks of age were used. For the characterization of *Tfr2*^{*-*/*-*} mice, older mice (6 and 12 months) were also used. Male *Tfr2*^{*f*/*f*};*Osx-cre* and *Tfr2*^{*f*/}*^{f};Ctsk-cre* and the corresponding cre-negative littermate controls were sacrificed at 10-12 weeks for bone phenotype analysis.

*Ovariectomy:* Female 11-14-week-old WT or *Tfr2*^{*-*/*-*} mice were bilaterally ovariectomized or sham operated. After four weeks, mice were sacrificed for further analyses. Each group consisted of 510 mice.

*Iron-rich diet:* WT animals received a 2% iron-enriched standard diet from weaning (14 days old) until sacrifice (8 weeks of treatment). Four-five mice per group.

*Iron-free diet:* Male *Tfr2*^{*-*/*-*} and WT mice received an iron-free diet (Envigo, Italy) from weaning until 10 weeks of age. Control mice received a standard diet containing 0.2 g iron/kg food (GLOBAL DIET 2018, Envigo, Italy). Nine mice per group.

*Iron chelation:* Ten-week-old male *Tfr2*^{*-*/*-*} and WT mice received daily intraperitoneal injections of 250 mg/kg DFO (Sigma, Germany, dissolved in PBS) or PBS for three weeks. This experiment was performed two independent times with 3-5 mice.

*Full bone marrow transplantation:* Bone marrow cells were isolated from 12-week-old male *Tfr2*^{*-*/*-*} mice or WT controls. Two million cells were transplanted into lethally irradiated (8 Gy) male WT or *Tfr2*^{*-*/*-*} mice by retro-orbital venous plexus injection. Engraftment efficiency was monitored every four weeks using flow cytometry. After 16 weeks, mice were sacrificed for bone analyses. This experiment was performed twice with each 7-12 mice per group.

*Anisomycin treatment:* Female 11-week-old WT and *Tfr2*^{*-*/*-*} mice were treated with 5 mg/kg anisomycin (i.p.) 3x/week for three weeks. This experiment was performed twice with each 5 mice per group.

*Heterotopic ossification (HO):* The HO model was performed according to Wosczyna et al³². Briefly, 2.5 µl of 1 mg/ml recombinant BMP-2 (Thermo Fisher) or 2.5 µl of 1 mg/ml Tfr2-ECD were mixed with 47.5 µl matrigel (BD Bioscience) on ice. For the local combination treatment, 2.5 µl BMP-2 were mixed with 2.5 µl Tfr2-ECD and 45 µl matrigel. The matrigel-mixtures were injected into the midbelly of the tibialis anterior muscle of 10-week-old female WT and *Tfr2*-deficient mice. Some mice were treated daily with palovarotene through oral gavage using a previously published protocol⁵⁸. Palovarotene (Hycultec) was dissolved in DMSO and diluted 1:4 with corn oil. Mice received palovarotene at a dose of 100 µg/mouse for the first five days and 50 µg/mouse for the remainder of the experiment (days 6-14). Two weeks after BMP-2 injection, the legs were harvested for analysis. This experiment was performed three times with 3-11 mice per group. To analyze the *chondrogenic phase* of HO, we performed an experiment as described above that was terminated on day 8. This was performed once with 4-6 mice per group.

For *systemic Tfr2-ECD treatment,* WT mice were treated every other day with Tfr2-ECD intraperitoneally for two weeks. Mice received 250 µg Tfr2-ECD (10 mg/kg BW) per injection for the first 10 days after BMP-2/matrigel injection into the muscle and 125 µg per injection (5 mg/kg BW) for the remaining time. This experiment was performed once with 8-10 mice per group. *Drop-weight HO:* This experiment was performed according to Liu et al. with minor modifications⁶⁵. Female 10-12-week-old WT mice were anesthetized and placed on a ridge of a plastic container over which the right leg was bent so the femur was lying horizontally. Mice received an injection of 1 µg BMP-2 mixed in 50 µl matrigel. Afterwards, a stainless-steel ball of 16 g (16 mm diameter) was dropped from a distance of 80 cm height onto the quadriceps muscle. Mice either received a single dose of 1 µg Tfr2-ECD, which was co-injected with the BMP-2/matrigel mixture, or palovarotene (Hycultec), which was administered daily by oral gavage. Palovarotene was dissolved in DMSO and diluted 1:4 with corn oil. Mice received palovarotene at a dose of 100 µg/mouse for the first five days and 50 µg/mouse for the remainder of the experiment (days 621). One group of mice received ibuprofen via the drinking water at a dose of 100 mg/ml which was changed every other day⁶⁶. Mice received methamizole (200 mg/kg) to reduce pain for the entire duration of the experiment. This experiment was performed twice with 6 mice per group.

### Micro-CT, bone micromineralization density, and biomechanical testing

Bone microarchitecture was analyzed using the vivaCT40 (Scanco Medical, Switzerland). The femur and the fourth lumbar vertebra were imaged at a resolution of 10.5 µm with X-ray energy of 70 kVp, 114 mA, and an integration time of 200 ms. The trabecular bone in the femur was assessed in the metaphysis 20 slices below the growth plate using 150 slices. In the vertebral bone, 150 slices were measured between both growth plates. The cortical bone was determined in the femoral midshaft (150 slices). Pre-defined scripts from Scanco were used for the evaluation.

Bone micro-mineralization densities were determined by quantitative back scattered electronscanning electron microscopy (qBSE-SEM). Neutral buffered formalin fixed fourth lumbar vertebrae (L4) from 12 week old male mice were embedded in methacrylate. Longitudinal block faces were cut through specimens, which were then polished and coated with 25 nm of carbon using a high resolution sputter coater (Agar Scientific Stanstead UK). Samples were imaged using backscattered electrons at 20 kV, 0.4 nA and a working distance of 17 mm with a Tescan VEGA3 XMU (Tescan, Brno, Czech Republic) equipped with a Deben 24 mm 4-quadrant backscatter detector (Deben, Bury St. Edmunds, UK). Bone mineralization densities were determined by comparison to halogenated dimethacrylate standards, and an eight-interval pseudocolor scheme was used to represent the graduations of micro-mineralization, as previously described⁶⁷.

Three-point bending of the femur was conducted to assess bone strength. The femurs were stored in 70% ethanol and rehydrated in PBS prior to testing. Mechanical testing was performed using the zwicki-Line from Zwick, Germany. Load was applied to the anterior side of the femoral shaft to measure the maximum load at failure (Fmax, N).

### Bone histomorphometry

Mice were injected with 20 mg/kg calcein (Sigma) five and two days before sacrifice. Dynamic bone histomorphometry was performed as described previously⁶⁸. Briefly, the third lumbar vertebra and tibia were fixed in 4% PBS-buffered paraformaldehyde and dehydrated in an ascending ethanol series. Subsequently, bones were embedded in methacrylate and cut into 7 µm sections to assess the fluorescent calcein labels. Unstained sections were analyzed using fluorescence microscopy to determine the mineralized surface/bone surface (MS/BS), the mineral apposition rate (MAR), and the bone formation rate/bone surface (BFR/BS) as well as the bone volume/total volume (BV/TV), trabecular number (Tb.N), trabecular separation (Tb.Sp), and trabecular thickness (Tb.Th).

To determine numbers of osteoclasts, the femur and fourth lumbar vertebra were decalcified for one week using Osteosoft (Merck), dehydrated, and embedded into paraffin. Tartrate-resistant acid phosphatase (TRAP) staining was used to assess the osteoclast surface per bone surface (Oc.S/BS). Bone sections were analyzed using the Osteomeasure software (Osteometrics, USA) following international standards.

To assess HO using the hematoxylin/eosin staining, the calves (HO) and thighs (drop weight) were decalcified, dehydrated and embedded into paraffin. Limbs were cut into 2 µm sections and stained with hematoxylin/eosin. For von Kossa/van Giemson and Safranin O staining, legs were not decalcified, embedded into methacrylate and cut into 4 µm thick sections.

### Immunohistochemistry

For immunohistochemical analysis, paraffin sections from WT and *Tfr2*^{*-*/*-*} bones were dewaxed, rehydrated, and heat-retrieved of antigens. Endogenous peroxidase activity was blocked using 0.3% H₂O₂/PBS for 10 min at room temperature and non-specific binding sites using the blocking buffer of the VECTASTAIN Elite ABC Kit (VECTOR Laboratories) for 45 min at room temperature. Afterwards, sections were incubated with an anti-Tfr2 antibody (H-140, Santa Cruz), a β-catenin antibody (BD Bioscience) or an axin-2 antibody (#ab107613, Abcam) overnight at 4 °C. Subsequently, slides were treated with an anti-mouse secondary antibody conjugated to biotin and then developed utilizing avidin-conjugated HRP with diaminiobenzidine as substrate (DAKO). Slides were examined using a Zeiss Axio Imager M.1 microscope. Two-hundred cells were counted per slide and graded according to no staining (0), weak staining (1), and strong staining (2).

### Measurement of the iron content in the liver and bone

The iron concentration in the liver was determined using 20 mg of dried liver tissue as previously published⁸. The iron concentration in the bone was determined using atomic absorption spectroscopy (PerkinElmer Analyst 800) of dried bone tissue (bone marrow-flushed femur and tibia) as previously published⁶⁹.

### Serum analysis

The bone turnover markers C-terminal telopeptide (CTX) and pro-collagen type I N-terminal peptide (P1NP) were measured in the serum using ELISAs (IDS, Germany). Serum dickkopf-1 and BMP-2 were measured using ELISAs from R&D Systems (Germany). Mouse sclerostin was measured with an ELISA from Alpco (USA). Serum ferritin and iron were measured using routine methods for clinical analyses on a Roche Modular PPE analyzer. The transferrin saturation was determined using a total iron binding capacity kit from Randox.

### Primary osteoclast culture

Osteoclasts were generated from the bone marrow of WT mice and seeded at a density of 1x10⁶ cells/cm². Alpha-MEM (Biochrom, Germany) with 10% FCS, 1% penicillin/streptomycin, and 25 ng/ml M-CSF (all from Life Technologies) was used for the first two days of differentiation. Afterwards, medium was supplemented with 30 ng/ml RANKL (Life Technologies) for the remainder of the culture (5-7 days). RNA was isolated at various time points and mature osteoclasts were used for immunofluorescence analysis.

### Primary osteoblast culture

Primary murine osteoblasts were differentiated from the bone marrow using standard osteogenic medium in DMEM with 10% FCS, 1% penicillin/streptomycin (Life Technologies, Germany). RNA was isolated at various time points and day 7 osteoblasts were used for immunofluorescence analysis of Tfr2 and for the deep sequencing analysis.

*Signaling studies:* day 7 differentiated cells were treated with 50 ng/ml BMP-2, BMP-4 or BMP-6 for 0, 20, and 40 min and lysed in protein lysis buffer (at least two independent experiments with 3 n each). Anisomycin was used to activate MAPK signaling on day 7 differentiated osteoblasts. Cells were treated with 100 nM anisomycin for 20 min for subsequent protein analysis. For RNA isolation and detection of gene expression, cells were treated with different doses of anisomycin for 24 h (two independent experiments with 3 n each).

*Overexpression:* One µg of the pcDNA3.1 vector containing the murine *Tfr2* gene was transfected into 70-80% confluent cells using Fugene HD (Roche)⁸. An empty pcDNA3.1 vector was used as control. In addition, the overexpression vectors pCMV6-MAPK1 (ERK2), pCMV6-MAPK14 (p38α), pCMV6-Smad1, and pCMV6-Smad4 were purchased from Origene to overexpress the respective signaling proteins. The pCMV6-Entry vector was used as control. Each experiment was performed once with cells from 4 different mice.

### RNA isolation, RT and real-time PCR

RNA from cell cultures was isolated with the High Pure RNA Isolation Kit (Roche) and RNA from the bones of mice was isolated by crushing flushed bones (femur and tibia) in liquid nitrogen and collecting the bone powder in Trifast (Peqlab, Germany). Other organs were homogenized directly in Trifast using an ultraturrax (IKA, Germany). Five-hundred ng RNA were reverse transcribed using Superscript II (Invitrogen, Germany) und subsequently used for SYBR green-based realtime PCRs using a standard protocol (Life Technologies). The results were calculated using the ΔΔCT method and are presented in x-fold increase relative to *β-actin* (or *GAPDH* where indicated) mRNA levels.

### Protein isolation and Western blot

Cells were lysed in a buffer containing 20 mM Tris/HCI pH 7.4, 1% SDS, and a protease inhibitor (complete mini, Roche, Germany). To isolate protein from tissues, the protein fraction of the Trifast procedure was used and further processed according to the manufacturer's protocol. The protein concentration was determined using the BCA method (Pierce, Germany). Twenty µg of heatdenatured protein was loaded onto a 10% gel, separated, and transferred onto a 0.2 µm nitrocellulose membrane (Whatman, Germany). After blocking for 1 h with 5% non-fat dry milk or 2% BSA in Tris-buffered saline with 1% Tween-20 (TBS-T), membranes were incubated with primary antibodies to signaling proteins (Cell Signaling) overnight and washed three times with TBS-T. For the detection of Tfr2, the H-140 antibody from Santa Cruz (Germany) was used, which detects an epitope corresponding to amino acids 531-670. Other antibodies used were: lamin A/C (#sc-20681, Santa Cruz), connexin-43 (#3512, Cell Signaling), tubulin (#2146, Cell Signaling), GAPDH (#5G4, Hytest). Thereafter, membranes were incubated with the appropriate HRPconjugated secondary antibodies for 1h at RT. Finally, membranes were washed with TBS-T and incubated with an ECL substrate (Thermo Fisher Scientific). The proteins were visualized using the MF-ChemiBIS 3.2 bioimaging system (Biostep, Germany).

### Subcellular protein fractionation

For separation of cytoplasmic, membrane and nuclear protein extracts of osteoblasts, primary murine osteoblasts were differentiated from the bone marrow of three WT mice. At day 7, cells were harvested and the subcellular protein fractions were isolated using the subcellular protein fractionation kit (Thermo Fisher Scientific) according to manufacturer's recommendation.

### Immunofluorescence staining

For immunofluorescence staining, cells were grown on glass slides. At the desired time point, cells were fixed with 100% methanol for 15 min, permeabilized with 0.5% Triton X-100 for 10 min and after washing for three times, blocked with 1% BSA in PBS for 30 min. Afterwards, cells were incubated with an anti-mouse Tfr2 antibody (H-140, Santa Cruz) over night at 4 °C. After washing, cells were stained with an anti-mouse osterix antibody (sc-393325, Santa Cruz) or phalloidin at RT for 1 h. Subsequently, cells were washed and incubated for 1 h with an Alexa Fluor 488 or Alexa Fluor 594-labelled secondary antibody (Life Technologies), washed, and stained with DAPI for 5 min. After washing again, glass slides were embedded in a small droplet of mounting medium (Dako). Slides were examined using a Zeiss LSM 510 confocal microscope (Zeiss EC PlanNeofluar 40x/1.3 Oil), and photographs were taken and processed with the Zen 2009 software.

### Co-immunoprecipitation

Human hepatoma cells (HuH7) were transfected with 7.5 µg of pCMV-3XFLAG-BMPR-IA and 7.5 µg pcDNA3-TFR2-HA or pcDNA3-LDLR-HA using TransIT^{®}-LT1 Transfection Reagent (Mirus Bio LLC) following the manufacture's protocol. Forty-eight hours after transfection cells were treated with 50 ng/ml of BMP-2 (Peprotech) for 1.5 h, where indicated. Cell lysates were incubated with pre-equilibrated anti-FLAG M2 affinity gel (Sigma Aldrich) at 4°C for 2 h. Samples were then eluted with 50 µl of lysis buffer containing 300 µg/ml 3X FLAG Peptide (Sigma Aldrich). 10% of the total lysate was used as input (In). Immunorecognition was visualized using αFLAG and αHA antibodies (1:1,000, Sigma Aldrich).

### Next generation sequencing and data analysis

Total RNA was isolated from day 7 differentiated cells of *Tfr2*^{*-*/*-*} and WT mice using Trifast. RNA quality was assessed using the Agilent Bioanalyzer and total RNA with an integrity number of ≥ 9 was used. mRNA was isolated from 1 µg total RNA using the NEBNext Poly(A) mRNA Magnetic Isolation Module according to the manufacturer's instructions. After chemical fragmentation, samples were subjected to strand-specific RNA-Seq library preparation (Ultra Directional RNA Library Prep, NEB). After ligation of adaptors (Oligo1 5'-ACA CTC TTT CCC TAC ACG ACG CTC TTC CGA TCT-3', Oligo2: 5'-P-GAT CGG AAG AGC ACA CGT CTG AAC TCC AGT CAC-3') residual oligos were depleted by bead purification (XP, Beckman Coulter). During subsequent PCR enrichment (15 cycles) libraries were indexed (Primer1: Oligo_Seq AAT GAT ACG GCG ACC ACC GAG ATC TAC ACT CTT TCC CTA CAC GAC GCT CTT CCG ATC T, primer2: GTG ACT GGA GTT CAG ACG TGT GCT CTT CCG ATC T, primer3: CAA GCA GAA GAC GGC ATA CGA GAT NNNNNN GTG ACT GGA GTT. After final purification (XP beads) libraries were quantified (Qubit dsDNA HS Assay Kit, Invitrogen), equimolarly pooled and distributed on multiple lanes for 75bp single read sequencing on a Illumina HiSeq 2500 and a Illumina NextSeq 500.

After sequencing, FastQC (http://www.bioinformatics.babraham.ac.uk/) was used for a basic quality control. Reads were then mapped onto the mouse genome (mm10) using GSNAP (version 2014-12-17) together with known splice sites (Ensembl v75) as support. Library diversity was assessed by investigating the redundancy in the mapped reads. A table with counts per gene was obtained by running featureCounts (v1.4.6) on the uniquely mapped reads using Ensembl v75 gene annotations. Normalization of the raw read counts based on the library size and testing for differential expression between the KO and WT was performed with the R package DESeq2 (v1.6.3). Genes with an adjusted (Benjamini-Hochberg) p-value of less than 0.05 were considered as differentially expressed.

Gene ontology analyses were performed with Cytoscape 3.2.1 and the ClueGO plugin. Only significantly (p<0.05) up- or down-regulated genes were fed into the analyses. Gene Set Enrichment Analysis (GSEA) was carried out using the Broad Institute GSEA software "GseaPreRanked" tool (nperm=1000, set_min=5, set_max=500, scoring_scheme=weighted) to analyze a list of 18,106 non-redundant gene symbols ranked by their log2 fold-change of expression between *Tfr2*^{*-*/*-*} and WT conditions. In total 58 gene sets were used for the analysis including 50 Hallmark gene sets, three osteoblast specific gene sets from Park et al. and Zaidi et al., and five other gene sets from Sanjuan-Pla et al.⁷⁰⁻⁷².

### Expression of the Tfr2 extracellular domain

The coding sequence of the full-length extracellular domain (ECD, aa 103-798) of murine Tfr2 was synthesized by Genscript (Germany). Recombinant His-MBP-c3-Tfr2-ECD was expressed in Sf9 insect cells using the baculovirus expression system (pOCC211-Tfr2-ECD). Culture supernatant (5 liters) was harvested, filtered, and loaded on a HisTrap column, after extensive wash with PBS, the Tfr2-ECD protein was eluted using PBS with 250 mM imidazole. The yield in the first protein production was 40 mg and in the second 46 mg Tfr2-ECD. Presence of Tfr2 was analyzed using Coomassie staining of a SDS-PAGE with reducing conditions and Western blot.

Experiments were repeated with a commercially produced Tfr2-ECD from Cusabio. This fragment also contained the entire ECD (aa 103-798) and was dissolved in PBS only.

### Surface plasmon resonance binding and kinetic analysis

Interactions of the Tfr2-ECD and holo-Tf, BMP ligands (BMP-2, -4, -6, -7, R&D Systems) and BMP receptors (BMPR-IA, BMPR-II, R&D Systems) were analyzed using a Biacore T100 instrument (GE Healthcare). Tfr2-ECD, BMP-2 and BMP-4 were immobilized onto Series S Sensor Chips C1 (GE Healthcare) via its amine groups at 25 °C. The carboxyl groups on the chip surface were activated for 7 min with a mixture containing 196 mM 1-ethyl-3-(3dimethylaminopropyl) carbodiimide hydrochloride and 50 mM N-hydroxysuccinimide at a flow rate of 10 µl/min. Next 5 µg/ml of Tfr2-ECD diluted in sodium acetate buffer (pH 4.5) or 2 µg/ml BMP2 or BMP-4 were injected at 5 µl/min flow rate until an immobilization levels of approx. 200 RU in case of Tfr2-ECD or 100 RU in case of BMP-2 and BMP-4 were achieved. Unreacted groups were deactivated via injection of 1 M ethanolamine-HCI, pH 8.5 (7 min, 10 µL/min). A reference surface was created according to the same protocol but omitting the Tfr2 injection.

The binding analysis was performed at 37 °C at a flow rate of 30 µl/min. Each analyte was diluted in running buffer (HBS-P (pH 7.4), 150 mM NaCl, supplemented with 50 nM FeCl₃). In some experiments, 500 mM NaCl were used to reduce potential non-specific binding. BMP ligands were used at the indicated concentrations (0-50 nM); BMP receptors at a concentration of 2-200 nM, holo-Tf at 2.5-100 µM and Tfr2-ECD at 10-5,000 nM. In some experiments, BMP-2/BMPR-IA and BMP-2/holo-Tf were injected at the same time. Concentration-dependent binding of holo-Tf was performed without intermediate regeneration.

For binding analysis, an injection of analyte for 240 s or 300 s over a Tfr2-ECD surface was followed by 1000 s dissociation. The values of the binding levels were recorded from referenced signals 10 s before the end of injection relative to baseline response. They were then emended for the respective molecular weight. After dissociation for 1000 s, the chip surface was regenerated for 60 s with 5 M NaCl, 50 mM NaOH in HBS-P, followed by a 1000 s stabilization time.

Single cycle kinetics with five sequential analyte injections were carried out with a sensitivity enhanced Biacore T200 (GE Healthcare) to determine the Kd value range of Holo-Tf/Tfr2 and the dissociation rates k_{off} (complex stabilities) for Tfr2 binding to BMP-2 and BMP-4 surfaces. The kinetic fitting was performed by global fitting using the 1:1 Langmuir binding model (A + B = AB). Steady state analyses were conducted to determine the affinities (Kd). Therefore, a 1:1 interaction of Tfr2 with BMP-2 or BMP-4 was assumed by fitting the measured binding responses at equilibrium against the concentration. To achieve a robust fit and the typical curvature of the plot, a wide range of Tfr2 concentrations was analyzed (10-5000 nM). Binding and kinetic parameters were evaluated with Biacore^{™} T200 evaluation software 3.1.

### BMP-2 competitive ELISA

The Duo Set BMP-2 ELISA kit from R&D Systems was used. After coating the plate with the BMP2 capture antibody overnight, 1.5 ng/ml BMP-2 was added together with increasing concentrations of the Tfr2-ECD or the BMPR-IA (positive control, R&D Systems). After 1 h incubation at RT and extensive washing, the detection antibody was added according to the manufacturer's protocol and the amount of BMP-2 was quantified. This experiment was performed at least three independent times.

### Statistical analysis

Data are presented as mean ± standard deviation (SD). Graphs and statistics were prepared using the Graphpad Prism 6.0 software. Normality of data was determined using the KolmogorovSmirnov test. In case data were normally distributed, statistical evaluations of two group comparisons were performed using a two-sided Student's t-test. One-way analysis of variance (ANOVA) was used for experiments with more than two groups. Two-way ANOVA with Bonferroni post-hoc tests was used for analyzing genotype and treatment effects. If data were not normally distributed, the Mann-Whitney test and the Wilcoxon signed rank test were used to analyze data. Frequency distributions of micromineralization densities from qBSE-SEM gray scale images were compared using the Kolmogorov-Smirnov test⁶⁷

### References

1 Muckenthaler, M. U., Rivella, S., Hentze, M. W. & Galy, B. A Red Carpet for Iron Metabolism. Cell 168, 344-361, doi:10.1016/j.cell.2016.12.034 (2017).
2 Imel, E. A. et al. Serum fibroblast growth factor 23, serum iron and bone mineral density in premenopausal women. Bone 86, 98-105, doi:10.1016/j.bone.2016.03.005 (2016).
3 Guggenbuhl, P. et al. Bone mineral density in men with genetic hemochromatosis and HFE gene mutation. Osteoporosis international : a journal established as result of cooperation between the European Foundation for Osteoporosis and the National Osteoporosis Foundation of the USA 16, 1809-1814, doi:10.1007/s00198-005-1934-0 (2005).
4 Nemeth, E. et al. Hepcidin regulates cellular iron efflux by binding to ferroportin and inducing its internalization. Science 306, 2090-2093, doi:10.1126/science.1104742 (2004).
5 Powell, L. W., Seckington, R. C. & Deugnier, Y. Haemochromatosis. Lancet 388, 706716, doi:10.1016/S0140-6736(15)01315-X (2016).
6 Camaschella, C. et al. The gene TFR2 is mutated in a new type of haemochromatosis mapping to 7q22. Nature genetics 25, 14-15, doi:10.1038/75534 (2000).
7 Fleming, R. E. et al. Targeted mutagenesis of the murine transferrin receptor-2 gene produces hemochromatosis. Proceedings of the National Academy of Sciences of the United States of America 99, 10653-10658, doi:10.1073/pnas.162360699 (2002).
8 Forejtnikova, H. et al. Transferrin receptor 2 is a component of the erythropoietin receptor complex and is required for efficient erythropoiesis. Blood 116, 5357-5367, doi:10.1182/blood-2010-04-281360 (2010).
9 Wallace, D. F., Summerville, L., Lusby, P. E. & Subramaniam, V. N. First phenotypicdescription of transferrin receptor 2 knockout mouse, and the role of hepcidin. Gut 54, 980-986, doi:10.1136/gut.2004.062018 (2005).
10 Johnson, M. B. & Enns, C. A. Diferric transferrin regulates transferrin receptor 2 protein stability. Blood 104, 4287-4293, doi:10.1182/blood-2004-06-2477 (2004).
11 Poli, M. et al. Transferrin receptor 2 and HFE regulate furin expression via mitogenactivated protein kinase/extracellular signal-regulated kinase (MAPK/Erk) signaling. Implications for transferrin-dependent hepcidin regulation. Haematologica 95, 1832-1840, doi:10.3324/haematol.2010.027003 (2010).
12 D'Alessio, F., Hentze, M. W. & Muckenthaler, M. U. The hemochromatosis proteins HFE, TfR2, and HJV form a membrane-associated protein complex for hepcidin regulation. Journal of hepatology 57, 1052-1060, doi:10.1016/j.jhep.2012.06.015 (2012).
13 Wallace, D. F. et al. Combined deletion of Hfe and transferrin receptor 2 in mice leads to marked dysregulation of hepcidin and iron overload. Hepatology 50, 1992-2000, doi:10.1002/hep.23198 (2009).
14 Hogan, B. L. Bone morphogenetic proteins: multifunctional regulators of vertebrate development. Genes & development 10, 1580-1594 (1996).
15 Andriopoulos, B., Jr. et al. BMP6 is a key endogenous regulator of hepcidin expression and iron metabolism. Nature genetics 41, 482-487, doi:10.1038/ng.335 (2009).
16 Babitt, J. L. et al. Bone morphogenetic protein signaling by hemojuvelin regulates hepcidin expression. Nature genetics 38, 531-539, doi:10.1038/ng1777 (2006).
17 Mayeur, C., Leyton, P. A., Kolodziej, S. A., Yu, B. & Bloch, K. D. BMP type II receptors have redundant roles in the regulation of hepatic hepcidin gene expression and iron metabolism. Blood 124, 2116-2123, doi:10.1182/blood-2014-04-572644 (2014).
18 Steinbicker, A. U. et al. Perturbation of hepcidin expression by BMP type I receptor deletion induces iron overload in mice. Blood 118, 4224-4230, doi: 1 0.1182/blood-2011-03339952 (2011).
19 Wang, R. H. et al. A role of SMAD4 in iron metabolism through the positive regulation of hepcidin expression. Cell metabolism 2, 399-409, doi:10.1016/j.cmet.2005.10.010 (2005).
20 Yu, P. B. et al. Dorsomorphin inhibits BMP signals required for embryogenesis and iron metabolism. Nature chemical biology 4, 33-41, doi:10.1038/nchembio.2007.54 (2008).
21 Shore, E. M. et al. A recurrent mutation in the BMP type I receptor ACVR1 causes inherited and sporadic fibrodysplasia ossificans progressiva. Nature genetics 38, 525-527, doi:10.1038/ng1783 (2006).
22 Wallace, D. F. et al. A critical role for murine transferrin receptor 2 in erythropoiesisduring iron restriction. British journal of haematology 168, 891-901, doi:10.1111/bjh.13225 (2015).
23 Nai, A. et al. The erythroid function of transferrin receptor 2 revealed by Tmprss6 inactivation in different models of transferrin receptor 2 knockout mice. Haematologica 99, 10161021, doi:10.3324/haematol.2013.103143 (2014).
24 Urist, M. R. Bone: formation by autoinduction. Science 150, 893-899 (1965).
25 Roetto, A. et al. Comparison of 3 Tfr2-deficient murine models suggests distinct functions for Tfr2-alpha and Tfr2-beta isoforms in different tissues. Blood 115, 3382-3389, doi:10.1182/blood-2009-09-240960 (2010).
26 Herrmann, T. et al. Iron overload in adult Hfe-deficient mice independent of changes in the steady-state expression of the duodenal iron transporters DMT1 and Ireg1/ferroportin. Journal of molecular medicine 82, 39-48, doi:10.1007/s00109-003-0508-x (2004).
27 Altamura, S. et al. Resistance of ferroportin to hepcidin binding causes exocrine pancreatic failure and fatal iron overload. Cell metabolism 20, 359-367, doi:10.1016/j.cmet.2014.07.007 (2014).
28 Tsay, J. et al. Bone loss caused by iron overload in a murine model: importance of oxidative stress. Blood 116, 2582-2589, doi:10.1182/blood-2009-12-260083 (2010).
29 Rishi, G., Secondes, E. S., Wallace, D. F. & Subramaniam, V. N. Normal systemic iron homeostasis in mice with macrophage-specific deletion of transferrin receptor 2. American journal of physiology. Gastrointestinal and liver physiology 310, G171-180, doi:10.1152/ajpgi.00291.2015 (2016).
30 Kamiya, N. et al. Wnt inhibitors Dkk1 and Sost are downstream targets of BMP signaling through the type IA receptor (BMPRIA) in osteoblasts. Journal of bone and mineral research: the official journal of the American Society for Bone and Mineral Research 25, 200-210, doi:10.1359/jbmr.090806 (2010).
31 Simsek Kiper, P. O. et al. Cortical-Bone Fragility--Insights from sFRP4 Deficiency in Pyle's Disease. The New England journal of medicine 374, 2553-2562, doi:10.1056/NEJMoa1509342 (2016).
32 Wosczyna, M. N., Biswas, A. A., Cogswell, C. A. & Goldhamer, D. J. Multipotent progenitors resident in the skeletal muscle interstitium exhibit robust BMP-dependent osteogenic activity and mediate heterotopic ossification. Journal of bone and mineral research: the official journal of the American Society for Bone and Mineral Research 27, 1004-1017, doi:10.1002/jbmr.1562 (2012).
33 Shimono, K. et al. Potent inhibition of heterotopic ossification by nuclear retinoic acid receptor-gamma agonists. Nature medicine 17, 454-460, doi:10.1038/nm.2334 (2011). 34 Fransen, M. et al. Safety and efficacy of routine postoperative ibuprofen for pain and disability related to ectopic bone formation after hip replacement surgery (HIPAID): randomised controlled trial. Bmj 333, 519, doi:10.1136/bmj.38925.471146.4F (2006).
35 Shen, G. S. et al. Hepcidin1 knockout mice display defects in bone microarchitecture and changes of bone formation markers. Calcified tissue international 94, 632-639, doi:10.1007/s00223-014-9845-8 (2014).
36 Guggenbuhl, P. et al. Bone status in a mouse model of genetic hemochromatosis. Osteoporosis international : a journal established as result of cooperation between the European Foundation for Osteoporosis and the National Osteoporosis Foundation of the USA 22, 2313-2319, doi:10.1007/s00198-010-1456-2 (2011).
37 Doyard, M. et al. Decreased Bone Formation Explains Osteoporosis in a Genetic Mouse Model of Hemochromatosiss. PloS one 11, e0148292, doi:10.1371/journal.pone.0148292 (2016).
38 Calzolari, A. et al. TfR2 localizes in lipid raft domains and is released in exosomes to activate signal transduction along the MAPK pathway. Journal of cell science 119, 4486-4498, doi:10.1242/jcs.03228 (2006).
39 Keller, S., Nickel, J., Zhang, J. L., Sebald, W. & Mueller, T. D. Molecular recognition of BMP-2 and BMP receptor IA. Nature structural & molecular biology 11, 481-488, doi:10.1038/nsmb756 (2004).
40 Yin, H., Yeh, L. C., Hinck, A. P. & Lee, J. C. Characterization of ligand-binding properties of the human BMP type II receptor extracellular domain. Journal of molecular biology 378, 191203, doi:10.1016/j.jmb.2008.02.031 (2008).
41 Canali, S., Wang, C. Y., Zumbrennen-Bullough, K. B., Bayer, A. & Babitt, J. L. Bone morphogenetic protein 2 controls iron homeostasis in mice independent of Bmp6. American journal of hematology, doi:10.1002/ajh.24888 (2017).
42 Koch, P. S. et al. Angiocrine Bmp2 signaling in murine liver controls normal iron homeostasis. Blood 129, 415-419, doi:10.1182/blood-2016-07-729822 (2017).
43 Ramos, E. et al. Evidence for distinct pathways of hepcidin regulation by acute and chronic iron loading in mice. Hepatology 53, 1333-1341, doi:10.1002/hep.24178 (2011). 44 Li, X. et al. Targeted deletion of the sclerostin gene in mice results in increased bone formation and bone strength. Journal of bone and mineral research : the official journal of the American Society for Bone and Mineral Research 23, 860-869, doi:10.1359/jbmr.080216 (2008).
45 MacDonald, B. T. et al. Bone mass is inversely proportional to Dkk1 levels in mice. Bone 41, 331-339, doi:10.1016/j.bone.2007.05.009 (2007).
46 van Bezooijen, R. L. et al. Sclerostin is an osteocyte-expressed negative regulator of bone formation, but not a classical BMP antagonist. The Journal of experimental medicine 199, 805-814, doi:10.1084/jem.20031454 (2004).
47 Kamiya, N. et al. BMP signaling negatively regulates bone mass through sclerostin by inhibiting the canonical Wnt pathway. Development 135, 3801-3811, doi:10.1242/dev.025825 (2008).
48 Yu, C. et al. Advanced oxidation protein products induce apoptosis, and upregulate sclerostin and RANKL expression, in osteocytic MLO-Y4 cells via JNK/p38 MAPK activation. Molecular medicine reports 15, 543-550, doi:10.3892/mmr.2016.6047 (2017).
49 Croons, V., Martinet, W., Herman, A. G., Timmermans, J. P. & De Meyer, G. R. The protein synthesis inhibitor anisomycin induces macrophage apoptosis in rabbit atherosclerotic plaques through p38 mitogen-activated protein kinase. The Journal of pharmacology and experimental therapeutics 329, 856-864, doi:10.1124/jpet.108.149948 (2009).
50 Kamiya, N., Kaartinen, V. M. & Mishina, Y. Loss-of-function of ACVR1 in osteoblasts increases bone mass and activates canonical Wnt signaling through suppression of Wnt inhibitors SOST and DKK1. Biochemical and biophysical research communications 414, 326330, doi:10.1016/j.bbrc.2011.09.060 (2011).
51 Biswas, S. et al. BMPRIA is required for osteogenic differentiation and RANKL expression in adult bone marrow mesenchymal stromal cells. Scientific reports 8, 8475, doi:10.1038/s41598-018-26820-8 (2018).
52 Witcher, P. C. et al. Sclerostin neutralization unleashes the osteoanabolic effects of Dkk1 inhibition. JCI insight 3, doi:10.1172/jci.insight.98673 (2018).
53 Lowery, J. W. et al. Loss of BMPR2 leads to high bone mass due to increased osteoblast activity. Journal of cell science 128, 1308-1315, doi:10.1242/jcs.156737 (2015).
54 Bao, Q. et al. Disruption of bone morphogenetic protein type IA receptor in osteoblasts impairs bone quality and bone strength in mice. Cell and tissue research, doi:10.1007/s00441018-2873-3 (2018).
55 Zhang, Y. et al. Loss of BMP signaling through BMPR1A in osteoblasts leads to greater collagen cross-link maturation and material-level mechanical properties in mouse femoral trabecular compartments. Bone 88, 74-84, doi:10.1016/j.bone.2016.04.022 (2016).
56 Forsberg, J. A. et al. Heterotopic ossification in high-energy wartime extremity injuries: prevalence and risk factors. The Journal of bone and joint surgery. American volume 91, 10841091, doi:10.2106/JBJS.H.00792 (2009).
57 Regis, D., Sandri, A. & Sambugaro, E. Incidence of heterotopic ossification after surface and conventional total hip arthroplasty: a comparative study using anterolateral approach and indomethacin prophylaxis. BioMed research international 2013, 293528, doi:10.1155/2013/293528 (2013).
58 Chakkalakal, S. A. et al. Palovarotene Inhibits Heterotopic Ossification and Maintains Limb Mobility and Growth in Mice With the Human ACVR1(R206H) Fibrodysplasia Ossificans Progressiva (FOP) Mutation. Journal of bone and mineral research : the official journal of the American Society for Bone and Mineral Research 31, 1666-1675, doi:10.1002/jbmr.2820 (2016).
59 Agarwal, S. et al. mTOR inhibition and BMP signaling act synergistically to reduce muscle fibrosis and improve myofiber regeneration. JCI insight 1, e89805, doi:10.1172/jci.insight.89805 (2016).
60 Hino, K. et al. Activin-A enhances mTOR signaling to promote aberrant chondrogenesis in fibrodysplasia ossificans progressiva. The Journal of clinical investigation 127, 3339-3352, doi:10.1172/JCI93521 (2017).
61 Rodda, S. J. & McMahon, A. P. Distinct roles for Hedgehog and canonical Wnt signaling in specification, differentiation and maintenance of osteoblast progenitors. Development 133, 3231-3244, doi:10.1242/dev.02480 (2006).
62 Nakamura, T. et al. Estrogen prevents bone loss via estrogen receptor alpha and induction of Fas ligand in osteoclasts. Cell 130, 811-823, doi:10.1016/j.cell.2007.07.025 (2007). 63
   Clausen, B. E., Burkhardt, C., Reith, W., Renkawitz, R. & Forster, I. Conditional gene targeting in macrophages and granulocytes using LysMcre mice. Transgenic research 8, 265277 (1999).
64 Rhee, Y. et al. PTH receptor signaling in osteocytes governs periosteal bone formation and intracortical remodeling. Journal of bone and mineral research : the official journal of the American Society for Bone and Mineral Research 26, 1035-1046, doi:10.1002/jbmr.304 (2011).
65 Liu, X. et al. A novel mouse model of trauma induced heterotopic ossification. Journal of orthopaedic research : official publication of the Orthopaedic Research Society 32, 183-188, doi:10.1002/jor.22500 (2014).
66 Norden, D. M. et al. Ibuprofen ameliorates fatigue- and depressive-like behavior in tumor-bearing mice. Life sciences 143, 65-70, doi:10.1016/j.lfs.2015.10.020 (2015).
67 Bassett, J. H. et al. Optimal bone strength and mineralization requires the type 2 iodothyronine deiodinase in osteoblasts. Proceedings of the National Academy of Sciences of the United States of America 107, 7604-7609, doi:10.1073/pnas.0911346107 (2010).
68 Rauner, M. et al. Increased EPO Levels Are Associated With Bone Loss in Mice Lacking PHD2 in EPO-Producing Cells. Journal of bone and mineral research : the official journal of the American Society for Bone and Mineral Research 31, 1877-1887, doi:10.1002/jbmr.2857 (2016).
69 Theurl, I. et al. On-demand erythrocyte disposal and iron recycling requires transient macrophages in the liver. Nature medicine 22, 945-951, doi:10.1038/nm.4146 (2016).
70 Sanjuan-Pla, A. et al. Platelet-biased stem cells reside at the apex of the haematopoietic stem-cell hierarchy. Nature 502, 232-236, doi:10.1038/nature12495 (2013).
71 Mootha, V. K. et al. PGC-1alpha-responsive genes involved in oxidative phosphorylation are coordinately downregulated in human diabetes. Nature genetics 34, 267-273, doi:10.1038/ng1180 (2003).
72 Subramanian, A. et al. Gene set enrichment analysis: a knowledge-based approach for interpreting genome-wide expression profiles. Proceedings of the National Academy of Sciences of the United States of America 102, 15545-15550, doi:10.1073/pnas.0506580102 (2005).

### Other Non-patent literature cited

- Shore EM, Kaplan FS (2008) Insights from a rare genetic disorder of extra-skeletal bone formation, fibrodysplasia ossificans progressiva(FOP). Bone 43: 427-433.
- Kölbl O, Barthel T, Krödel A, Seegenschmiedt MH (2003) Prävention von heterotopen Ossifikationen nach Totalendoprothese des Hüftgelenks [Prevention of heterotopic ossification following total replacement of the hip joint]. Deutsches Arzteblatt [German medical journal] 45: 2944-2954. Baschant U, Sastre EA, Roetto A, Platzbecker U, Hofbauer LC, Rauner M (2017) P-OCBR-7: The iron-sensing receptor Tfr2 regulates osteoclastogenesis. Abstracts of the ECTS congress 2017. ECTS 2017. 13-16 May 2017, Salzburg, Austria. 44th European Calcified Tissue Society Congress: Page 43.
- Roetto, A. et al. Comparison of 3 Tfr2-deficient murine models suggests distinct functions for Tfr2-alpha and Tfr2-beta isoforms in different tissues. Blood 115, 33823389, doi:10.1182/blood-2009-09-240960 (2010).
- Wosczyna MW, Biswas AA, Cogswell CA, and Goldhamer DJ (2012) Multipotent Progenitors Resident in the Skeletal Muscle Interstitium Exhibit Robust BMP-Dependent Osteogenic Activity and Mediate Heterotopic Ossification. J Bone Miner Res 27: 1004-1017.

**Table 1: List of reference signs**

| | |
|---|---|
| 1 | BMP |
| 2 | BMP-I |
| 3 | BMP-II |
| 4 | Tfr2α |
| 5 | protein |
| 6 | Smad protein |
| 7 | MAP kinase |
| 8 | phosphorylation |
| 9 | osteoblast genes |
| 10 | bone formation |
| 11 | capture antibody |
| 12 | detection antibody |

**Table 2: Sequences**

| **SEQ ID NO:** | |
|---|---|
| 1 | Human Tfr2-ECD amino acid sequence |
| 2 | Mouse Tfr2-ECD amino acid sequence |
| 3 | Human Tfr2a amino acid sequence |
| 4 | Mouse Tfr2a amino acid sequence |
| 5 | Human Tfr2 protease-associated (PA) domain amino acid sequence |
| 6 | Human peptidase M28 domain amino acid sequence |
| 7 | Human Tfr-like dimerization domain amino acid sequence |
| 8 | Human Tfr2 nucleotide sequence |
| 9 | Mouse Tfr2 nucleotide sequence |
| 10 | Human Tfr2-his amino acid sequence |
| 11 | Human Tfr2-Fc (N-terminal fusion, ie, Fc fused at N-terminal side of Tfr2 ECD) amino acid sequence |
| 12 | Human Tfr2 (C-terminal fusion) amino acid sequence |

### Protein-Sequences

**Human Tfr2a (SEQ ID NO: 3)**
**Human Tfr2b = Tfr2-ECD (SEQ ID NO: 1)** 
RGSCQACGDSVLVVSEDVNYEPDLDFHQGRLYWSDLQAMFLQFLGEGRLEDTIRQTSLRERV
AGSAGMAALTQDIRAALSRQKLDHVWTDTHYVGLQFPDPAHPNTLHWVDEAGKVGEQLPLED
PDVYCPYSAIGNVTGELVYAHYGRPEDLQDLRARGVDPVGRLLLVRVGVISFAQKVTNAQDFG
 
AQGVLIYPEPADFSQDPPKPSLSSQQAVYGHVHLGTGDPYTPGFPSFNQTQFPPVASSGLPSI
PAQPISADIASRLLRKLKGPVAPQEWQGSLLGSPYHLGPGPRLRLVVNNHRTSTPINNIFGCIEG
RSEPDHYVVIGAQRDAWGPGAAKSAVGTAILLELVRTFSSMVSNGFRPRRSLLFISWDGGDFG
SVGSTEWLEGYLSVLHLKAVVYVSLDNAVLGDDKFHAKTSPLLTSLIESVLKQVDSPNHSGQTL
YEQVVFTNPSWDAEVIRPLPMDSSAYSFTAFVGVPAVEFSFMEDDQAYPFLHTKEDTYENLHK
VLQGRLPAVAQAVAQLAGQLLIRLSHDRLLPLDFGRYGDVVLRHIGNLNEFSGDLKARGLTLQ
WVYSARGDYIRAAEKLRQEIYSSEERDERLTRMYNVRIMRVEFYFLSQYVSPADSPFRHIFMG
RGDHTLGALLDHLRLLRSNSSGTPGATSSTGFQESRFRRQLALLTWTLQGAANALSGDVWNID
NNF
**Mouse Tfr2a (SEQ ID NO: 4)** 
MEQRWGLLRRVQQWSPRPSQTIYRRVEGPQLEHLEEEDREEGAELPAQFCPMELKGPEHLG
SCPGRSIPIPWAAAGRKAAPYLVLITLLIFTGAFLLGYVAFRGSCQACGDSVLVVDEDVNPEDSG
RTTLYWSDLQAMFLRFLGEGRMEDTIRLTSLRERVAGSARMATLVQDILDKLSRQKLDHVWTD
THYVGLQFPDPAHANTLHWVDADGSVQEQLPLEDPEVYCPYSATGNATGKLVYAHYGRSEDL
QDLKAKGVELAGSLLLVRVGITSFAQKVAVAQDFGAQGVLIYPDPSDFSQDPHKPGLSSHQAV
YGHVHLGTGDPYTPGFPSFNQTQFPPVESSGLPSIPAQPISADIADQLLRKLTGPVAPQEWKG
HLSGSPYRLGPGPDLRLVVNNHRVSTPISNIFACIEGFAEPDHYVVIGAQRDAWGPGAAKSAV
GTAILLELVRTFSSMVSNGFRPRRSLLFISWDGGDFGSVGATEWLEGYLSVLHLKAVVYVSLDN
SVLGDGKFHAKTSPLLVSLIENILKQVDSPNHSGQTLYEQVALTHPSWDAEVIQPLPMDSSAYS
FTAFAGVPAVEFSFMEDDRVYPFLHTKEDTYENLHKMLRGRLPAVVQAVAQLAGQLLIRLSHD
HLLPLDFGRYGDVVLRHIGNLNEFSGDLKERGLTLQWVYSARGDYIRAAEKLRKEIYSSERNDE
RLMRMYNVRIMRVEFYFLSQYVSPADSPFRHIFLGQGDHTLGALVDHLRMLRADGSGAASSRL
TAGLGFQESRFRRQLALLTWTLQGAANALSGDVWNIDNNF
**Mouse Tfr2-ECD (SEQ ID NO: 2)** 
RGSCQACGDSVLVVDEDVNPEDSGRTTLYWSDLQAMFLRFLGEGRMEDTIRLTSLRERVAGS
ARMATLVQDILDKLSRQKLDHVWTDTHYVGLQFPDPAHANTLHWVDADGSVQEQLPLEDPEV
YCPYSATGNATGKLVYAHYGRSEDLQDLKAKGVELAGSLLLVRVGITSFAQKVAVAQDFGAQG
VLIYPDPSDFSQDPHKPGLSSHQAVYGHVHLGTGDPYTPGFPSFNQTQFPPVESSGLPSIPAQ
PISADIADQLLRKLTGPVAPQEWKGHLSGSPYRLGPGPDLRLVVNNHRVSTPISNIFACIEGFAE
PDHYVVIGAQRDAWGPGAAKSAVGTAILLELVRTFSSMVSNGFRPRRSLLFISWDGGDFGSVG
ATEWLEGYLSVLHLKAVVYVSLDNSVLGDGKFHAKTSPLLVSLIENILKQVDSPNHSGQTLYEQ
 
VALTHPSWDAEVIQPLPMDSSAYSFTAFAGVPAVEFSFMEDDRVYPFLHTKEDTYENLHKMLR
GRLPAVVQAVAQLAGQLLIRLSHDHLLPLDFGRYGDVVLRHIGNLNEFSGDLKERGLTLQWVY
SARGDYIRAAEKLRKEIYSSERNDERLMRMYNVRIMRVEFYFLSQYVSPADSPFRHIFLGQGDH
TLGALVDHLRMLRADGSGAASSRLTAGLGFQESRFRRQLALLTWTLQGAANALSGDVWNIDN
NF
**Human Tfr2 protease-associated (PA) domain amino acid sequence (SEQ ID NO: 5)** 
Tyr Cys Pro Tyr Ser Ala Ile Gly Asn Val Thr Gly Glu Leu Val Tyr
Ala His Tyr Gly Arg Pro Glu Asp Leu Gln Asp Leu Arg Ala Arg Gly
Val Asp Pro Val Gly Arg Leu Leu Leu Val Arg Val Gly Val Ile Ser
Phe Ala Gln Lys Val Thr Asn Ala Gln Asp Phe Gly Ala Gln Gly Val
Leu Ile Tyr Pro Glu Pro Ala Asp Phe Ser Gln Asp Pro Pro Lys Pro
Ser Leu Ser Ser Gln Gln Ala Val Tyr Gly His Val His Leu
**Human peptidase M28 domain amino acid sequence (SEQ ID NO: 6)** 
Gly Arg Ser Glu Pro Asp His Tyr Val Val Ile Gly Ala Gln Arg Asp
Ala Trp Gly Pro Gly Ala Ala Lys Ser Ala Val Gly Thr Ala Ile Leu
Leu Glu Leu Val Arg Thr Phe Ser Ser Met Val Ser Asn Gly Phe Arg
Pro Arg Arg Ser Leu Leu Phe Ile Ser Trp Asp Gly Gly Asp Phe Gly
Ser Val Gly Ser Thr Glu Trp Leu Glu Gly Tyr Leu Ser Val Leu His
Leu Lys Ala Val Val Tyr Val Ser Leu Asp Asn Ala Val Leu Gly Asp
Asp Lys Phe His Ala Lys Thr Ser Pro Leu Leu Thr Ser Leu Ile Glu
Ser Val Leu Lys Gln Val Asp Ser Pro Asn His Ser Gly Gln Thr Leu
Tyr Glu Gln Val Val Phe Thr Asn Pro Ser Trp Asp Ala Glu Val Ile
Arg Pro Leu Pro Met Asp Ser Ser Ala Tyr Ser Phe Thr Ala Phe Val
Gly Val Pro Ala Val Glu Phe Ser Phe Met Glu Asp Asp Gln Ala Tyr
Pro Phe Leu His Thr Lys Glu Asp Thr Tyr
**Human Tfr-like dimerization domain amino acid sequence (SEQ ID NO: 7)** 
Leu Lys Ala Arg Gly Leu Thr Leu Gln Trp Val Tyr Ser Ala Arg Gly
Asp Tyr Ile Arg Ala Ala Glu Lys Leu Arg Gln Glu Ile Tyr Ser Ser
Glu Glu Arg Asp Glu Arg Leu Thr Arg Met Tyr Asn Val Arg Ile Met
Arg Val Glu Phe Tyr Phe Leu Ser Gln Tyr Val Ser Pro Ala Asp Ser
Pro Phe Arg His Ile Phe Met Gly Arg Gly Asp His Thr Leu Gly Ala
 
Leu Leu Asp His Leu Arg Leu Leu Arg Ser Asn Ser Ser Gly Thr Pro
Gly Ala Thr Ser Ser Thr Gly Phe Gln Glu Ser Arg Phe Arg Arg Gln
Leu Ala Leu Leu Thr Trp Thr Leu Gln Gly Ala Ala Asn Ala
**Nukleotid-Sequenzen Human Tfr2 (SEQ ID NO: 8)** 
GTTCGTTGCAACAAATTGATGAGCAATGCTTTTTTATAATGCCAACTTTGTACAAAAAAGTTGGCACCAT
GGAGCGGCTTTGGGGTCTATTCCAGAGAGCGCAACAACTGTCCCCAAGATCCTCTCAGACCGTCTACCAG
CGTGTGGAAGGCCCCCGGAAAGGGCACCTGGAGGAGGAAGAGGAAGACGGGGAGGAGGGGGCGGAGACATTG
GCCCACTTCTGCCCCATGGAGCTGAGGGGCCCTGAGCCCCTGGGCTCTAGACCCAGGCAGCCAAACCT
CATTCCCTGGGCGGCAGCAGGACGGAGGGCTGCCCCCTACCTGGTCCTGACGGCCCTGCTGATCTTCACT
GGGGCCTTCCTACTGGGCTACGTCGCCTTCCGAGGGTCCTGCCAGGCGTGCGGAGACTCTGTGTTGGTGG
TCAGTGAGGATGTCAACTATGAGCCTGACCTGGATTTCCACCAGGGCAGACTCTACTGGAGCGACCTCCA
GGCCATGTTCCTGCAGTTCCTGGGGGAGGGGCGCCTGGAGGACACCATCAGGCAAACCAGCCTTCGGGAA
CGGGTGGCAGGCTCGGCCGGGATGGCCGCTCTGACTCAGGACATTCGCGCGGCGCTCTCCCGCCAGAAGC
TGGACCACGTGTGGACCGACACGCACTACGTGGGGCTGCAATTCCCGGATCCGGCTCACCCCAACACCCT
GCACTGGGTCGATGAGGCCGGGAAGGTCGGAGAGCAGCTGCCGCTGGAGGACCCTGACGTCTACTGCCCC
TACAGCGCCATCGGCAACGTCACGGGAGAGCTGGTGTACGCCCACTACGGGCGGCCCGAAGACCTGCAGG
ACCTGCGGGCCAGGGGCGTGGATCCAGTGGGCCGCCTGCTGCTGGTGCGCGTGGGGGTGATCAGCTTCGC
CCAGAAGGTGACCAATGCTCAGGACTTCGGGGCTCAAGGAGTGCTCATATACCCAGAGCCAGCGGACTTC
TCCCAGGACCCACCCAAGCCAAGCCTGTCCAGCCAGCAGGCAGTGTATGGACATGTGCACCTGGGAACTG
GAGACCCCTACACACCTGGCTTCCCTTCCTTCAATCAAACCCAGTTCCCTCCAGTTGCATCATCAGGCCT
TCCCAGCATCCCAGCCCAGCCCATCAGTGCAGACATTGCCTCCCGCCTGCTGAGGAAGCTCAAAGGCCCT
GTGGCCCCCCAAGAATGGCAGGGGAGCCTCCTAGGCTCCCCTTATCACCTGGGCCCCGGGCCACGACTTC
GGCTAGTGGTCAACAATCACAGGACCTCCACCCCCATCAACAACATCTTCGGCTGCATCGAAGGCCGCTC
AGAGCCAGATCACTACGTTGTCATCGGGGCCCAGAGGGATGCATGGGGCCCAGGAGCAGCTAAATCCGCT
GTGGGGACGGCTATACTCCTGGAGCTGGTGCGGACCTTTTCCTCCATGGTGAGCAACGGCTTCCGGCCCC
GCAGAAGTCTCCTCTTCATCAGCTGGGACGGTGGTGACTTTGGAAGCGTGGGCTCCACGGAGTGGCTAGA
GGGCTACCTCAGCGTGCTGCACCTCAAAGCCGTAGTGTACGTGAGCCTGGACAACGCAGTGCTGGGGGAT
GACAAGTTTCATGCCAAGACCAGCCCCCTTCTGACAAGTCTCATTGAGAGTGTCCTGAAGCAGGTGGATT
CTCCCAACCACAGTGGGCAGACTCTCTATGAACAGGTGGTGTTCACCAATCCCAGCTGGGATGCTGAGGT
GATCCGGCCCCTACCCATGGACAGCAGTGCCTATTCCTTCACGGCCTTTGTGGGAGTCCCTGCCGTCGAG
TTCTCCTTTATGGAGGACGACCAGGCCTACCCATTCCTGCACACAAAGGAGGACACTTATGAGAACCTGC
 
ATAAGGTGCTGCAAGGCCGCCTGCCCGCCGTGGCCCAGGCCGTGGCCCAGCTCGCAGGGCAGCTCCTCAT
CCGGCTCAGCCACGATCGCCTGCTGCCCCTCGACTTCGGCCGCTACGGGGACGTCGTCCTCAGGCACATC
GGGAACCTCAACGAGTTCTCTGGGGACCTCAAGGCCCGCGGGCTGACCCTGCAGTGGGTGTACTCGGCGC
GGGGGGACTACATCCGGGCGGCGGAAAAGCTGCGGCCGGAGATCTACAGCTCGGAGGAGAGACGAGCG
ACTGACACGCATGTACAACGTGCGCATAATGCGGGTGGAGTTCTACTTCCTTTCCCAGTACGTGTCGCCA
GCCGACTCCCCGTTCCGCCACATCTTCATGGGCCGTGGAGACCACACGCTGGGCGCCCTGCTGGACCACC
TGCGGCTGCTGCGCTCCAACAGCTCCGGGACCCCCGGGGCCACCTCCTCCACTGGCTTCCAGGAGAGCCG
TTTCCGGCGTCAGCTAGCCCTGCTCACCTGGACGCTGCAAGGGGCAGCCAATGCGCTTAGCGGGGATGTC
TGGAACATTGATAACAACTTCTTGCCAACTTTCTTGTACAAAGTTGGCATTATAAGAAAGCATTGCTTAT
CAATTTGTTGCAACGAAC
**Mouse Tfr2 (SEQ ID NO: 9)** 
GAGTCTCCTGGGAGCATGGTCCAAGAAACCCAGAGACCTGTTGCTGAGCTGAACTTGGCTGCTGTGTCTT
CCCACTCAGGACTCGGCTTTGACAGGCACGAGGCAGGGACTGGGGTACTGGTGAGCCCCTACCTCTCAGA
TCTTTCTGGACCTGGCTGCAGGTCCTGGTGTCTTCGTCGCGGCTTGGATTTCAAACTGGAGGAGTTCAGG
AGGGGGCACAAGCATGGAGCAACGTTGGGGTCTACTTCGGAGAGTGCAACAGTGGTCCCCAAGACCCTCT
CAGACCATCTACAGACGCGTGGAAGGCCCTCAGCTGGAGCACCTGGAGGAGGAAGACAGGGAGGAAGGGG
CGGAGCTTCCTGCCCAGTTCTGCCCCATGGAACTCAAAGGCCCTGAGCACTTAGGCTCCTGTCCCGGGAG
GTCAATTCCCATACCCTGGGCTGCAGCAGGTCGAAAGGCTGCCCCCTATCTGGTCCTGATCACCCTGCTA
ATCTTCACTGGGGCCTTCCTCCTAGGCTACGTGGCCTTTCGAGGGTCCTGCCAGGCGTGTGGGGACTCCG
TGTTGGTGGTCGATGAAGATGTCAACCCTGAGGACTCCGGCCGGACCACGTTGTACTGGAGCGACCTCCA
GGCCATGTTTCTCCGGTTCCTTGGGGAGGGGCGCATGGAAGACACCATCAGGCTGACCAGCCTCCGGGAA
CGCGTGGCTGGCTCAGCCAGAATGGCCACCCTGGTCCAAGATATCCTCGATAAGCTCTCGCGCCAGAAGC
TGGACCACGTGTGGACTGACACGCACTACGTGGGACTTCAGTTCCCAGATCCGGCTCACGCTAACACCCT
GCACTGGGTGGATGCAGACGGGAGCGTCCAGGAGCAGCTACCGCTGGAGGATCCGGAAGTCTACTGTCCC
TACAGCGCCACCGGCAACGCCACGGGCAAGCTGGTGTACGCCCACTACGGGCGGTCGGAGGACCTACAGG
ACCTAAAAGCCAAGGGCGTGGAGCTGGCCGGCAGCCTCCTGCTAGTGCGAGTTGGAATTACTAGCTTCGC
CCAGAAGGTAGCCGTTGCCCAGGACTTTGGGGCTCAAGGAGTGCTGATATACCCTGACCCATCAGACTTC
TCCCAGGATCCCCACAAGCCAGGCCTGTCTAGCCACCAGGCTGTGTACGGACATGTGCACCTGGGAACTG
GAGACCCTTACACACCTGGCTTCCCGTCCTTCAATCAAACCCAGTTCCCTCCAGTAGAATCATCAGGCCT
 
TCCCAGCATCCCCGCCCAGCCCATCAGTGCTGACATTGCTGATCAATTGCTCAGGAAACTCACAGGCCCC
GTGGCTCCCCAGGAGTGGAAAGGTCACCTCTCAGGCTCTCCTTATCGGCTGGACCTGGCCCGACTTAC
GCCTTGTGGTCAACAACCACAGAGTCTCTACCCCCATCAGTAACATCTTTGCGTGCATCGAGGGCTTTGC
AGAGCCAGATCACTATGTTGTCATTGGGGCCCAGAGGGATGCATGGGGCCCAGGAGCAGCCAAGTCTGCA
GTGGGGACTGCCATCCTGCTGGAGCTGGTTCGGACCTTCTCTTCCATGGTCAGCAATGGGTTCAGACCTC
GAAGAAGTCTTTTGTTCATCAGCTGGGACGGAGGTGACTTTGGCAGCGTGGGAGCCACAGAGTGGTTGGA
GGGCTACCTCAGCGTGCTACACCTCAAAGCTGTTGTGTACGTGAGCCTGGACAACTCCGTGTTGGGAGAT
GGCAAATTCCATGCTAAGACCAGCCCCCTTCTCGTCAGCCTCATTGAGAATATCTTGAAGCAGGTGGACT
CCCCTAACCATAGTGGACAGACCCTCTATGAACAAGTGGCACTCACCCACCCCAGCTGGGATGCTGAAGT
GATTCAGCCCCTGCCCATGGACAGCAGTGCATATTCCTTCACAGCCTTTGCGGGGGTCCCAGCTGTGGAG
TTCTCCTTCATGGAGGATGATCGGGTGTACCCATTCCTGCACACGAAGGAGGACACATATGAGAATCTGC
ACAAGATGCTGCGAGGTCGCCTGCCCGCCGTGGTCCAGGCAGTGGCTCAGCTCGCGGGCCAGCTCCTCAT
CCGACTGAGCCACGATCACCTACTGCCGCTAGACTTCGGCCGCTATGGAGACGTGGTTCTCAGGCACATC
GGCAACCTCAATGAGTTCTCTGGGGACCTCAAGGAGCGCGGGCTGACCCTGCAGTGGGTGTACTCTGCAA
GGGGGGACTACATCCGTGCGGCGGAAAAGCTGCGGAAGGAGATTTACAGCTCGGAGCGGAACGATGAGCG
TCTGATGCGCATGTACAACGTGCGCATCATGAGGGTGGAGTTCTACTTCCTGTCCCAGTATGTGTCGCCA
GCCGACTCCCCATTCCGCCACATTTTCCTAGGCCAAGGCGACCACACTTTGGGTGCCCTGGTAGACCACC
TGCGGATGCTGCGCGCCGATGGCTCAGGAGCCGCCTCTTCCCGGTTGACAGCAGGTCTGGGCTTCCAGGA
GAGTCGCTTCCGGCGCCAGCTGGCGCTGCTCACCTGGACACTGCAGGGGGCAGCCAACGCTCTCAGTGGC
GACGTTTGGAACATTGACAATAACTTTTGAAGCCAAAAGCCCTCCATGGGCCCCACGTGATTCTCCTTTC
TCCCTCTTTGAGTGGTGCAGGCAAAGGAGGTGCCTGAGATTGTAACCTATTCTTAACACCCTTGGTCCTG
CAATGCTGGTGCGCCATATTTTCTCAGTGTGGTTGTCATGCCGTTGCTTACCCAGAAAGCGGTTTTCTTC
CCATCACAGGCCCTTCTGTCTTCAGGAGCAAAGTTCCCCATATCTAGAGACTATCTAGATGCTGGGATCT
GATCAGCTCTCTTAGAGAGTGAGATGGACAGCGTCATTATTTATGACACATGAGCTACGGTATGTGAGC
AGCCCAAGGGGATTAGATGTCAATAAACCAATTGTAACCCCTGTTGTCCATACGCAATTTAGCTTCCTCT
TCATGCCGTACCCACTCCTCATATCCGCCTTGAGACTAGGGAAGAAGGCACAGAAGGCACCTGACAGCAT
GCTTGCAAGATGCTATCCACATGGGAAAAATAACTGTTCTGATGTCTAAGAAAACTGCCTAAAGATAATG
GATAGGAGGTGGGGCAGTGGGGATAACTCATCAGGAATGGGTGATTGCGGCCAAGCCTGATGACCTGAGT
 
TTAATCCCCAGGACCAACATGGCAAAGGAGAGAACTAGTTCCCACAAGTTTTCCTATATCCTCCAAATG
TATGCCCATAAAAGCACAAATAGATAAATGTTTTTGCTTTTGTTTTTTAAAGTGGCTTTGTGGTTAAGAG
CACTGGATGCCATTCTAGAAGACACCGGTTCGATTCCCAGCACCCACATGGCCGCTTACAACTGTCTGTA
ACTCCAGTTGCTGGGGATCAGATGCCCTCTTCTGGTATGGTGTACGACTGCATGCATGTAGTATACATAC
AAGCAGGCAAAATACCCATACATGTAAAATTTTTAAAAAATAGTTTAAATTATAAATTAATTTAAAGAAC
AAATAAAAGATTAATGCCTTTAATCCC
**Human TfR2-his (SEQ ID NO: 10)** 
AFRGSCQACGDSVLVVSEDVNYEPDLDFHQGRLYWSDLQAMFLQFLGEGRLEDTIRQTSLRERVAGSAGMAALTQDI
RAALSRQKLDHVWTDTHYVGLQFPDPAHPNTLHWVDEAGKVGEQLPLEDPDVYCPYSAIGNVTGELVYAHYGRPEDL
QDLRARGVDPVGRLLLVRVGVISFAQKVTNAQDFGAQGVLIYPEPADFSQDPPKPSLSSQQAVYGHVHLGTGDPYTPG
FPSFNQTQFPPVASSGLPSIPAQPISADIASRLLRKLKGPVAPQEWQGSLLGSPYHLGPGPRLRLVVNNHRTSTPINNIFG
CIEGRSEPDHYVVIGAQRDAWGPGAAKSAVGTAILLELVRTFSSMVSNGFRPRRSLLFISWDGGDFGSVGSTEWLEGYL
SVLHLKAVVYVSLDNAVLGDDKFHAKTSPLLTSLIESVLKQVDSPNHSGQTLYEQVVFTNPSWDAEVIRPLPMDSSAYSF
TAFVGVPAVEFSFMEDDQAYPFLHTKEDTYENLHKVLQGRLPAVAQAVAQLAGQLLIRLSHDRLLPLDFGRYGDVVLR
HIGNLNEFSGDLKARGLTLQWVYSARGDYIRAAEKLRQEIYSSEERDERLTRMYNVRIMRVEFYFLSQYVSPADSPFRHIF
MGRGDHTLGALLDHLRLLRSNSSGTPGATSSTGFQESRFRRQLALLTWTLQGAANALSGDVWNIDGGGGSHHHHHH
**Human TfR2-Fc (N-terminal fusion) (SEQ ID NO: 11)** 
EPKSCDKTHTCPPCPAPELLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHYTQKSLSLSPGKIEGRR
GSCQACGDSVLVVSEDVNYEPDLDFHQGRLYWSDLQAMFLQFLGEGRLEDTIRQTSLRERVAGSAGMAALTQDIRAA
LSRQKLDHVWTDTHYVGLQFPDPAHPNTLHWVDEAGKVGEQLPLEDPDVYCPYSAIGNVTGELVYAHYGRPEDLQDL
RARGVDPVGRLLLVRVGVISFAQKVTNAQDFGAQGVLIYPEPADFSQDPPKPSLSSQQAVYGHVHLGTGDPYTPGFPS
FNQTQFPPVASSGLPSIPAQPISADIASRLLRKLKGPVAPQEWQGSLLGSPYHLGPGPRLRLVVNNHRTSTPINNIFGCIE
GRSEPDHYVVIGAQRDAWGPGAAKSAVGTAILLELVRTFSSMVSNGFRPRRSLLFISWDGGDFGSVGSTEWLEGYLSV
LHLKAVVYVSLDNAVLGDDKFHAKTSPLLTSLIESVLKQVDSPNHSGQTLYEQVVFTNPSWDAEVIRPLPMDSSAYSFTA
FVGVPAVEFSFMEDDQAYPFLHTKEDTYENLHKVLQGRLPAVAQAVAQLAGQLLIRLSHDRLLPLDFGRYGDVVLRHIG
 
NLNEFSGDLKARGLTLQWVYSARGDYIRAAEKLRQEIYSSEERDERLTRMYNVRIMRVEFYFLSQYVSPADSPFRHIFMG
RGDHTLGALLDHLRLLRSNSSGTPGATSSTGFQESRFRRQLALLTWTLQGAANALSGDVWNIDNNF
**Human TfR2 (C-terminal fusion) (SEQ ID NO: 12)**

RGSCQACGDSVLVVSEDVNYEPDLDFHQGRLYWSDLQAMFLQFLGEGRLEDTIRQTSLRERVAGSAGMAALTQDIRA
ALSRQKLDHVWTDTHYVGLQFPDPAHPNTLHWVDEAGKVGEQLPLEDPDVYCPYSAIGNVTGELVYAHYGRPEDLQD
LRARGVDPVGRLLLVRVGVISFAQKVTNAQDFGAQGVLIYPEPADFSQDPPKPSLSSQQAVYGHVHLGTGDPYTPGFP
SFNQTQFPPVASSGLPSIPAQPISADIASRLLRKLKGPVAPQEWQGSLLGSPYHLGPGPRLRLVVNNHRTSTPINNIFGCI
EGRSEPDHYVVIGAQRDAWGPGAAKSAVGTAILLELVRTFSSMVSNGFRPRRSLLFISWDGGDFGSVGSTEWLEGYLS
VLHLKAVVYVSLDNAVLGDDKFHAKTSPLLTSLIESVLKQVDSPNHSGQTLYEQVVFTNPSWDAEVIRPLPMDSSAYSFT
AFVGVPAVEFSFMEDDQAYPFLHTKEDTYENLHKVLQGRLPAVAQAVAQLAGQLLIRLSHDRLLPLDFGRYGDVVLRHI
GNLNEFSGDLKARGLTLQWVYSARGDYIRAAEKLRQEIYSSEERDERLTRMYNVRIMRVEFYFLSQYVSPADSPFRHIFM
GRGDHTLGALLDHLRLLRSNSSGTPGATSSTGFQESRFRRQLALLTWTLQGAANALSGDVWNIDNNFIEGREPKSCDKT
HTCPPCPAPELLGGPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPGK

**Table 3: Primer Sequences (Example 3)**

| **SEQ ID NO.** | **Gene** | **Primer sequences** |
|---|---|---|
| SEQ ID NO. 13 | β-actin | s: ATCTGGCACCACACCTTCT |
| SEQ ID NO. 14 | | as: GGGGTGTTGAAGGTCTCAAA |
| SEQ ID NO. 15 | Axin2 | s: GCAGTGATGGAGGAAAATGC |
| SEQ ID NO. 16 | | as: ATTCAAGGTGGGGAGGTAGC |
| SEQ ID NO. 17 | Bmp2 | s: CGCTCCACAAACGAGAAAAG |
| SEQ ID NO. 18 | | as: CAGTCATTCCACCCCACATC |
| SEQ ID NO. 19 | Bmp4 | s: CTCAAGGGAGTGGAGATTGG |
| SEQ ID NO. 20 | | as: CTTCTGCGGGTCAAGGTATG |
| SEQ ID NO. 21 | Bmp6 | s: TAGCAATCTGTGGGTGGTGA |
| SEQ ID NO. 22 | | as: GAAGGGCTGCTTGTCGTAAG |
| SEQ ID NO. 23 | Cd44 | s: TCCTTCGATGGACCGGTTACC |
| SEQ ID NO. 24 | | as: GTGGAGCCGCTGCTGACATC |
| SEQ ID NO. 25 | Dkk1 | s: GAGGGGAAATTGAGGAAAGC |
| SEQ ID NO. 26 | | as: AGCCTTCTTGTCCTTTGGTG |
| SEQ ID NO. 27 | Dmp1 | s: TGTCATTCTCCTTGTGTTCCTTTG |
| SEQ ID NO. 28 | | as: AGAGCTTTCAGATTCAGTATTGTCGTAT |
| SEQ ID NO. 29 | Id1 | s: CCCACTGGACCGATCCGCCA |
| SEQ ID NO. 30 | | as: TGCTCTCGGTTCCCCAGGGG |
| SEQ ID NO. 31 | Id2 | s: TCTGGGGGATGCTGGGCACC |
| SEQ ID NO. 32 | | as: GCTTGGGCATCTCCCGGAGC |
| SEQ ID NO. 33 | Lef1 | s: CAAATAAAGTGCCCGTGGTG |
| SEQ ID NO. 34 | | as: TCGTCGCTGTAGGTGATGAG |
| SEQ ID NO. 35 | Phex | s: GGAAGAAAACCATTGCCAATTATT |
| SEQ ID NO. 36 | | as: CGCCTGCTGAGGTTTGGA |
| SEQ ID NO. 37 | Sfrp | s: AAGTCAGGGTGATTGGGGGAATCC |
| SEQ ID NO. 38 | | as: AAACCATCTCCTCGGATAGGGCAC |
| SEQ ID NO. 39 | Smad6 | s: ATTCTCGGCTGTCTCCTCCT |
| SEQ ID NO. 40 | | as: CCCTGAGGTAGGTCGTAGAA |
| SEQ ID NO. 41 | Sost | s: CGTGCCTCATCTGCCTACTT |
| SEQ ID NO. 42 | | as: TGACCTCTGTGGCATCATTC |
| SEQ ID NO. 43 | Tcf7 | s: GGACATCAGCCAGAAGCAAG |
| SEQ ID NO. 44 | | as: GGACAGGGGGTAGAGAGGAG |
| SEQ ID NO. 45 | α-Tfr2 | s: GCCATGTTTCTCCCGGTTCCT |
| SEQ ID NO. 46 | | as: TGGCGCGAGAGCTTATCG |
| SEQ ID NO. 47 | β-Tfr2 | s: CCTGGCCCCTAGTGTGATTTC |
| SEQ ID NO. 48 | | as: TGGCGCGAGAGCTTATCG |
| SEQ ID NO. 49 | Tgf-β | s: GACCTCCATAGAAGACACC |
| SEQ ID NO. 50 | | as: AACCCGTTGATGTCCACTTGC |

**Table 4: Constant Regions for Antibodies, Fragments, Inhibitors & Proteins of the Invention**

| | | | | |
|---|---|---|---|---|
| SEQ ID NO. 51 | Human IgG1 constant region | IGHG1*01 | Human Heavy Chain Constant Region (IGHG1*01) Nucleotide Sequence | gcctccaccaagggcccatcggtcttccccctggcaccctcctccaagagcacctctgggggcacagcggccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacccagacctacatctgcaacgtgaatcacaagcccagcaacaccaaggtggacaagaaagtt gagcccaaatcttgtgacaaaactcacacatgcccaccgtgcccagcacctgaactcctggggggaccgtcag tcttcctcttccccccaaaacccaaggacaccctcatgatctcccggacccctgaggtcacatgcgtggtggtgg acgtgagccacgaagaccctgaggtcaagttcaactggtacgtggacggcgtggaggtgcataatgccaaga caaagccgcgggaggagcagtacaacagcacgtaccgggtggtcagcgtcctcaccgtcctgcaccaggact ggctgaatggcaaggagtacaagtgcaaggtctccaacaaagccctcccagcccccatcgagaaaaccatct ccaaagccaaagggcagccccgagaaccacaggtgtacaccctgcccccatcccgggatgagctgaccaaga accaggtcagcctgacctgcctggtcaaaggcttctatcccagcgacatcgccgtggagtgggagagcaatgg gcagccggagaacaactacaagaccacgcctcccgtgctggactccgacggctccttcttcctctacagcaagc tcaccgtggacaagagcaggtggcagcaggggaacgtcttctcatgctccgtgatgcatgaggctctgcacaa ccactacacgcagaagagcctctccctgtctccgggtaaa |
| SEQ ID NO. 52 | | | Human Heavy Chain Constant Region (IGHG1*01) Protein Sequence (P01857) | |
| SEQ ID NO. 53 | Human IgG1 constant region | IGHG1*02 or IGHG1*05 | Human Heavy Chain Constant Region (IGHG1*02 or IGHG1*05) Nucleotide Sequence | gcctccaccaagggcccatcggtcttccccctggcaccctcctccaagagcacctctgggggcacagcggccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacccagacctacatctgcaacgtgaatcacaagcccagcaacaccaaggtggacaagaaagtt gagcccaaatcttgtgacaaaactcacacatgcccaccgtgcccagcacctgaactcctggggggaccgtcag tcttcctcttccccccaaaacccaaggacaccctcatgatctcccggacccctgaggtcacatgcgtggtggtgg acgtgagccacgaagaccctgaggtcaagttcaactggtacgtggacggcgtggaggtgcataatgccaaga caaagccgcgggaggagcagtacaacagcacgtaccgtgtggtcagcgtcctcaccgtcctgcaccaggactg gctgaatggcaaggagtacaagtgcaaggtctccaacaaagccctcccagcccccatcgagaaaaccatctcc aaagccaaagggcagccccgagaaccacaggtgtacaccctgcccccatcccgggatgagctgaccaagaa ccaggtcagcctgacctgcctggtcaaaggcttctatcccagcgacatcgccgtggagtgggagagcaatggg cagccggagaacaactacaagaccacgcctcccgtgctggactccgacggctccttcttcctctacagcaagct caccgtggacaagagcaggtggcagcaggggaacgtcttctcatgctccgtgatgcatgaggctctgcacaac cactacacgcagaagagcctctccctgtctccgggtaaa |
| SEQ ID NO. 54 | | | Human Heavy Chain Constant Region (IGHG1*02) Protein Sequence | |
| SEQ ID NO. 55 | Human IgG1 constant region | IGHG1*03 | Human Heavy Chain Constant Region (IGHG1*03) Nucleotide Sequence (Y14737) | gcctccaccaagggcccatcggtcttccccctggcaccctcctccaagagcacctctgggggcacagcggccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacccagacctacatctgcaacgtgaatcacaagcccagcaacaccaaggtggacaagagagtt gagcccaaatcttgtgacaaaactcacacatgcccaccgtgcccagcacctgaactcctggggggaccgtcag tcttcctcttccccccaaaacccaaggacaccctcatgatctcccggacccctgaggtcacatgcgtggtggtgg acgtgagccacgaagaccctgaggtcaagttcaactggtacgtggacggcgtggaggtgcataatgccaaga caaagccgcgggaggagcagtacaacagcacgtaccgtgtggtcagcgtcctcaccgtcctgcaccaggactg gctgaatggcaaggagtacaagtgcaaggtctccaacaaagccctcccagcccccatcgagaaaaccatctcc aaagccaaagggcagccccgagaaccacaggtgtacaccctgcccccatcccgggaggagatgaccaagaa ccaggtcagcctgacctgcctggtcaaaggcttctatcccagcgacatcgccgtggagtgggagagcaatggg cagccggagaacaactacaagaccacgcctcccgtgctggactccgacggctccttcttcctctatagcaagct caccgtggacaagagcaggtggcagcaggggaacgtcttctcatgctccgtgatgcatgaggctctgcacaac cactacacgcagaagagcctctccctgtccccgggtaaa |
| SEQ ID NO. 56 | | | Human Heavy Chain Constant Region (IGHG1*03) Protein Sequence | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVV VDVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTK NQVSLTCLVKGFYPSDIAVEWESNGQPENNYKTTPPVLDSDGSFFLYS KLTVDKSRWQQGNVFSCSVMHEALHNHYTQKSLSLSPGK |
| SEQ ID NO. 57 | Human IgG1 constant region | IGHG1 *04 | Human Heavy Chain Constant Region (IGHG1*04) Nucleotide Sequence | gcctccaccaagggcccatcggtcttccccctggcaccctcctccaagagcacctctgggggcacagcggccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacccagacctacatctgcaacgtgaatcacaagcccagcaacaccaaggtggacaagaaagtt gagcccaaatcttgtgacaaaactcacacatgcccaccgtgcccagcacctgaactcctggggggaccgtcag tcttcctcttccccccaaaacccaaggacaccctcatgatctcccggacccctgaggtcacatgcgtggtggtgg acgtgagccacgaagaccctgaggtcaagttcaactggtacgtggacggcgtggaggtgcataatgccaaga caaagccgcgggaggagcagtacaacagcacgtaccgtgtggtcagcgtcctcaccgtcctgcaccaggactg gctgaatggcaaggagtacaagtgcaaggtctccaacaaagccctcccagcccccatcgagaaaaccatctcc aaagccaaagggcagccccgagaaccacaggtgtacaccctgcccccatcccgggatgagctgaccaagaa ccaggtcagcctgacctgcctggtcaaaggcttctatcccagcgacatcgccgtggagtgggagagcaatggg cagccggagaacaactacaagaccacgcctcccgtgctggactccgacggctccttcttcctctacagcaagct caccgtggacaagagcaggtggcagcaggggaacatcttctcatgctccgtgatgcatgaggctctgcacaac cactacacgcagaagagcctctccctgtctccgggtaaa |
| SEQ ID NO. 58 | | | Human Heavy Chain Constant Region (IGHG1*04) Protein Sequence | ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNIFSCSVMHEALHNHYTQKSLSLSPGK |
| SEQ ID NO. 59 | Disabled Human IgG1 heavy chain constant region | Disabled human IGHG1 *01 | Disabled Human IGHG1*01 Heavy Chain Constant Region Nucleotide Sequence. | Gcctccaccaagggcccatcggtcttccccctggcaccctcctccaagagcacctctgggggcacagcggccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacccagacctacatctgcaacgtgaatcacaagcccagcaacaccaaggtggacaagaaagtg gagcccaaatcttgtgacaaaactcacacatgcccaccgtgcccagcacctgaactcgcgggggcaccgtcag tcttcctcttccccccaaaacccaaggacaccctcatgatctcccggacccctgaggtcacatgcgtggtggtgg acgtgagccacgaagaccctgaggtcaagttcaactggtacgtggacggcgtggaggtgcataatgccaaga caaagccgcgggaggagcagtacaacagcacgtaccgtgtggtcagcgtcctcaccgtcctgcaccaggactg gctgaatggcaaggagtacaagtgcaaggtctccaacaaagccctcccagcccccatcgagaaaaccatctcc aaagccaaagggcagccccgagaaccacaggtgtacaccctgcccccatcccgggatgagctgaccaagaa ccaggtcagcctgacctgcctggtcaaaggcttctatcccagcgacatcgccgtggagtgggagagcaatggg cagccggagaacaactacaagaccacgcctcccgtgctggactccgacggctccttcttcctctacagcaagct caccgtggacaagagcaggtggcagcaggggaacgtcttctcatgctccgtgatgcatgaggctctgcacaac cactacacgcagaagagcctctccctgtctccgggtaaa |
| SEQ ID NO. 60 | | | Disabled Human IGHG1*01 Heavy Chain Constant Region Amino Acid Sequence. Two residues that differ from the wild-type sequence are identified in bold. | ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELAGA PSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| SEQ ID NO. 61 | Human IgG2 constant region | IGHG2*01 or IGHG2*04 or IGHG2*05 | Human Heavy Chain Constant Region (IGHG2*01 or IGHG2*03 or IGHG2*05) Nucleotide Sequence | gcctccaccaagggcccatcggtcttccccctggcgccctgctccaggagcacctccgagagcacagccgccct gggctgcctggtcaaggactacttccccgaaccggtgacggtgtcgtggaactcaggcgctctgaccagcggc gtgcacaccttcccagctgtcctacagtcctcaggactctactccctcagcagcgtggtgaccgtgccctccagc aacttcggcacccagacctacacctgcaacgtagatcacaagcccagcaacaccaaggtggacaagacagtt gagcgcaaatgttgtgtcgagtgcccaccgtgcccagcaccacctgtggcaggaccgtcagtcttcctcttcccc ccaaaacccaaggacaccctcatgatctcccggacccctgaggtcacgtgcgtggtggtggacgtgagccacg aagaccccgaggtccagttcaactggtacgtggacggcgtggaggtgcataatgccaagacaaagccacggg aggagcagttcaacagcacgttccgtgtggtcagcgtcctcaccgttgtgcaccaggactggctgaacggcaa ggagtacaagtgcaaggtctccaacaaaggcctcccagcccccatcgagaaaaccatctccaaaaccaaagg gcagccccgagaaccacaggtgtacaccctgcccccatcccgggaggagatgaccaagaaccaggtcagcct gacctgcctggtcaaaggcttctaccccagcgacatcgccgtggagtgggagagcaatgggcagccggagaa caactacaagaccacacctcccatgctggactccgacggctccttcttcctctacagcaagctcaccgtggaca agagcaggtggcagcaggggaacgtcttctcatgctccgtgatgcatgaggctctgcacaaccactacacgca gaagagcctctccctgtctccgggtaaa |
| SEQ ID NO. 62 | | | Human Heavy Chain Constant Region (IGHG2*01) Protein Sequence | ASTKGPSVFPLAPCSRSTSESTAALGCLVKDYFPEPVTVSWNSGALTSGVHTFPAVLQSS GLYSLSSVVTVPSSNFGTQTYTCNVDHKPSNTKVDKTVERKCCVECPPCPAPPVAGPSVF LFPPKPKDTLMISRTPEVTCVVVDVSHEDPEVQFNWYVDGVEVHNAKTKPREEQFNST FRVVSVLTVVHQDWLNGKEYKCKVSNKGLPAPIEKTISKTKGQPREPQVYTLPPSREEMT KNQVSLTCLVKGFYPSDIAVEWESNGQPENNYKTTPPMLDSDGSFFLYSKLTVDKSRW QQGNVFSCSVMHEALHNHYTQKSLSLSPGK |
| SEQ ID NO. 63 | Human IgG2 constant region | IGHG2*02 | Human Heavy Chain Constant Region (IGHG2*02) Nucleotide Sequence | GCCTCCACCAAGGGCCCATCGGTCTTCCCCCTGGCGCCCTGCTCCAGGAGCACCTCC GAGAGCACAGCGGCCCTGGGCTGCCTGGTCAAGGACTACTTCCCCGAACCGGTGAC GGTGTCGTGGAACTCAGGCGCTCTGACCAGCGGCGTGCACACCTTCCCGGCTGTCCT ACAGTCCTCAGGACTCTACTCCCTCAGCAGCGTGGTGACCGTGACCTCCAGCAACTT CGGCACCCAGACCTACACCTGCAACGTAGATCACAAGCCCAGCAACACCAAGGTGG ACAAGACAGTTGAGCGCAAATGTTGTGTCGAGTGCCCACCGTGCCCAGCACCACCT GTGGCAGGACCGTCAGTCTTCCTCTTCCCCCCAAAACCCAAGGACACCCTCATGATCT CCCGGACCCCTGAGGTCACGTGCGTGGTGGTGGACGTGAGCCACGAAGACCCCGA GGTCCAGTTCAACTGGTACGTGGACGGCATGGAGGTGCATAATGCCAAGACAAAGC CACGGGAGGAGCAGTTCAACAGCACGTTCCGTGTGGTCAGCGTCCTCACCGTCGTG CACCAGGACTGGCTGAACGGCAAGGAGTACAAGTGCAAGGTCTCCAACAAAGGCCT CCCAGCCCCCATCGAGAAAACCATCTCCAAAACCAAAGGGCAGCCCCGAGAACCAC AGGTGTACACCCTGCCCCCATCCCGGGAGGAGATGACCAAGAACCAGGTCAGCCTG ACCTGCCTGGTCAAAGGCTTCTACCCCAGCGACATCGCCGTGGAGTGGGAGAGCAA TGGGCAGCCGGAGAACAACTACAAGACCACACCTCCCATGCTGGACTCCGACGGCT CCTTCTTCCTCTACAGCAAGCTCACCGTGGACAAGAGCAGGTGGCAGCAGGGGAAC |
| | | | | GTCTTCTCATGCTCCGTGATGCATGAGGCTCTGCACAACCACTACACACAGAAGAGC CTCTCCCTGTCTCCGGGTAAA |
| SEQ ID NO. 64 | | | Human Heavy Chain Constant Region (IGHG2*02) Protein Sequence | ASTKGPSVFPLAPCSRSTSESTAALGCLVKDYFPEPVTVSWNSGALTSGVHTFPAVLQSS GLYSLSSVVTVTSSNFGTQTYTCNVDHKPSNTKVDKTVERKCCVECPPCPAPPVAGPSVF LFPPKPKDTLMISRTPEVTCVVVDVSHEDPEVQFNWYVDGMEVHNAKTKPREEQFNST FRVVSVLTVVHQDWLNGKEYKCKVSNKGLPAPIEKTISKTKGQPREPQVYTLPPSREEMT KNQVSLTCLVKGFYPSDIAVEWESNGQPENNYKTTPPMLDSDGSFFLYSKLTVDKSRW QQGNVFSCSVMHEALHNHYTQKSLSLSPGK |
| SEQ ID NO. 65 | Human IgG2 constant region | IGHG2*04 | Human Heavy Chain Constant Region (IGHG2*04) Nucleotide Sequence | gcctccaccaagggcccatcggtcttccccctggcgccctgctccaggagcacctccgagagcacagcggccct gggctgcctggtcaaggactacttccccgaaccggtgacggtgtcgtggaactcaggcgctctgaccagcggc gtgcacaccttcccagctgtcctacagtcctcaggactctactccctcagcagcgtggtgaccgtgccctccagc agcttgggcacccagacctacacctgcaacgtagatcacaagcccagcaacaccaaggtggacaagacagtt gagcgcaaatgttgtgtcgagtgcccaccgtgcccagcaccacctgtggcaggaccgtcagtcttcctcttcccc ccaaaacccaaggacaccctcatgatctcccggacccctgaggtcacgtgcgtggtggtggacgtgagccacg aagaccccgaggtccagttcaactggtacgtggacggcgtggaggtgcataatgccaagacaaagccacggg aggagcagttcaacagcacgttccgtgtggtcagcgtcctcaccgttgtgcaccaggactggctgaacggcaa ggagtacaagtgcaaggtctccaacaaaggcctcccagcccccatcgagaaaaccatctccaaaaccaaagg gcagccccgagaaccacaggtgtacaccctgcccccatcccgggaggagatgaccaagaaccaggtcagcct gacctgcctggtcaaaggcttctaccccagcgacatcgccgtggagtgggagagcaatgggcagccggagaa |
| | | | | caactacaagaccacacctcccatgctggactccgacggctccttcttcctctacagcaagctcaccgtggaca agagcaggtggcagcaggggaacgtcttctcatgctccgtgatgcatgaggctctgcacaaccactacacgca gaagagcctctccctgtctccgggtaaa |
| SEQ ID NO. 66 | | | Human Heavy Chain Constant Region (IGHG2*04) Protein Sequence | ASTKGPSVFPLAPCSRSTSESTAALGCLVKDYFPEPVTVSWNSGALTSGVHTFPAVLQSS GLYSLSSVVTVPSSSLGTQTYTCNVDHKPSNTKVDKTVERKCCVECPPCPAPPVAGPSVF LFPPKPKDTLMISRTPEVTCVVVDVSHEDPEVQFNWYVDGVEVHNAKTKPREEQFNST FRVVSVLTVVHQDWLNGKEYKCKVSNKGLPAPIEKTISKTKGQPREPQVYTLPPSREEMT KNQVSLTCLVKGFYPSDIAVEWESNGQPENNYKTTPPMLDSDGSFFLYSKLTVDKSRW QQGNVFSCSVMHEALHNHYTQKSLSLSPGK |
| SEQ ID NO. 67 | Human IgG2 constant region | IGHG2*06 | Human Heavy Chain Constant Region (IGHG2*06) Nucleotide Sequence | GCCTCCACCAAGGGCCCATCGGTCTTCCCCCTGGCGCCCTGCTCCAGGAGCACCTCC GAGAGCACAGCGGCCCTGGGCTGCCTGGTCAAGGACTACTTCCCCGAACCGGTGAC GGTGTCGTGGAACTCAGGCGCTCTGACCAGCGGCGTGCACACCTTCCCGGCTGTCCT ACAGTCCTCAGGACTCTACTCCCTCAGCAGCGTGGTGACCGTGCCCTCCAGCAACTT CGGCACCCAGACCTACACCTGCAACGTAGATCACAAGCCCAGCAACACCAAGGTGG ACAAGACAGTTGAGCGCAAATGTTGTGTCGAGTGCCCACCGTGCCCAGCACCACCT GTGGCAGGACCGTCAGTCTTCCTCTTCCCCCCAAAACCCAAGGACACCCTCATGATCT CCCGGACCCCTGAGGTCACGTGCGTGGTGGTGGACGTGAGCCACGAAGACCCCGA GGTCCAGTTCAACTGGTACGTGGACGGCGTGGAGGTGCATAATGCCAAGACAAAGC CACGGGAGGAGCAGTTCAACAGCACGTTCCGTGTGGTCAGCGTCCTCACCGTCGTG CACCAGGACTGGCTGAACGGCAAGGAGTACAAGTGCAAGGTCTCCAACAAAGGCCT |
| | | | | CCCAGCCCCCATCGAGAAAACCATCTCCAAAACCAAAGGGCAGCCCCGAGAACCAC AGGTGTACACCCTGCCCCCATCCCGGGAGGAGATGACCAAGAACCAGGTCAGCCTG ACCTGCCTGGTCAAAGGCTTCTACCCCAGCGACATCTCCGTGGAGTGGGAGAGCAA TGGGCAGCCGGAGAACAACTACAAGACCACACCTCCCATGCTGGACTCCGACGGCT CCTTCTTCCTCTACAGCAAGCTCACCGTGGACAAGAGCAGGTGGCAGCAGGGGAAC GTCTTCTCATGCTCCGTGATGCATGAGGCTCTGCACAACCACTACACACAGAAGAGC CTCTCCCTGTCTCCGGGTAAA |
| SEQ ID NO. 68 | | | Human Heavy Chain Constant Region (IGHG2*06) Protein Sequence | ASTKGPSVFPLAPCSRSTSESTAALGCLVKDYFPEPVTVSWNSGALTSGVHTFPAVLQSS GLYSLSSVVTVPSSNFGTQTYTCNVDHKPSNTKVDKTVERKCCVECPPCPAPPVAGPSVF LFPPKPKDTLMISRTPEVTCVVVDVSHEDPEVQFNWYVDGVEVHNAKTKPREEQFNST FRVVSVLTVVHQDWLNGKEYKCKVSNKGLPAPIEKTISKTKGQPREPQVYTLPPSREEMT KNQVSLTCLVKGFYPSDISVEWESNGQPENNYKTTPPMLDSDGSFFLYSKLTVDKSRWQ QGNVFSCSVMHEALHNHYTQKSLSLSPGK |
| SEQ ID NO. 69 | Human IgG4 constant region | IGHG4*01 or IGHG4*04 | Human Heavy Chain Constant Region (IGHG4*01 or IGHG4*04) Nucleotide Sequence | gcttccaccaagggcccatccgtcttccccctggcgccctgctccaggagcacctccgagagcacagccgccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacgaagacctacacctgcaacgtagatcacaagcccagcaacaccaaggtggacaagagagtt gagtccaaatatggtcccccatgcccatcatgcccagcacctgagttcctggggggaccatcagtcttcctgttc cccccaaaacccaaggacactctcatgatctcccggacccctgaggtcacgtgcgtggtggtggacgtgagcc aggaagaccccgaggtccagttcaactggtacgtggatggcgtggaggtgcataatgccaagacaaagccgc gggaggagcagttcaacagcacgtaccgtgtggtcagcgtcctcaccgtcctgcaccaggactggctgaacgg caaggagtacaagtgcaaggtctccaacaaaggcctcccgtcctccatcgagaaaaccatctccaaagccaa agggcagccccgagagccacaggtgtacaccctgcccccatcccaggaggagatgaccaagaaccaggtca |
| | | | | gcctgacctgcctggtcaaaggcttctaccccagcgacatcgccgtggagtgggagagcaatgggcagccgga gaacaactacaagaccacgcctcccgtgctggactccgacggctccttcttcctctacagcaggctaaccgtgg acaagagcaggtggcaggaggggaatgtcttctcatgctccgtgatgcatgaggctctgcacaaccactacac acagaagagcctctccctgtctctgggtaaa |
| SEQ ID NO. 70 | | | Human Heavy Chain Constant Region (IGHG4*01) Protein Sequence (P01861) | ASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPSCPAPEFLGGPSVF LFPPKPKDTLMISRTPEVTCVVVDVSQEDPEVQFNWYVDGVEVHNAKTKPREEQFNST YRVVSVLTVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTVDKSRWQ EGNVFSCSVMHEALHNHYTQKSLSLSLGK |
| SEQ ID NO. 71 | Human IgG4 constant region | IGHG4*02 | Human Heavy Chain Constant Region (IGHG4*02) Nucleotide Sequence | gcttccaccaagggcccatccgtcttccccctggcgccctgctccaggagcacctccgagagcacagccgccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacgaagacctacacctgcaacgtagatcacaagcccagcaacaccaaggtggacaagagagtt gagtccaaatatggtcccccgtgcccatcatgcccagcacctgagttcctggggggaccatcagtcttcctgttc cccccaaaacccaaggacactctcatgatctcccggacccctgaggtcacgtgcgtggtggtggacgtgagcc aggaagaccccgaggtccagttcaactggtacgtggatggcgtggaggtgcataatgccaagacaaagccgc gggaggagcagttcaacagcacgtaccgtgtggtcagcgtcctcaccgtcgtgcaccaggactggctgaacgg caaggagtacaagtgcaaggtctccaacaaaggcctcccgtcctccatcgagaaaaccatctccaaagccaa agggcagccccgagagccacaggtgtacaccctgcccccatcccaggaggagatgaccaagaaccaggtca |
| | | | | gcctgacctgcctggtcaaaggcttctaccccagcgacatcgccgtggagtgggagagcaatgggcagccgga gaacaactacaagaccacgcctcccgtgctggactccgacggctccttcttcctctacagcaggctaaccgtgg acaagagcaggtggcaggaggggaatgtcttctcatgctccgtgatgcatgaggctctgcacaaccactacac gcagaagagcctctccctgtctctgggtaaa |
| SEQ ID NO. 72 | | | Human Heavy Chain Constant Region (IGHG4*02) Protein Sequence | ASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPSCPAPEFLGGPSVF LFPPKPKDTLMISRTPEVTCVVVDVSQEDPEVQFNWYVDGVEVHNAKTKPREEQFNST YRVVSVLTVVHQDWLNGKEYKCKVSNKGLPSSIEKTISKAKGQPREPQVYTLPPSQEEM TKNQVSLTCLVKGFYPSDIAVEWESNGQPENNYKTTPPVLDSDGSFFLYSRLTVDKSRW QEGNVFSCSVMHEALHNHYTQKSLSLSLGK |
| SEQ ID NO. 73 | Human IgG4 constant region | IGHG4*03 | Human Heavy Chain Constant Region (IGHG4*03) Nucleotide Sequence | gcttccaccaagggcccatccgtcttccccctggcgccctgctccaggagcacctccgagagcacagccgccct gggctgcctggtcaaggactacttccccgaaccggtgacggtgtcgtggaactcaggcgccctgaccagcggc gtgcacaccttcccggctgtcctacagtcctcaggactctactccctcagcagcgtggtgaccgtgccctccagc agcttgggcacgaagacctacacctgcaacgtagatcacaagcccagcaacaccaaggtggacaagagagtt gagtccaaatatggtcccccatgcccatcatgcccagcacctgagttcctggggggaccatcagtcttcctgttc cccccaaaacccaaggacactctcatgatctcccggacccctgaggtcacgtgcgtggtggtggacgtgagcc aggaagaccccgaggtccagttcaactggtacgtggatggcgtggaggtgcataatgccaagacaaagccgc gggaggagcagttcaacagcacgtaccgtgtggtcagcgtcctcaccgtcctgcaccaggactggctgaacgg caaggagtacaagtgcaaggtctccaacaaaggcctcccgtcctccatcgagaaaaccatctccaaagccaa agggcagccccgagagccacaggtgtacaccctgcccccatcccaggaggagatgaccaagaaccaggtca gcctgacctgcctggtcaaaggcttctaccccagcgacatcgccgtggagtgggagagcaatgggcagccgga |
| | | | | gaacaactacaagaccacgcctcccgtgctggactccgacggctccttcttcctctacagcaagctcaccgtgg acaagagcaggtggcaggaggggaacgtcttctcatgctccgtgatgcatgaggctctgcacaaccactacac gcagaagagcctctccctgtctctgggtaaa |
| SEQ ID NO. 74 | | | Human Heavy Chain Constant Region (IGHG4*03) Protein Sequence | ASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPSCPAPEFLGGPSVF LFPPKPKDTLMISRTPEVTCVVVDVSQEDPEVQFNWYVDGVEVHNAKTKPREEQFNST YRVVSVLTVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSKLTVDKSRWQ EGNVFSCSVMHEALHNHYTQKSLSLSLGK |
| SEQ ID NO. 75 | Human IgG4PE constant region | IGHG4-PE | Human Heavy Chain Constant Region (IGHG4-PE) Nucleotide Sequence Version A | gcctccaccaagggcccatccgtcttccccctggcgccctgctccaggagcacctccgagagcacggccgccct gggctgcctggtcaaggactacttccccgaaccagtgacggtgtcgtggaactcaggcgccctgaccagcggc gtgcacaccttcccggctgtcctacagtcctcaggactctactccctcagcagcgtggtgaccgtgccctccagc agcttgggcacgaagacctacacctgcaacgtagatcacaagcccagcaacaccaaggtggacaagagagtt gagtccaaatatggtcccccatgcccaccatgcccagcgcctgaatttgaggggggaccatcagtcttcctgttc cccccaaaacccaaggacactctcatgatctcccggacccctgaggtcacgtgcgtggtggtggacgtgagcc aggaagaccccgaggtccagttcaactggtacgtggatggcgtggaggtgcataatgccaagacaaagccgc gggaggagcagttcaacagcacgtaccgtgtggtcagcgtcctcaccgtcctgcaccaggactggctgaacgg caaggagtacaagtgcaaggtctccaacaaaggcctcccgtcatcgatcgagaaaaccatctccaaagccaa agggcagccccgagagccacaggtgtacaccctgcccccatcccaggaggagatgaccaagaaccaggtca gcctgacctgcctggtcaaaggcttctaccccagcgacatcgccgtggagtgggagagcaatgggcagccgga gaacaactacaagaccacgcctcccgtgctggactccgacggatccttcttcctctacagcaggctaaccgtgg |
| | | | | acaagagcaggtggcaggaggggaatgtcttctcatgctccgtgatgcatgaggctctgcacaaccactacac acagaagagcctctccctgtctctgggtaaa |
| SEQ ID NO. 76 | | | Human Heavy Chain Constant Region (IGHG4-PE) Nucleotide Sequence Version B | gcctccaccaagggacctagcgtgttccctctcgcccctgttccaggtccacaagcgagtccaccgctgccctc ggctgtctggtgaaagactactttcccgagcccgtgaccgtctcctggaatagcggagccctgacctccggcgt gcacacatttcccgccgtgctgcagagcagcggactgtatagcctgagcagcgtggtgaccgtgcccagctcca gcctcggcaccaaaacctacacctgcaacgtggaccacaagccctccaacaccaaggtggacaagcgggtgg agagcaagtacggcccccccttgccctccttgtcctgcccctgagttcgagggaggaccctccgtgttcctgtttcc ccccaaacccaaggacaccctgatgatctcccggacacccgaggtgacctgtgtggtcgtggacgtcagccag gaggaccccgaggtgcagttcaactggtatgtggacggcgtggaggtgcacaatgccaaaaccaagcccagg gaggagcagttcaattccacctacagggtggtgagcgtgctgaccgtcctgcatcaggattggctgaacggca aggagtacaagtgcaaggtgtccaacaagggactgcccagctccatcgagaagaccatcagcaaggctaag ggccagccgagggagccccaggtgtataccctgcctcctagccaggaagagatgaccaagaaccaagtgtcc ctgacctgcctggtgaagggattctacccctccgacatcgccgtggagtgggagagcaatggccagcccgaga acaactacaaaacaacccctcccgtgctcgatagcgacggcagcttctttctctacagccggctgacagtggac aagagcaggtggcaggagggcaacgtgttctcctgttccgtgatgcacgaggccctgcacaatcactacaccc agaagagcctctccctgtccctgggcaag |
| SEQ ID NO. 77 | | | Human Heavy Chain Constant Region (IGHG4-PE) Nucleotide Sequence Version C | gccagcaccaagggcccttccgtgttccccctggccccttgcagcaggagcacctccgaatccacagctgccct gggctgtctggtgaaggactactttcccgagcccgtgaccgtgagctggaacagcggcgctctgacatccggcg tccacacctttcctgccgtcctgcagtcctccggcctctactccctgtcctccgtggtgaccgtgcctagctcctcc ctcggcaccaagacctacacctgtaacgtggaccacaaaccctccaacaccaaggtggacaaacgggtcgag agcaagtacggccctccctgccctccttgtcctgcccccgagttcgaaggcggacccagcgtgttcctgttccct cctaagcccaaggacaccctcatgatcagccggacacccgaggtgacctgcgtggtggtggatgtgagccagg |
| | | | | aggaccctgaggtccagttcaactggtatgtggatggcgtggaggtgcacaacgccaagacaaagccccggg aagagcagttcaactccacctacagggtggtcagcgtgctgaccgtgctgcatcaggactggctgaacggcaa ggagtacaagtgcaaggtcagcaataagggactgcccagcagcatcgagaagaccatctccaaggctaaag gccagccccgggaacctcaggtgtacaccctgcctcccagccaggaggagatgaccaagaaccaggtgagcc tgacctgcctggtgaagggattctacccttccgacatcgccgtggagtgggagtccaacggccagcccgagaa caattataagaccacccctcccgtcctcgacagcgacggatccttctttctgtactccaggctgaccgtggataa gtccaggtggcaggaaggcaacgtgttcagctgctccgtgatgcacgaggccctgcacaatcactacacccag aagtccctgagcctgtccctgggaaag |
| SEQ ID NO. 78 | | | Human Heavy Chain Constant Region (IGHG4-PE) Protein Sequence (Amino acid substitution shown in BOLD) | ASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPPCPAPEFEGGPSVF LFPPKPKDTLMISRTPEVTCVVVDVSQEDPEVQFNWYVDGVEVHNAKTKPREEQFNST YRVVSVLTVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTVDKSRWQ EGNVFSCSVMHEALHNHYTQKSLSLSLGK |
| SEQ ID NO. 79 | Inactivate d Human IgG4 constant region | Inactivated IGHG4 | Inactivated Human Heavy Chain Constant Region (IGHG4) | gcctccaccaagggcccatccgtcttccccctggcgccctgctccaggagcacctccgagagcacggccgccct gggctgcctggtcaaggactacttccccgaaccagtgacggtgtcgtggaactcaggcgccctgaccagcggc gtgcacaccttcccggctgtcctacagtcctcaggactctactccctcagcagcgtggtgaccgtgccctccagc agcttgggcacgaagacctacacctgcaacgtagatcacaagcccagcaacaccaaggtggacaagagagtt gagtccaaatatggtcccccatgcccaccatgcccagcgcctccagttgcggggggaccatcagtcttcctgttc cccccaaaacccaaggacactctcatgatctcccggacccctgaggtcacgtgcgtggtggtggacgtgagcc |
| | | | Nucleotide Sequence | aggaagaccccgaggtccagttcaactggtacgtggatggcgtggaggtgcataatgccaagacaaagccgc gggaggagcagttcaacagcacgtaccgtgtggtcagcgtcctcaccgtcctgcaccaggactggctgaacgg caaggagtacaagtgcaaggtctccaacaaaggcctcccgtcatcgatcgagaaaaccatctccaaagccaa agggcagccccgagagccacaggtgtacaccctgcccccatcccaggaggagatgaccaagaaccaggtca gcctgacctgcctggtcaaaggcttctaccccagcgacatcgccgtggagtgggagagcaatgggcagccgga gaacaactacaagaccacgcctcccgtgctggactccgacggatccttcttcctctacagcaggctaaccgtgg acaagagcaggtggcaggaggggaatgtcttctcatgctccgtgatgcatgaggctctgcacaaccactacac acagaagagcctctccctgtctctgggtaaa |
| SEQ ID NO. 80 | | | Inactivated Human Heavy Chain Constant Region (IGHG4) Protein Sequence (inactivating mutations from human IgG4 shown in bold) | ASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPPCPAPPVAGGPSV FLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEEM TKNQVSLTCLVKGFYPSDIAVEWESNGQPENNYKTTPPVLDSDGSFFLYSRLTVDKSRW QEGNVFSCSVMHEALHNHYTQKSLSLSLGK |
| SEQ ID NO. 81 | Human Cκ constant region | IGKC*01 | Human Cκ Light Chain Constant Region (IGKC*01) Nucleotide Sequence | cgtacggtggccgctccctccgtgttcatcttcccaccttccgacgagcagctgaagtccggcaccgcttctgtcg tgtgcctgctgaacaacttctacccccgcgaggccaaggtgcagtggaaggtggacaacgccctgcagtccgg caactcccaggaatccgtgaccgagcaggactccaaggacagcacctactccctgtcctccaccctgaccctgt ccaaggccgactacgagaagcacaaggtgtacgcctgcgaagtgacccaccagggcctgtctagccccgtga ccaagtctttcaaccggggcgagtgt |
| SEQ ID NO. 82 | | | Cκ Light Chain Constant Region (IGKC*01) Amino Acid Sequence | |
| SEQ ID NO. 83 | Human Cκ constant region | IGKC*02 | Cκ Light Chain Constant Region (IGKC*02) Nucleotide Sequence | cgaactgtggctgcaccatctgtcttcatcttcccgccatctgatgagcagttgaaatctggaactgcctctgttg tgtgcctgctgaataacttctatcccagagaggccaaagtacagtggaaggtggataacgccctccaatcgggt aactcccaggagagtgtcacagagcaggagagcaaggacagcacctacagcctcagcagcaccctgacgct gagcaaagcagactacgagaaacacaaagtctacgcctgcgaagtcacccatcagggcctgagctcgcccgt cacaaagagcttcaacaggggagagtgt |
| SEQ ID NO. 84 | | | Cκ Light Chain Constant Region (IGKC*02) Amino Acid Sequence | RTVAAPSVFIFPPSDEQLKSGTASVVCLLNNFYPREAKVQWKVDNALQSGNSQESVTEQ ESKDSTYSLSSTLTLSKADYEKHKVYAGEVTHQGLSSPVTKSFNRGEC |
| SEQ ID NO. 85 | Human Cκ constant region | IGKC*03 | Cκ Light Chain Constant Region (IGKC*03) Nucleotide Sequence | cgaactgtggctgcaccatctgtcttcatcttcccgccatctgatgagcagttgaaatctggaactgcctctgttg tgtgcctgctgaataacttctatcccagagaggccaaagtacagcggaaggtggataacgccctccaatcggg taactcccaggagagtgtcacagagcaggagagcaaggacagcacctacagcctcagcagcaccctgacgct gagcaaagcagactacgagaaacacaaagtctacgcctgcgaagtcacccatcagggcctgagctcgcccgt cacaaagagcttcaacaggggagagtgt |
| SEQ ID NO. 86 | | | Cκ Light Chain Constant Region (IGKC*03) Amino Acid Sequence | RTVAAPSVFIFPPSDEQLKSGTASVVCLLNNFYPREAKVQRKVDNALQSGNSQESVTEQ ESKDSTYSLSSTLTLSKADYEKHKVYACEVTHQGLSSPVTKSFNRGEC |
| SEQ ID NO. 87 | Human Cκ constant region | IGKC*04 | Cκ Light Chain Constant Region (IGKC*04) Nucleotide Sequence | cgaactgtggctgcaccatctgtcttcatcttcccgccatctgatgagcagttgaaatctggaactgcctctgttg tgtgcctgctgaataacttctatcccagagaggccaaagtacagtggaaggtggataacgccctccaatcgggt aactcccaggagagtgtcacagagcaggacagcaaggacagcacctacagcctcagcagcaccctgacgctg agcaaagcagactacgagaaacacaaactctacgcctgcgaagtcacccatcagggcctgagctcgcccgtc acaaagagcttcaacaggggagagtgt |
| SEQ ID NO. 88 | | | Cκ Light Chain Constant Region (IGKC*04) Amino Acid Sequence | RTVAAPSVFIFPPSDEQLKSGTASVVCLLNNFYPREAKVQWKVDNALQSGNSQESVTEQ DSKDSTYSLSSTLTLSKADYEKHKLYACEVTHQGLSSPVTKSFNRGEC |
| SEQ ID NO. 89 | Human CK constant region | IGKC*05 | Cκ Light Chain Constant Region (IGKC*05) Nucleotide Sequence | cgaactgtggctgcaccatctgtcttcatcttcccgccatctgatgagcagttgaaatctggaactgcctctgttg tgtgcctgctgaataacttctatcccagagaggccaaagtacagtggaaggtggataacgccctccaatcgggt aactcccaggagagtgtcacagagcaggacagcaaggacagcacctacagcctcagcaacaccctgacgctg agcaaagcagactacgagaaacacaaagtctacgcctgcgaagtcacccatcagggcctgagctcgcccgtc acaaagagcttcaacaggggagagtgc |
| SEQ ID NO. 90 | | | Cκ Light Chain Constant Region (IGKC*05) Amino Acid Sequence | RTVAAPSVFIFPPSDEQLKSGTASVVCLLNNFYPREAKVQWKVDNALQSGNSQESVTEQ DSKDSTYSLSNTLTLSKADYEKHKVYACEVTHQGLSSPVTKSFNRGEC |
| SEQ ID NO. 91 | Human Cλ constant region | IGLC1*01 | Cλ Light Chain Constant Region (IGLC1*01) Nucleotide Sequence (ENST0000039 0321.2) | cccaaggccaaccccacggtcactctgttcccgccctcctctgaggagctccaagccaacaaggccacactagt gtgtctgatcagtgacttctacccgggagctgtgacagtggcttggaaggcagatggcagccccgtcaaggcg ggagtggagacgaccaaaccctccaaacagagcaacaacaagtacgcggccagcagctacctgagcctgac gcccgagcagtggaagtcccacagaagctacagctgccaggtcacgcatgaagggagcaccgtggagaaga cagtggcccctacagaatgttca |
| SEQ ID NO. 92 | | | Cλ Light Chain Constant Region (IGLC1*01) Amino Acid Sequence (A0A075B6K8) | PKANPTVTLFPPSSEELQANKATLVCLISDFYPGAVTVAWKADGSPVKAGVETTKPSKQS NNKYAASSYLSLTPEQWKSHRSYSCQVTHEGSTVEKTVAPTECS |
| SEQ ID NO. 93 | Human Cλ constant region | IGLC1*02 | Cλ Light Chain Constant Region (IGLC1*02) Nucleotide Sequence Version A | ggtcagcccaaggccaaccccactgtcactctgttcccgccctcctctgaggagctccaagccaacaaggccac actagtgtgtctgatcagtgacttctacccgggagctgtgacagtggcctggaaggcagatggcagccccgtca aggcgggagtggagaccaccaaaccctccaaacagagcaacaacaagtacgcggccagcagctacctgagc ctgacgcccgagcagtggaagtcccacagaagctacagctgccaggtcacgcatgaagggagcaccgtggag aagacagtggcccctacagaatgttca |
| SEQ ID NO. 94 | | | Cλ Light Chain Constant Region (IGLC1*02) Nucleotide Sequence Version B | ggtcagcccaaggccaaccccactgtcactctgttcccgccctcctctgaggagctccaagccaacaaggccac actagtgtgtctgatcagtgacttctacccgggagctgtgacagtggcctggaaggcagatggcagccccgtca aggcgggagtggagaccaccaaaccctccaaacagagcaacaacaagtacgcggccagcagctacctgagc ctgacgcccgagcagtggaagtcccacagaagctacagctgccaggtcacgcatgaagggagcaccgtggag aagacagtggcccctacagaatgttca |
| SEQ ID NO. 95 | | | Cλ Light Chain Constant Region (IGLC1*02) Amino Acid Sequence | GQPKANPTVTLFPPSSEELQANKATLVCLISDFYPGAVTVAWKADGSPVKAGVETTKPS KQSNNKYAASSYLSLTPEQWKSHRSYSCQVTHEGSTVEKTVAPTECS |
| SEQ ID NO. 96 | Human Cλ constant region | IGLC2*01 | Cλ Light Chain Constant Region (IGLC2*01) Nucleotide Sequence Version A | ggccagcctaaggccgctccttctgtgaccctgttcccccatcctccgaggaactgcaggctaacaaggccac cctcgtgtgcctgatcagcgacttctaccctggcgccgtgaccgtggcctggaaggctgatagctctcctgtgaa ggccggcgtggaaaccaccaccccttccaagcagtccaacaacaaatacgccgcctcctcctacctgtccctga cccctgagcagtggaagtcccaccggtcctacagctgccaagtgacccacgagggctccaccgtggaaaaga ccgtggctcctaccgagtgctcc |
| SEQ ID NO. 97 | | | Cλ Light Chain Constant Region (IGLC2*01) Nucleotide Sequence Version B | ggccagcctaaagctgcccccagcgtcaccctgtttcctccctccagcgaggagctccaggccaacaaggcca ccctcgtgtgcctgatctccgacttctatcccggcgctgtgaccgtggcttggaaagccgactccagccctgtca aagccggcgtggagaccaccacaccctccaagcagtccaacaacaagtacgccgcctccagctatctctccct gacccctgagcagtggaagtcccaccggtcctactcctgtcaggtgacccacgagggctccaccgtggaaaag accgtcgcccccaccgagtgctcc |
| SEQ ID NO. 98 | | | Cλ Light Chain Constant Region (IGLC1*02) Amino Acid Sequence | GQPKAAPSVTLFPPSSEELQANKATLVCLISDFYPGAVTVAWKADSSPVKAGVETTTPSK QSNNKYAASSYLSLTPEQWKSHRSYSCQVTHEGSTVEKTVAPTECS |
| SEQ ID NO. 99 | Human Cλ constant region | IGLC2*02 or IGLC2*03 | Cλ Light Chain Constant Region (IGLC2*02 or IGLC2*03) Nucleotide Sequence | ggtcagcccaaggctgccccctcggtcactctgttcccgccctcctctgaggagcttcaagccaacaaggccac actggtgtgtctcataagtgacttctacccgggagccgtgacagtggcctggaaggcagatagcagccccgtca aggcgggagtggagaccaccacaccctccaaacaaagcaacaacaagtacgcggccagcagctatctgagc ctgacgcctgagcagtggaagtcccacagaagctacagctgccaggtcacgcatgaagggagcaccgtggag aagacagtggcccctacagaatgttca |
| SEQ ID NO. 100 | | | Cλ Light Chain Constant Region (IGLC2*02) Amino Acid Sequence | GQPKAAPSVTLFPPSSEELQANKATLVCLISDFYPGAVTVAWKADSSPVKAGVETTTPSK QSNNKYAASSYLSLTPEQWKSHRSYSCQVTHEGSTVEKTVAPTECS |
| SEQ ID NO. 101 | Human Cλ constant region | IGLC3*01 | Cλ Light Chain Constant Region (IGLC3*01) Nucleotide Sequence | cccaaggctgccccctcggtcactctgttcccaccctcctctgaggagcttcaagccaacaaggccacactggt gtgtctcataagtgacttctacccgggagccgtgacagttgcctggaaggcagatagcagccccgtcaaggcg ggggtggagaccaccacaccctccaaacaaagcaacaacaagtacgcggccagcagctacctgagcctgac gcctgagcagtggaagtcccacaaaagctacagctgccaggtcacgcatgaagggagcaccgtggagaaga cagttgcccctacggaatgttca |
| SEQ ID NO. 102 | | | Cλ Light Chain Constant Region (IGLC3*01) Amino Acid Sequence | PKAAPSVTLFPPSSEELQANKATLVCLISDFYPGAVTVAWKADSSPVKAGVETTTPSKQS NNKYAASSYLSLTPEQWKSHKSYSCQVTHEGSTVEKTVAPTECS |
| SEQ ID NO. 103 | Human Cλ constant region | IGLC3*02 | Cλ Light Chain Constant Region (IGLC3*02) Nucleotide Sequence | ggtcagcccaaggctgccccctcggtcactctgttcccaccctcctctgaggagcttcaagccaacaaggccac actggtgtgtctcataagtgacttctacccgggagccagtgacagttgcctggaaggcagatagcagccccgtca aggcgggggtggagaccaccacaccctccaaacaaagcaacaacaagtacgcggccagcagctacctgagc ctgacgcctgagcagtggaagtcccacaaaagctacagctgccaggtcacgcatgaagggagcaccgtggag aagacagtggcccctacggaatgttca |
| SEQ ID NO. 104 | SEQ ID NO. 102 | | Cλ Light Chain Constant Region (IGLC1*02) Amino Acid Sequence | GQPKAAPSVTLFPPSSEELQANKATLVCLISDFYPGPVTVAWKADSSPVKAGVETTTPSK QSNNKYAASSYLSLTPEQWKSHKSYSCQVTHEGSTVEKTVAPTECS |
| SEQ ID NO. 105 | Human Cλ constant region | IGLC3*03 | Cλ Light Chain Constant Region (IGLC3*03) Nucleotide Sequence | ggtcagcccaaggctgccccctcggtcactctgttcccaccctcctctgaggagcttcaagccaacaaggccac actggtgtgtctcataagtgacttctacccgggagccgtgacagtggcctggaaggcagatagcagccccgtca aggcgggagtggagaccaccacaccctccaaacaaagcaacaacaagtacgcggccagcagctacctgagc ctgacgcctgagcagtggaagtcccacaaaagctacagctgccaggtcacgcatgaagggagcaccgtggag aagacagtggcccctacagaatgttca |
| SEQ ID NO. 106 | | | Cλ Light Chain Constant Region (IGLC3*03) Amino Acid Sequence | GQPKAAPSVTLFPPSSEELQANKATLVCLISDFYPGAVTVAWKADSSPVKAGVETTTPSK QSNNKYAASSYLSLTPEQWKSHKSYSCQVTHEGSTVEKTVAPTECS |
| SEQ ID NO. 107 | Human Cλ constant region | IGLC3*04 | Cλ Light Chain Constant Region (IGLC3*04) Nucleotide Sequence | ggtcagcccaaggctgccccctcggtcactctgttcccgccctcctctgaggagcttcaagccaacaaggccac actggtgtgtctcataagtgacttctacccgggagccgtgacagtggcctggaaggcagatagcagccccgtca aggcgggagtggagaccaccacaccctccaaacaaagcaacaacaagtacgcggccagcagctacctgagc ctgacgcctgagcagtggaagtcccacagaagctacagctgccaggtcacgcatgaagggagcaccgtggag aagacagtggcccctacagaatgttca |
| SEQ ID NO. 108 | | | Cλ Light Chain Constant Region (IGLC3*04) Amino Acid Sequence | GQPKAAPSVTLFPPSSEELQANKATLVCLISDFYPGAVTVAWKADSSPVKAGVETTTPSK QSNNKYAASSYLSLTPEQWKSHRSYSCQVTHEGSTVEKTVAPTECS |
| SEQ ID NO. 109 | Human Cλ constant region | IGLC6*01 | Cλ Light Chain Constant Region (IGLC6*01) Nucleotide Sequence | ggtcagcccaaggctgccccatcggtcactctgttcccgccctcctctgaggagcttcaagccaacaaggccac actggtgtgcctgatcagtgacttctacccgggagctgtgaaagtggcctggaaggcagatggcagccccgtc aacacgggagtggagaccaccacaccctccaaacagagcaacaacaagtacgcggccagcagctacctgag cctgacgcctgagcagtggaagtcccacagaagctacagctgccaggtcacgcatgaagggagcaccgtgga gaagacagtggcccctgcagaatgttca |
| SEQ ID NO. 110 | | | Cλ Light Chain Constant Region (IGLC6*01) Amino Acid Sequence | GQPKAAPSVTLFPPSSEELQANKATLVCLISDFYPGAVKVAWKADGSPVNTGVETTTPSK QSNNKYAASSYLSLTPEQWKSHRSYSCQVTHEGSTVEKTVAPAECS |
| SEQ ID NO. 111 | Human Cλ constant region | IGLC7*01 or IGLC7*02 | Cλ Light Chain Constant Region (IGLC7*01 or IGLC7*02) Nucleotide Sequence | ggtcagcccaaggctgccccatcggtcactctgttcccaccctcctctgaggagcttcaagccaacaaggccac actggtgtgtctcgtaagtgacttctacccgggagccgtgacagtggcctggaaggcagatggcagccccgtca aggtgggagtggagaccaccaaaccctccaaacaaagcaacaacaagtatgcggccagcagctacctgagc ctgacgcccgagcagtggaagtcccacagaagctacagctgccgggtcacgcatgaagggagcaccgtggag aagacagtggcccctgcagaatgctct |
| SEQ ID NO. 112 | | | Cλ Light Chain Constant Region (IGLC7*01) Amino Acid Sequence | GQPKAAPSVTLFPPSSEELQANKATLVCLVSDFYPGAVTVAWKADGSPVKVGVETTKPS KQSNNKYAASSYLSLTPEQWKSHRSYSCRVTHEGSTVEKTVAPAECS |
| SEQ ID NO. 113 | Human Cλ constant region | IGLC7*03 | Cλ Light Chain Constant Region (IGLC7*03) Nucleotide Sequence | GGTCAGCCCAAGGCTGCCCCCTCGGTCACTCTGTTCCCACCCTCCTCTGAGGAGCTTC AAGCCAACAAGGCCACACTGGTGTGTCTCGTAAGTGACTTCAACCCGGGAGCCGTG ACAGTGGCCTGGAAGGCAGATGGCAGCCCCGTCAAGGTGGGAGTGGAGACCACCA AACCCTCCAAACAAAGCAACAACAAGTATGCGGCCAGCAGCTACCTGAGCCTGACG CCCGAGCAGTGGAAGTCCCACAGAAGCTACAGCTGCCGGGTCACGCATGAAGGGA GCACCGTGGAGAAGACAGTGGCCCCTGCAGAATGCTCT |
| SEQ ID NO. 114 | | | Cλ Light Chain Constant Region (IGLC7*03) Amino Acid Sequence | GQPKAAPSVTLFPPSSEELQANKATLVCLVSDFNPGAVTVAWKADGSPVKVGVETTKPS KQSNNKYAASSYLSLTPEQWKSHRSYSCRVTHEGSTVEKTVAPAECS |

**Table 5: Summary of the results obtained from gene set enrichment analysis (Example 3)**

| **HALLMARK pathway** | **SIZE** | **NES** | ***P* value** | **FDR q-value** |
|---|---|---|---|---|
| **Down-regulated** | | | | |
| MYOGENESIS | 192 | -1.499 | 0.0036 | 0.0891 |
| UNFOLDED_PROTEIN_RESPONSE | 105 | -1.610 | 0.0040 | 0.1081 |
| EPITHELIAL_MESENCHYMAL_TRANSITION | 189 | -1.374 | 0.0245 | 0.1675 |
| OSTEOBLAST_DIFFERENTIATION* | 12 | -1.573 | 0.0296 | 0.0822 |
| OSTEOBLAST_TRANSCRIPTION_FACTORS** | 14 | -1.525 | 0.0450 | 0.0909 |
| MTORC1_SIGNALING | 182 | -1.328 | 0.0450 | 0.2176 |
| GLYCOLYSIS | 188 | -1.299 | 0.0489 | 0.2437 |
| WNT_BETA_CATENIN_SIGNALING | 39 | -1.458 | 0.0492 | 0.1018 |

| **Up-regulated** | | | | |
|---|---|---|---|---|
| G2M_CHECKPOINT | 189 | 2.185 | 0.0000 | 0.0000 |
| E2F_TARGETS | 189 | 2.171 | 0.0000 | 0.0000 |
| INTERFERON_ALPHA_RESPONSE | 87 | 2.164 | 0.0000 | 0.0000 |
| MITOTIC_SPINDLE | 194 | 2.112 | 0.0000 | 0.0000 |
| INTERFERON_GAMMA_RESPONSE | 183 | 1.959 | 0.0000 | 0.0000 |
| INFLAMMATORY_RESPONSE | 190 | 1.860 | 0.0000 | 0.0035 |
| TNFA_SIGNALING_VIA_NFKB | 193 | 1.624 | 0.0000 | 0.0192 |
| ALLOGRAFT_REJECTION | 175 | 1.576 | 0.0000 | 0.0265 |
| COMPLEMENT | 178 | 1.370 | 0.0056 | 0.1166 |
| APOPTOSIS | 156 | 1.373 | 0.0207 | 0.1266 |

| | | | | |
|---|---|---|---|---|
| *P* value: corrected with Bonferroni post-hoc test. NES: normalized enrichment score: FDR: false discovery rate. *Dataset from Park et al.; **Dataset from Zaidi et al. | | | | |

**Table 6: Iron, blood and bone parameters of wild type mice treated systemically with Tfr2-ECD**

| | | | **PBS (n = 9)** | **Tfr2-ECD (n = 8)** | ***P* value** |
|---|---|---|---|---|---|
| Iron status | | | | | |
| | Plasma iron [µmol/L] | | 31.1 ± 4.5 | 30.1 ± 3.3 | 0.608 |
| | Liver iron [µg/g dry tissue] | | 600.4 ± 333 | 578.3 ± 273 | 0.889 |
| | Spleen iron [µg/g dry tissue] | | 692.0 ± 157 | 620.2 ± 113 | 0.301 |
| | Kidney iron [µg/g dry tissue] | | 112.2 ± 14.4 | 112.6 ± 15.0 | 0.961 |
| | Heart iron [µg/g dry tissue] | | 189.8 ± 54.3 | 207.8 ± 54.1 | 0.727 |

| Blood Counts | | | | | |
|---|---|---|---|---|---|
| | RBC [10¹²/L] | | 9.18 ± 1.39 | 10.1 ± 0.40 | 0.129 |
| | Hemoglobin [mmol/L] | | 8.91 ± 1.25 | 9.77 ± 0.41 | 0.105 |
| | HCT | | 0.47 ± 0.07 | 0.51 ± 0.02 | 0.189 |
| | Platelets [10⁹/L] | | 414 ± 99.5 | 385 ± 69.5 | 0.525 |
| | Reticulocytes [10⁹/L] | | 191 ± 69.6 | 197 ± 24.2 | 0.840 |
| | WBC [10⁹/L] | | 9.23 ± 4.11 | 11.0 ± 3.09 | 0.369 |
| | | *Differential counts, % absolute* | | | |
| | | Neutrophils | 9.52 ± 2.23 | 7.33 ± 2.82 | 0.104 |
| | | Lymphocytes | 88.6 ± 3.37 | 90.1 ± 4.35 | 0.428 |
| | | Monocytes | 1.63 ± 1.47 | 2.17 ± 2.55 | 0.603 |

| *µCT* | | | | | |
|---|---|---|---|---|---|
| | BV/TV [%] Femur | | 9.40 ± 2.62 | 10.7 ± 3.09 | 0.346 |
| | BV/TV [%] Vertebrae | | 25.1 ± 4.52 | 24.1 ± 3.36 | 0.612 |

| | | | | | |
|---|---|---|---|---|---|
| Data are means ± SD and were analyzed by the Student's t test. RBC, red blood cells; HCT, hematocrit; WBC, white blood cells, BV/TV, bone volume/total volume. | | | | | |

**Table 7: Antibody Heavy Chain Variable Region Gene Segments**

| **Gene Segment** | **Example Allele** | **Gene Segment** | **Example Allele** | **Gene Segment** | **Example Allele** | **Gene Segment** | **Example Allele** |
|---|---|---|---|---|---|---|---|
| JH6 | 02 | D1-14 | 01 | VH2-5 | 10 | VH3-43 | 01 |
| JH5 | 02 | D6-13 | 01 | VH3-7 | 01 | VH1-45 | 02 |
| JH4 | 02 | D5-12 | 01 | VH1-8 | 01 | VH1-46 | 01 |
| JH3 | 02 | D4-11 | 01 | VH3-9 | 01 | VH3-48 | 01 |
| JH2 | 01 | D3-10 | 01 | VH3-11 | 01 | VH3-49 | 05 |
| JH1 | 01 | D3-9 | 01 | VH3-13 | 01 | VH5-51 | 01 |
| D7-27 | 02 | D2-8 | 01 | VH3-15 | 01 | VH3-53 | 01 |
| D1-26 | 01 | D1-7 | 01 | VH1-18 | 01 | VH1-58 | 01 |
| D6-25 | 01 | D6-6 | 01 | VH3-20 | 01 | VH4-59 | 01 |
| D5-24 | 01 | D5-5 | 01 | VH3-21 | 03 | VH4-61 | 01 |
| D4-23 | 01 | D4-4 | 01 | VH3-23 | 04 | VH3-64 | 02 |
| D3-22 | 01 | D3-3 | 01 | VH1-24 | 01 | VH3-66 | 03 |
| D2-21 | 02 | D2-2 | 02 | VH2-26 | 01 | VH1-69 | 12 |
| D1-20 | 01 | D1-1 | 01 | VH4-28 | 05 | VH2-70 | 04 |
| D6-19 | 01 | VH6-1 | 01 | VH3-30 | 18 | VH3-72 | 01 |
| D5-18 | 01 | VH1-2 | 02 | VH4-31 | 03 | VH3-73 | 02 |
| D4-17 | 01 | VH1-3 | 01 | VH3-33 | 01 | VH3-74 | 01 |
| D3-16 | 02 | VH4-4 | 02 | VH4-34 | 01 | | |
| D2-15 | 01 | VH7-4 | 01 | VH4-39 | 01 | | |

**Table 8: Antibody Light Chain Variable Region Gene Segments**

| **Gene Segment** | **Example Allele** | **Gene Segment** | **Example Allele** | **Gene Segment** | **Example Allele** |
|---|---|---|---|---|---|
| Jκ5 | | Vκ3-15 | 01 | Vκ1-8 | 01 |
| Jκ4 | | Vκ1-16 | 02 | Vκ1-43 | 01 |
| Jκ3 | | Vκ1-17 | 01 | Vκ3-11 | 01 |
| Jκ2 | | Vκ3-20 | 01 | Vκ1-12 | 02 |
| Jκ1 | | Vκ6-21 | 01 | Vκ1-13 | 01 |
| Vκ4-1 | | Vκ2-24 | 01 | VK3-15 | 01 |
| Vκ5-2 | | Vκ1-27 | 01 | Vκ1-16 | 01 |
| Vκ1-5 | | Vκ2-28 | 01 | Vκ1-17 | 01 |
| Vκ1-6 | 01 | Vκ2-29 | 01 | Vκ3-20 | 01 |
| Vκ1-8 | 01 | Vκ2-30 | 01 | Vκ2-26 | 02 |
| Vκ1-9 | 01 | Vκ1-33 | 01 | Vκ2-28 | 01 |
| Vκ3-11 | 01 | Vκ1-39 | 01 | Vκ2-29 | 01 |
| Vκ1-12 | 01 | Vκ2-40 | 01 | Vκ2-30 | 01 |
| Vκ1-13 | 01 | Vκ3-7 | 01 | Vκ1-33 | 01 |

The invitation also pertains to the following:
1. A transferrin receptor 2 (Tfr2) antagonist or agonist for use in a method of treating or preventing a bone disease or condition in a human or animal subject, the method comprising administering the antagonist or agonist to the subject and antagonising Tfr2 in the subject.
2. The antagonist of item 1, wherein the method comprises
   (i) inhibiting p38 MAP kinase pathway signalling in bone cells by antagonising Tfr2 of the cells;
   (ii) upregulating Wnt expression in bone cells by antagonising Tfr2 in the subject;
   (iii) inhibiting sclerostin, Dkk1 and/or Activin A activity or expression in bone cells by antagonising Tfr2 of the cells; or
   (iv) inhibiting expression of *Phex, Dmp1, Dkk1* and/or *Sost* in bone cells by antagonising Tfr2 of the cells.
3. The antagonist of any preceding item , wherein the method comprises inhibiting the binding of Tfr2 to a BMP in the subject.
4. The antagonist of any preceding item , wherein
   (i) the disease or condition is osteoporosis;
   (ii) the method is for causing one or more of the following:-
      (a) Increased bone volume;
      (b) Increased bone density;
      (c) Increased trabecular number (Tb.N);
      (d) Increased trabecular thickness (Tb.Th);
      (e) Decreased trabecular spacing (Tb.Sp);
      (f) Increased bone mineral density;
      (g) Increased bone micro-mineralisation density;
      (h) Increased bone mass; and
      (i) Increased bone strength;
   (iii) the method is for increasing bone formation in the subject;
   (iv) the method is for decreasing bone resorption in the subject;
   (v) the method is for increasing osteoblasts (or bone formation activity thereof) in the subject;
   (vi) the method is for decreasing osteoclasts (or bone resorption activity thereof) in the subject;
   (vii) the method is for increasing bone turnover in the subject;
   (viii) the method is for increasing pro-collagen type I N-terminal peptide (P1NP) in the subject;
   (ix) the method is for increasing C-terminal telopeptide of type I collagen (CTX) in the subject; or
   (x) the method is for increasing osteocytes in the subject.
5. The antagonist of any preceding claim, wherein the method comprises administering a sclerostin, Dkk1 or Activin A antagonist to the subject.
6. The agonist of item 1, wherein the method comprises (i) stimulating p38 MAP kinase pathway signalling in bone cells by agonising Tfr2 of the cells; (ii) downregulating Wnt expression in bone cells by agonising Tfr2 of the cells; or (iii) stimulating sclerostin activity or expression in bone cells by agonising Tfr2 of the cells.
7. The agonist of 1 or 6, wherein the method comprises promoting BMP binding of Tfr2.
8. The agonist of item 7, wherein the BMP is one or more of BMP2, 4, 6 and 7.
9. The agonist of any one of items 1 and 6 to 8, wherein the method comprises administering a sclerostin, Dkk1 or Activin A agonist to the subject.
10. A Bone Morphogenetic Protein (BMP)-binding agent for use in a method of treating or preventing a disease or condition in a human or animal subject, the method comprising administering the BMP-binding agent to the subject, wherein the agent competes with soluble Tfr2-ECD for binding to the BMP, and wherein the disease or condition is mediated by said BMP.
11. The agent of item 10, wherein the agent is a BMP trap.
12. The agent of item 10 or 11, wherein the BMP is a BMP2, 4, 6 or 7.
13. The agent of any one of items 10 to 12, wherein the agent binds to one, more or all of BMP2, 4, 6 and 7 more strongly than the binding of holo-tranferrin and/or BMPR to said BMP(s).
14. The agent of any one of items 10 to 13, wherein the agent comprises Tfr2 extracellular domain (Tfr2-ECD), optionally wherein the agent is comprised by a fusion protein comprising a second moiety.
15. The agent of item 14 wherein the second moiety is a half-life-extending moiety for enhancing half-life of the trap in a subject; optionally wherein the second moiety is selected from an antibody Fc region, a polyethylene glycol (PEG) moiety, serum albumin and an anti-serum albumin binding moiety.
16. The agent of any one of items 10 to 15, wherein the disease or condition is a bone disease or condition.
17. The antagonist, agonist or agent of any preceding claim, wherein the disease or condition is
   (a) a sclerosing disease or condition;
   (b) a disease or condition comprising pathological bone formation; or
   (c) an ossification disease or condition, optionally a heterotypic ossification (HO) disease or condition, or *fibrodysplasia ossificans progressive* (FOP) disease or condition.
18. The antagonist, agonist or agent of item 17, wherein the disease or condition is selected from HO of muscle, Van Buchem disease and Sclerosteosis.
19. The agonist or agent of any one of items 1 and 10 to 18, wherein the method comprises administering a retinoic acid receptor gamma (RAR-γ) agonist (optionally palovarotene) to the subject simultaneously or sequentially with a Tfr2 agonist or the trap.
20. The agonist or agent of any one of items 1 and 10 to 19, wherein the method comprises administering a BMP signalling antagonist to the subject simultaneously or sequentially with a Tfr2 agonist or the trap.
21. The agent of any one of items 10 to 20, wherein the method inhibits cartilage formation in the subject.
22. The agent of any one of items 10 to 21, wherein the method inhibits chondrocyte formation in the subject.
23. The agent of any one of items 10 to 22, wherein the disease or condition is BMP-2-induced HO; BMP-4-induced HO; BMP-6-induced HO; or BMP-7-induced HO.
24. The antagonist, agonist or agent of any preceding item , wherein the antagonist or agonist is (i) an anti-Tfr2 antibody or antibody fragment that specifically binds to Tfr2; (ii) a Tfr2-ECD monomer; or (iii) a Tfr2-ECD dimer.
25. The antagonist, agonist or agent of item 24, wherein the antibody or fragment competes with an antibody selected from 1B1 (MyBioSource, MBS833691), 3C5 (Abnova, H00007036-M01), CY-TFR (Abnova, MAB6780) and B-6 (Santa Cruz Biotechnology, sc-376278), 353816 (R&D Systems, MAB3120) and 9F8 1C11 (Santa Cruz Biotechnology, sc-32271) for binding to Tfr2 as determined by surface plasmon resonance (SPR).
26. The antagonist, agonist or agent of item 24, wherein the Tfr2-ECD monomer is a Tfr2-ECD-Fc monomer; the Tfr2-ECD dimer is a Tfr2-ECD-Fc dimer; and the ECD and Fc are a human Tfr2 ECD and a human Fc.
27. The antagonist, agonist or agent of item 24 or 26, wherein the Tfr2-ECD is Tfr2α-ECD or Tfr2β-ECD.
28. A pharmaceutical composition comprising the antagonist, agonist or agent of any preceding item and a pharmaceutically acceptable diluent, excipient or carrier.
29. The composition of item 28, further comprising an anti-sclerostin antibody (optionally romosozumab), anti-Dkk1 antibody or anti-Activin A antibody, or fragment thereof.
30. A pharmaceutical composition comprising the antibody, fragment, dimer or monomer recited in any one of items 24 to 27 and a pharmaceutically acceptable diluent, excipient or carrier.
31. The composition of claim 30 when dependent from item 10, further comprising a retinoic acid receptor gamma (RAR-γ) agonist (optionally polovarotene) and/or a BMP antagonist.

## Claims

1. A Bone Morphogenetic Protein (BMP)-binding agent for use in a method of treating or preventing a disease or condition in a human or animal subject, the method comprising administering the BMP-binding agent to the subject, wherein the agent competes with soluble Tfr2-ECD for binding to the BMP, and wherein the disease or condition is mediated by said BMP.

2. The agent for use of claim 1, wherein the agent is a BMP trap.

3. The agent for use of claim 1 or 2, wherein the BMP is a BMP2, 4, 6 or 7.

4. The agent for use of any one of claims 1 to 3, wherein the agent binds to one, more or all of BMP2, 4, 6 and 7 more strongly than the binding of holo-tranferrin and/or BMPR to said BMP(s).

5. The agent for use of any one of claims 1 to 4, wherein the agent comprises Tfr2 extracellular domain (Tfr2-ECD), optionally wherein the agent is comprised by a fusion protein comprising a second moiety, preferably
wherein the second moiety is a half-life-extending moiety for enhancing half-life of the trap in a subject; optionally wherein the second moiety is selected from an antibody Fc region, a polyethylene glycol (PEG) moiety, serum albumin and an anti-serum albumin binding moiety.

6. The agent for use of any one of claims 1 to 5, wherein the disease or condition is a bone disease or condition, preferably
wherein the disease or condition is
(a) a sclerosing disease or condition;
(b) a disease or condition comprising pathological bone formation; or
(c) an ossification disease or condition, optionally a heterotypic ossification (HO) disease or condition, or *fibrodysplasia ossificans progressive* (FOP) disease or condition; more preferably
wherein the disease or condition is selected from HO of muscle, Van Buchem disease and Sclerosteosis.

7. The agent for use of any one of claims 1 to 6, wherein the method comprises administering a retinoic acid receptor gamma (RAR-γ) agonist (optionally palovarotene) to the subject simultaneously or sequentially with a Tfr2 agonist or the trap.

8. The agent for use of any one of claims 1 to 7, wherein the method comprises administering a BMP signalling antagonist to the subject simultaneously or sequentially with a Tfr2 agonist or the trap.

9. The agent for use of any one of claims 1 to 8, wherein the disease or condition is BMP-2-induced HO; BMP-4-induced HO; BMP-6-induced HO; or BMP-7-induced HO.

10. The agent for use of any preceding claim, wherein the agent is (i) an anti-Tfr2 antibody or antibody fragment that specifically binds to Tfr2; (ii) a Tfr2-ECD monomer; or (iii) a Tfr2-ECD dimer.

11. The agent for use of claim 10, wherein the antibody or fragment competes with an antibody selected from 1B1 (MyBioSource, MBS833691), 3C5 (Abnova, H00007036-M01), CY-TFR (Abnova, MAB6780) and B-6 (Santa Cruz Biotechnology, sc-376278), 353816 (R&D Systems, MAB3120) and 9F8 1C11 (Santa Cruz Biotechnology, sc-32271) for binding to Tfr2 as determined by surface plasmon resonance (SPR).

12. The agent for use of claim 10, wherein the Tfr2-ECD monomer is a Tfr2-ECD-Fc monomer; the Tfr2-ECD dimer is a Tfr2-ECD-Fc dimer; and the ECD and Fc are a human Tfr2 ECD and a human Fc.

13. The agent for use of claim 10 or 12, wherein the Tfr2-ECD is Tfr2α-ECD or Tfr2β-ECD.

14. A pharmaceutical composition comprising the agent of any preceding claim and a pharmaceutically acceptable diluent, excipient or carrier.

15. The composition of claim 14, further comprising an anti-sclerostin antibody (optionally romosozumab), anti-Dkk1 antibody or anti-Activin A antibody, or fragment thereof.
